# EUROPEAN PATENT SPECIFICATION

(11) **EP 2 902 495 B1**
(45) Date of publication and mention of the grant of the patent: **25.12.2019**
(21) Application number: 14184608.9
(22) Date of filing: 07.11.2008
(51) Int. Cl.: A61K 31/519, A61K 39/395, A61K 31/4196, A61P 37/06

(54) **Use of tam receptor activators as immunosuppressors**
Verwendung von Tam-Rezeptor-Aktivatoren als Immunsuppressiva
Utilisation d'activateurs de récepteurs Tam en tant qu'immunosuppresseurs

(30) Priority: 09.11.2007 US 986984 P; 13.12.2007 US 13598 P; 24.07.2008 US 83462 P
(43) Date of publication of application: 05.08.2015
(62) Divisional of application: 08876595.3
(73) Proprietor: The Salk Institute for Biological Studies, La Jolla, CA 92037 (US)
(72) Inventor: Rothlin, Carla, V., Del Mar CA California 92014 (US); Lemke, Greg, E., La Jolla CA California 92037 (US)
(74) Representative: Bezzubova, Olga

(56) References cited:
- WO-A1-96/28548
- WO-A2-03/039575
- WO-A2-2005/007183
- O'DONNELL K ET AL: "EXPRESSION OF RECEPTOR TYROSINE KINASE AXL AND ITS LIGAND GAS6 IN RHEUMATOID ARTHRITIS EVIDENCE FOR A NOVEL ENDOTHELIAL CELL SURVIVAL PATHWAY", AMERICAN JOURNAL OF PATHOLOGY; [10640], ELSEVIER INC, US, vol. 154, no. 4, 1 April 1999 (1999-04-01) , pages 1171-1180, XP000971169, ISSN: 0002-9440
- HAFIZI S ET AL: "Gas6 and protein S: Vitamin K-dependent ligands for the Axl receptor tyrosine kinase subfamily", FEBS JOURNAL 200612 GB, vol. 273, no. 23, December 2006 (2006-12), pages 5231-5244, XP009183738, ISSN: 1742-464X
- Gian Carlo Avanzi ET AL: "GAS6 Inhibits Granulocyte Adhesion to Endothelial Cells", Blood, 1 April 1998 (1998-04-01), page 2334, XP55183114, UNITED STATES Retrieved from the Internet: URL:http://bloodjournal.hematologylibrary. org/cgi/reprint/91/7/2334
- BENZAKOUR O ET AL: "Gas-6 and protein S: vitamin K-dependent factors and ligands for the TAM tyrosine kinase receptors family // Fonctions nouvelles de Gas-6 et de la protéine S , Facteurs vitamine K-dépendants et ligands des récepteurs tyrosine kinase de la famille TAM", M/S MEDECINE SCIENCES, SOCIETE DES PERIODIQUES FLAMMARION, PARIS, FR, vol. 23, no. 10, 1 October 2007 (2007-10-01), pages 826-833, XP002679231, ISSN: 0767-0974, DOI: 10.1051/MEDSCI/20072310826 [retrieved on 2007-10-15]
- LU Q ET AL: "Homeostatic regulation of the immune system by receptor tyrosine kinases of the Tyro 3 family.", 13 July 2001 (2001-07-13), SCIENCE (NEW YORK, N.Y.) 13 JUL 2001 LNKD- PUBMED:11452127, VOL. 293, NR. 5528, PAGE(S) 306 - 311, XP002615080, ISSN: 0036-8075 * See abstract and see page 307, third hand column; figure 2(d): triple mutant cells lacking tyro 3, axl, Mer genes are characterised by pronounced increase in IFN-gamma, * * See page 310, first hand column: Mer single mutant charged with bacterial lipopolysaccharide (LPS) expresses excessive amounts of TNF-alpha * * See page 310, third hand-column and figure 5D: triple mutants macrophages produce excessive amount of TNF-alpha *
- ROTHLIN CARLA V ET AL: "TAM receptors are pleiotropic inhibitors of the innate immune response.", 14 December 2007 (2007-12-14), CELL 14 DEC 2007 LNKD- PUBMED:18083102, VOL. 131, NR. 6, PAGE(S) 1124 - 1136, XP002615081, ISSN: 0092-8674 * See summary, figures (e.g. fig 1 and 7) and discussion: role of TAM in the immune response. TAM activation inhibits cytokine production, whereas TAM inhibition induces cytokine production, including TNF-alpha. Use of small TAM inhibitors to as vaccine adjuvants *
- ANNA ZAGÓRSKA ET AL: "Diversification of TAM receptor tyrosine kinase function", NATURE IMMUNOLOGY, vol. 15, no. 10, 7 September 2014 (2014-09-07), pages 920-928, XP055462121, New York ISSN: 1529-2908, DOI: 10.1038/ni.2986

### SEQUENCE LISTING

<110> The Salk Institute for Biological Studies Rothlin, Carla V. Lemke, Greg E.
<120> USE OF TAM RECEPTOR INHIBITORS AS IMMUNOENHANCERS AND TAM ACTIVATORS AS IMMUNOSUPPRESSORS
<130> 7158-78567-03
<150> 60/986, 984
   <151> 2007-11-09
<150> 61/013, 598
   <151> 2007-12-13
<150> 61/083, 462
   <151> 2008-07-24
<160> 21
<170> PatentIn version 3.5
<210> 1
   <211> 27
   <212> DNA
   <213> Artificial Sequence
<220>
   <223> Synthetic oligonucleotide primer for actin.
<400> 1
   ctcctcctga gcgcaagtac tctgtgt 27
<210> 2
   <211> 26
   <212> DNA
   <213> Artificial Sequence
<220>
   <223> Synthetic oligonucleotide primer for actin.
<400> 2
   gtgcacgatg gaggggccgg actcat 26
<210> 3
   <211> 21
   <212> DNA
   <213> Artificial Sequence
<220>
   <223> Synthetic oligonucleotide primer for AXL.
<400> 3
   atgccagtca agtggattgc t 21
<210> 4
   <211> 20
   <212> DNA
   <213> Artificial Sequence
<220>
   <223> Synthetic oligonucleotide primer for AXL.
<400> 4
   cacacatcgc tcttgctggt 20
<210> 5
   <211> 21
   <212> DNA
   <213> Artificial Sequence
<220>
   <223> Synthetic oligonucleotide primer for BAFF .
<400> 5
   tgctatgggt catgtcatcc a 21
<210> 6
   <211> 21
   <212> DNA
   <213> Artificial Sequence
<220>
   <223> Synthetic oligonucleotide primer for BAFF .
<400> 6
   ggcagtgttt tgggcatatt c 21
<210> 7
   <211> 20
   <212> DNA
   <213> Artificial Sequence
<220>
   <223> Synthetic oligonucleotide primer for GAPDH.
<400> 7
   tcccactctt ccaccttcga 20
<210> 8
   <211> 21
   <212> DNA
   <213> Artificial Sequence
<220>
   <223> Synthetic oligonucleotide primer for GAPDH.
<400> 8
   agttgggata gggcctctct t 21
<210> 9
   <211> 19
   <212> DNA
   <213> Artificial Sequence
<220>
   <223> Synthetic oligonucleotide primer IFN beta.
<400> 9
   atgagtggtg gttgcaggc 19
<210> 10
   <211> 25
   <212> DNA
   <213> Artificial Sequence
<220>
   <223> Synthetic oligonucleotide primer IFN beta.
<400> 10
   tgacctttca aatgcagtag attca 25
<210> 11
   <211> 22
   <212> DNA
   <213> Artificial Sequence
<220>
   <223> Synthetic oligonucleotide primer IRAK-M.
<400> 11
   agccagtctg aggtcacctt tc 22
<210> 12
   <211> 19
   <212> DNA
   <213> Artificial Sequence
<220>
   <223> Synthetic oligonucleotide primer IRAK-M.
<400> 12
   cgttgcaatc cgcttcact 19
<210> 13
   <211> 25
   <212> DNA
   <213> Artificial Sequence
<220>
   <223> Synthetic oligonucleotide primer for SHIP.
<400> 13
   accagcatga catttaaaac agttg 25
<210> 14
   <211> 23
   <212> DNA
   <213> Artificial Sequence
<220>
   <223> Synthetic oligonucleotide primer for SHIP.
<400> 14
   agatatggct gatccgattc tca 23
<210> 15
   <211> 17
   <212> DNA
   <213> Artificial Sequence
<220>
   <223> Synthetic oligonucleotide primer for SOCS1.
<400> 15
   ccgtgggtcg cgagaac 17
<210> 16
   <211> 24
   <212> DNA
   <213> Artificial Sequence
<220>
   <223> Synthetic oligonucleotide primer for SOCS1.
<400> 16
   aactcaggta gtcacggagt accg 24
<210> 17
   <211> 17
   <212> DNA
   <213> Artificial Sequence
<220>
   <223> Synthetic oligonucleotide primer for SOCS3.
<400> 17
   tcccatgccg ctcacag 17
<210> 18
   <211> 23
   <212> DNA
   <213> Artificial Sequence
<220>
   <223> Synthetic oligonucleotide primer foir SOCS3.
<400> 18
   acaggaccag ttccaggtaa ttg 23
<210> 19
   <211> 19
   <212> DNA
   <213> Artificial sequence
<220>
   <223> Synthetic oligonucleotide CpG-ODN 1668
<400> 19
   tccatgacgt tccgatgct 19
<210> 20
   <211> 53
   <212> PRT
   <213> Homo sapiens
<400> 20
   Gln Phe Leu Arg Pro Arg Gln Arg Arg Ala Phe Gln Val Phe Glu Glu
   1               5                   10                  15
   Ala Lys Gln Gly His Leu Glu Arg Glu Cys Val Glu Glu Leu Cys Ser
               20                  25                  30
   Arg Glu Glu Ala Arg Glu Val Phe Glu Asn Asp Pro Glu Thr Asp Tyr
           35                  40                  45
   Phe Tyr Pro Arg Tyr
       50
<210> 21
   <211> 60
   <212> PRT
   <213> Homo sapiens
<400> 21
   Glu Ala Asn Leu Leu Ser Lys Gln Gln Ala Ser Gln Val Leu Val Arg
   1               5                   10                  15
   Lys Arg Arg Ala Asn Ser Leu Leu Glu Glu Thr Lys Gln Gly Asn Leu
               20                  25                  30
   Glu Arg Glu Cys Ile Glu Glu Leu Cys Asn Lys Glu Glu Ala Arg Glu
           35                  40                  45
   Val Phe Glu Asn Asp Pro Glu Thr Asp Tyr Phe Tyr
       50                  55                  60

## Description

### FIELD OF THE DISCLOSURE

This disclosure concerns compositions and methods for immunoenhancement or immunosuppression. The disclosure concerns methods of using a Tyro 3, Axl, and Mer (TAM) receptor inhibitor for immunoenhancement, for example as a vaccine adjuvant, or for the treatment of sepsis or other disorder where immunoenhancement is desired. In addition the disclosure concerns methods of using a TAM receptor agonist for immunosuppression, for example for the treatment of autoimmune diseases, post-transplant immunosuppression, or for the treatment of other disorders where immunosuppression is desired.

### BACKGROUND

There are numerous diseases and conditions in which immunoenhancement would be desirable. For instance, the increasing threat of bio-weapons in both asymmetric warfare and terrorist attacks necessitates effective counter measures against pathogens that cause rapid onset diseases and morbidity, including *Bacillus anthracis, Yersinia pestis,* and Ebola and Lassa viruses. Given the nature of these bio-agents, effective vaccination requires the induction of a rapid and potent response with as few doses as possible. Currently approved vaccines, as well as the vaccines under development, mostly rely on cell-free filtrates, recombinant antigens, and synthetic peptides, such as the 'anthrax vaccine adsorbed' antigen. Therefore, they lack the immunogenecity of the whole, killed pathogens used in traditional vaccines. In order to translate recombinant approaches into effective vaccination protocols, there is a need to develop powerful immunoadjuvants that safely increase the recombinant-antigen-based response.

Similarly, severe sepsis could be treated with effective immunoenhancement. Sepsis refers to the systemic inflammatory response to an infection (see, for instance, American College of Chest Physicians Society of Critical Care Medicine, (1997) Chest, 101:1644-55), and can progress to severe sepsis, septic shock, multiple organ dysfunction, and ultimately death. Severe sepsis is associated with hypotension, disseminated intravascular coagulation, and hypoperfusion abnormalities, including lactic acidosis, oliguria, and changes in mental status. Patients with severe sepsis often also exhibit immunosuppression, which compromises their ability to eradicate the primary infection and predisposes them to secondary opportunistic and/or nosocomial infections. Every year, hundreds of thousands of people suffer from sepsis and die.

Additionally, immunodeficiency, a state in which the immune system's ability to fight infectious disease is compromised or entirely absent, could be treated with immunoenhancement. Immunodeficiency can be either congenital or acquired, and an immunocompromised person is very vulnerable to opportunistic infections. HIV infection is a major cause of immunodeficiency, and an estimated 39.5 million people worldwide were living with HIV infection in the year 2006. Despite recent, improved access to antiretroviral treatment and care in many regions of the world, the AIDS epidemic claimed an estimated 2.8 million lives in 2005, of which more than half a million were children.

Furthermore, immunoenhancement would be desirable for use with vaccines, for instance dendritic cell-based cancer vaccines, in which dendritic cells that have a natural or genetically engineered reactivity to a patients' cancer are expanded *in vitro* using a variety of means and then adoptively transferred into a cancer patient. Dendritic cell-based vaccines would be significantly more effective if used in conjunction with agents that boost the immune response of the host organism.

Moreover, there are numerous diseases and conditions in which immunosuppression would be desirable. For example, many human diseases and disease syndromes, including systemic lupus erythematosis (SLE), rheumatoid arthritis, Sjörgren's syndrome, inflammatory bowel disease (Crohn's Disease and ulcerative colitis), psoriasis, renal, pulmonary, and hepatic fibroses, type I diabetes, and multiple sclerosis, among others, result from chronic inflammation of the immune system. In addition, organ rejection following transplantation often is initiated by immune inflammation. In these settings, bacterial and viral infections almost always exacerbate inflammatory symptoms and disease. The acute inhibition of immune inflammation is therefore a clinical priority.

Current treatment options are either: (a) of marginal efficacy, as is the case for aspirin and other non-steroidal anti-inflammatory drugs; or (b) encumbered by substantive deleterious side effects, as is the case for glucocorticoids and other steroids, for calcineurin inhibitors such as cyclosporine, and for anti-tumor necrosis factor (TNF) antibodies such as infliximab (Remicade™) and TNF receptor decoys such as etanercept (Enbrel™). Given the foregoing, it would be desirable to have improved immunoenhancing agents, for instance for use as vaccine adjuvants, treatments for sepsis, and treatments for immunodeficiency. Further, it would also be desirable to have improved immunosuppressive agents, for instance for use in treating autoimmune diseases, for inducing post-transplant immunosuppression, and for the treatment of other disorders where immunosuppression is desired.

WO96/28548 discloses an activator of the Rse and Mer receptor protein tyrosine kinases. The activator is encoded by growth arrest-specific gene 6 (Gas6).

WO03/039575 relates to methods of exposing a patient suffering from CNS disorder to reagent that modulates the proliferation, migration, differentiation and survival of central nervous system cells *via* Reelin, Gas6 or Protein S signaling.

WO2005/007183 relates to a method for the treatment of anemia based on the administration of the product of Gas6.

O'Donnell et al. (AMERICAN JOURNAL OF PATHOLOGY, vol.154, no.4, 1 April 1999) reports on Gas6 and its use in the cells of a patient affected by rheumatoid arthritis.

HAFIZI S ET AL. (FEBS JOURNAL 200612 GB, (200612), vol. 273, no. 23) reports that Gas 6 and protein S are ligands and activators of axl and suppress inflammation.

Gian Carlo Avanzi ET AL, "GAS6 Inhibits Granulocyte Adhesion to Endothelial Cells", Blood, UNITED STATES, (19980401), page 2334, aims to evaluate the GAS6 effect on the adhesive function of endothelial cells.

BENZAKOUR O ET AL, "Gas-6 and protein S: vitamin K-dependent factors and ligands for the TAM tyrosine kinase receptors family ", M/S MEDECINE SCIENCES, SOCIETE DES PERIODIQUES FLAMMARION, PARIS, FR, vol. 23, no. 10, (20071001), pages 826 - 833, studies the molecular mechanisms underlying the role of vitamin K-dependent proteins in regulating apoptotic cell phagocytosis.

LU Q ET AL, Homeostatic regulation of the immune system by receptor tyrosine kinases of the Tyro 3 family, SCIENCE,13 JUL 2001, VOL. 293, PAGE(S) 306 - 311, reports using mutant mice that lack receptor tyrosine kinases, Tyro 3, Axl, and Mer, to study their immunoregulatory role.

ROTHLIN CARLA V ET AL, TAM receptors are pleiotropic inhibitors of the innate immune response, CELL 14 DEC 2007, VOL. 131, NR. 6, PAGE(S) 1124 - 1136, studies the signaling pathway of TAM receptor in the inhibition of inflammation.

### SUMMARY OF THE DISCLOSURE

The present invention provides a TAM receptor agonist for use in a method of treating or preventing an autoimmune disease, an allergy, a graft-versus-host disease, or a transplant rejection by suppressing the immune response in a subject, wherein the TAM receptor agonist is an antibody that binds to an extracellular domain of the TAM receptor, and wherein the TAM receptor is Axl.

According to some embodiments of the invention, the TAM receptor agonist can cross-link and activate the TAM receptor in a TAM receptor dimer.

According to some embodiments of the invention, the method further comprises administering to the subject an immunosuppressive agent, thereby suppressing the immune response in the subject.

According to some embodiments of the invention, the TAM receptor agonist is administered to the subject substantially concurrently with the immunosuppressive agent.

According to some embodiments of the invention, the autoimmune disease is rheumatoid arthritis, Hashimoto's thyroiditis, pernicious anemia, Crohn's disease, ulcerative colitis, psoriasis, renal fibrosis, pulmonary fibrosis, hepatic fibrosis, Addison's disease, type I diabetes, systemic lupus erythematosus, dermatomyositis, Sjogren's syndrome, multiple sclerosis, myasthenia gravis, Reiter's syndrome, or Grave's disease.

Within the context of the invention and its embodiments, a TAM receptor agonist is to be understood as an antibody that binds an extracellular domain of Axl.

Disclosed herein is the surprising discovery that TAM (Tyro 3, Axl, and Mer) receptor inhibitors are effective for immunoenhancement and TAM receptor agonists are effective for immunosuppression. Methods are disclosed for enhancing an immune response in a subject. In some examples, the method includes administering to a subject in need of immunoenhancement a therapeutically effective amount of a TAM receptor inhibitor, thereby enhancing the immune response in the subject. Methods are also disclosed for screening for an immunoenhancing agent. These methods can include contacting a cell expressing a TAM receptor with a test agent, and determining whether the test agent significantly reduces or inhibits TAM receptor activity.

Methods are also disclosed for suppressing an immune response in a subject. In some examples, the method includes administering to a subject in need of immunosuppression a therapeutically effective amount of a TAM receptor agonist, thereby suppressing the immune response in the subject. Methods are also disclosed for screening for an immunosuppressive agent. These methods can include contacting a cell expressing a TAM receptor with a test agent, and determining whether the test agent significantly increases or enhances TAM receptor activity.

The foregoing and other features will become more apparent from the following detailed description which proceeds with reference to the accompanying figures.

### BRIEF DESCRIPTION OF THE FIGURES

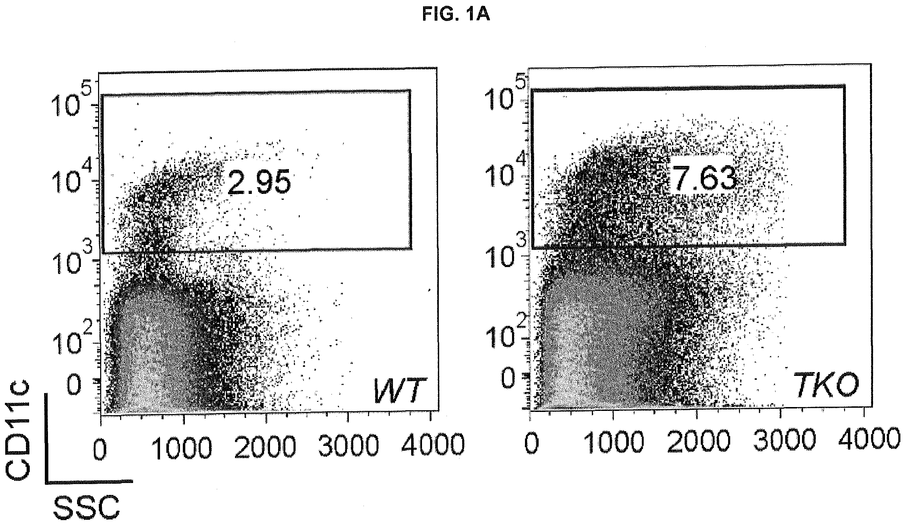

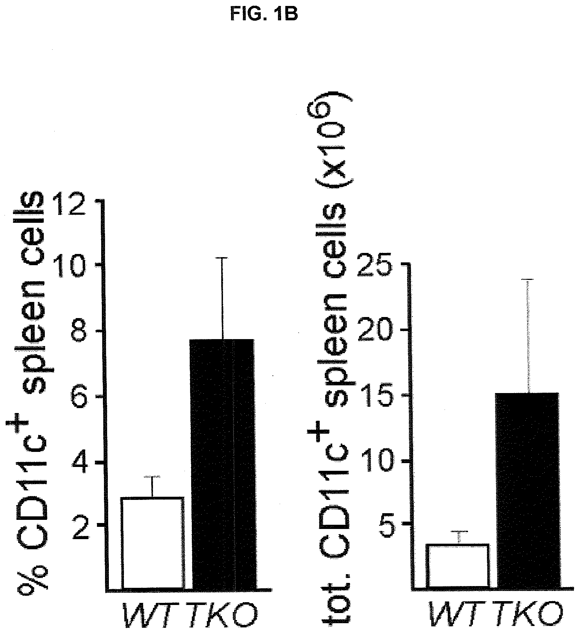

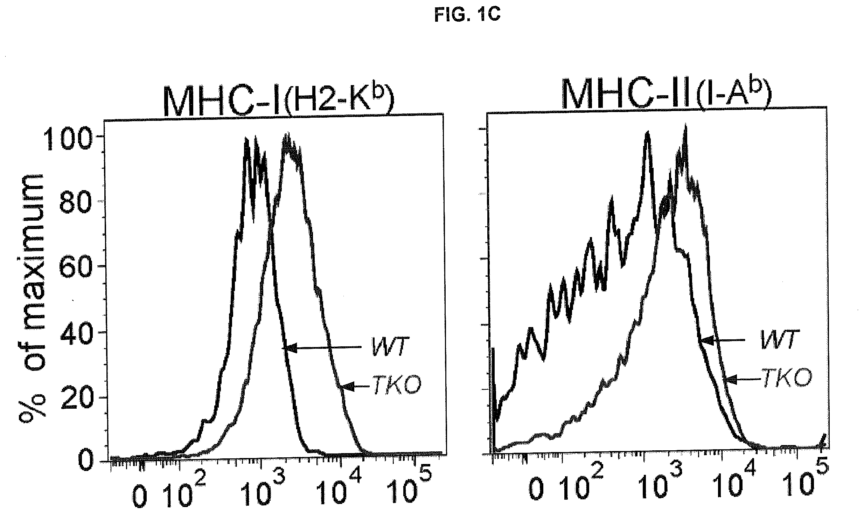

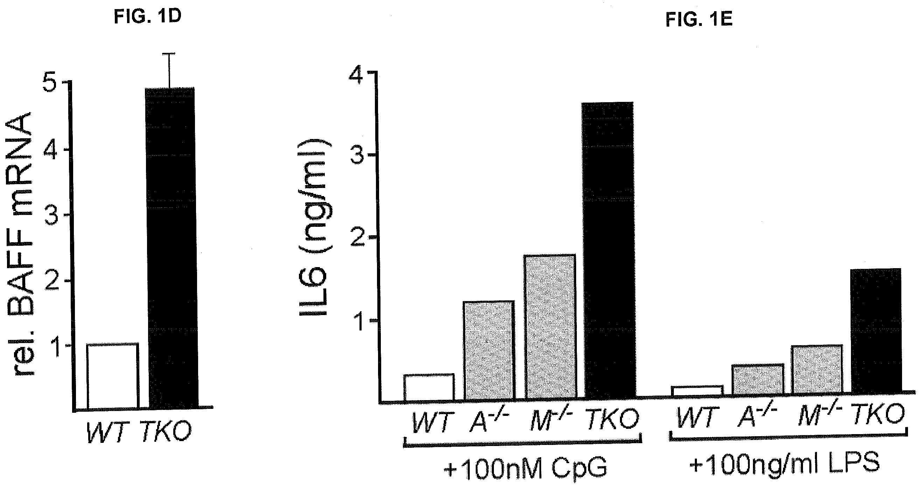

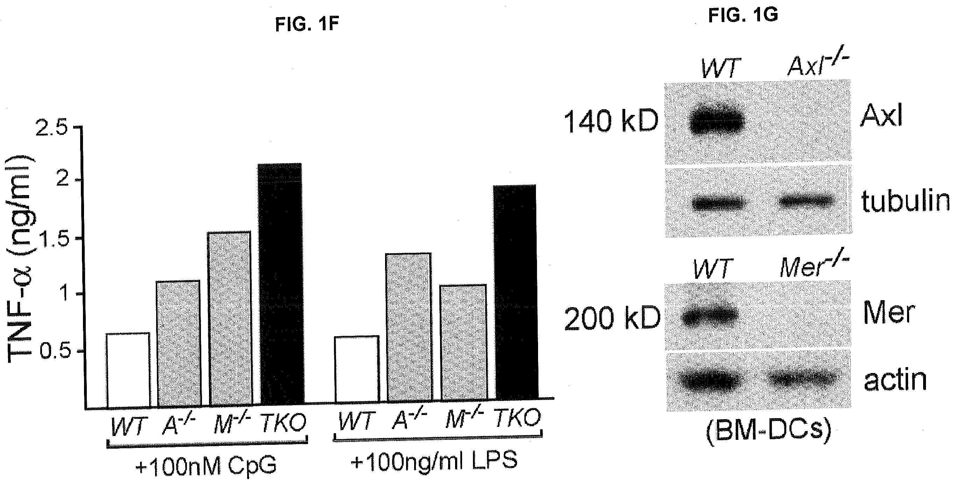

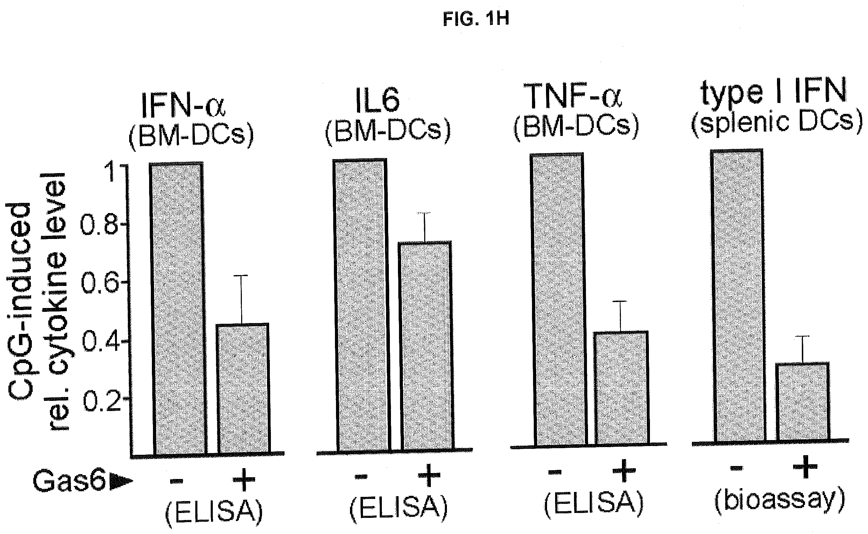

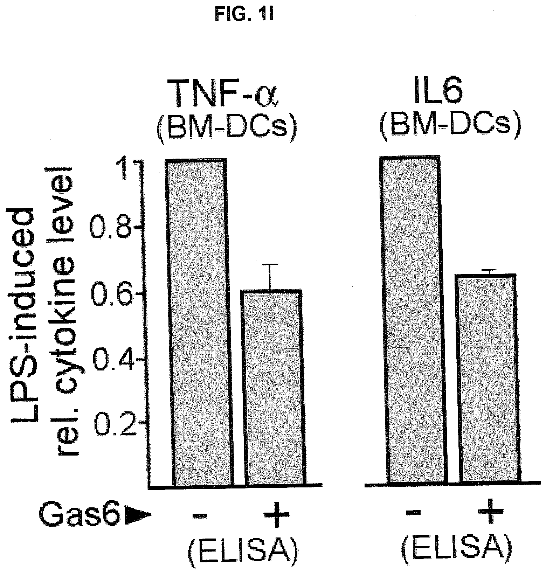

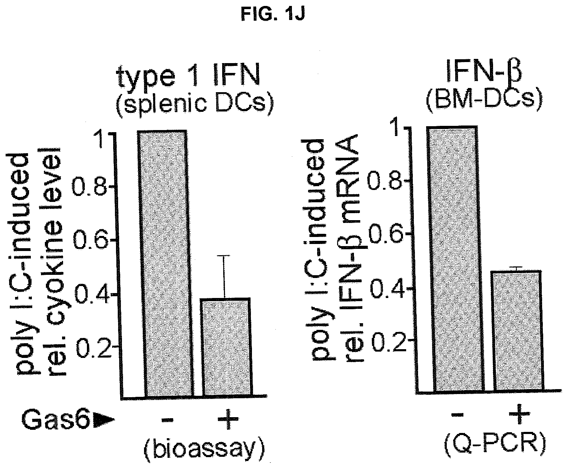

FIG. 1 includes several panels showing hyperactivation of dendritic cells (DCs) in *TAM* mutant mice and TAM inhibition of TLR-induced cytokine production. FIG. 1A shows representative FACS analyses of CD11c+ splenocytes from wild-type (WT) and TAM triple knockout (TKO) mice. Cells negative for NK1.1, CD19, and CD3 were gated for CD11c. Numbers inside boxed areas indicate percentage of cells in the gate. FIG. 1B shows the same protocol as that shown in FIG. 1A independently repeated, and with the data represented as bar graphs. Percentage (left) and total number (right) of CD11c+ cells in the spleen of WT and *TAM* TKO mice are shown. Error bars: mean ± S.D. (n=6 mice per group, p<0.01). FIG. 1C shows FACS histograms of MHC-I and MHC-II expression on CD11c+ cells from WT and *TAM TKO* spleens. Results depicted are representative of three independent experiments. FIG. ID shows the relative levels of BAFF mRNA expression in *TAM* TKO CD11c+ cells acutely isolated from the spleen, normalized to WT, as determined by quantitative PCR (Q-PCR). Error bars represent the mean ± S.D. (n=3, p<0.01). FIGS. 1E and 1F show IL6 and TNFα levels produced by WT, *Axl*^{*-*/}*⁻,* Mer^{-/-,} and *TAM* TKO spleen-derived CD11c+ cells following 15 hours of stimulation with the indicated TLR agonist, as determined by ELISA. Results depicted are representative of three independent studies. FIG. 1G shows Western blots of bone marrow-dendritic cell (BM-DC) cell lysates probed with Axl and tubulin (top blots) and Mer and actin (bottom blots) antibodies. Identical results were obtained with lysates prepared from CD11c+ cells acutely isolated from spleen. FIGS. 1H-1J show the relative production of the indicated cytokines following 15 hours of stimulation of DCs with 10 nM CpG (FIG. 1H), 10 ng/ml LPS (FIG. 1I) or 10 µg/ml Poly I:C (FIG. 1J), either alone (-) or concomitantly with (+) 50 nM Gas6. Cytokine production was measured as described below in Example 1. Results were normalized to the production of the corresponding cytokine in the presence of the TLR ligand alone. Error bars: mean ± S.D (n≥3 per group, *p*<0.01).

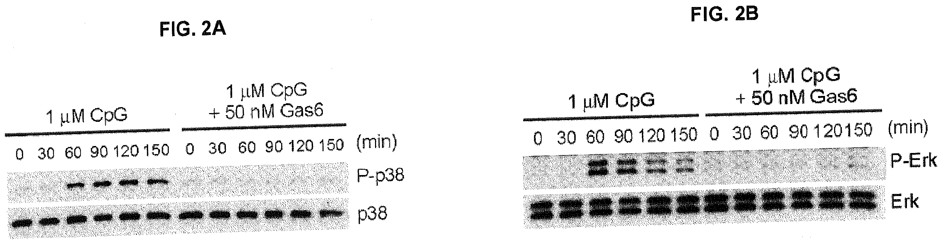

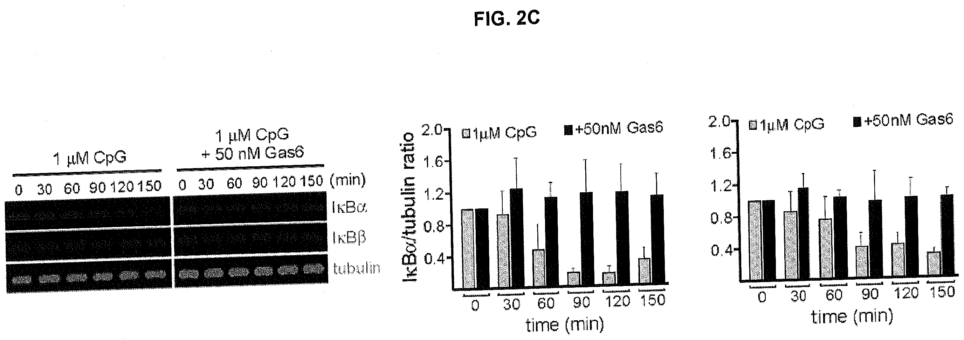

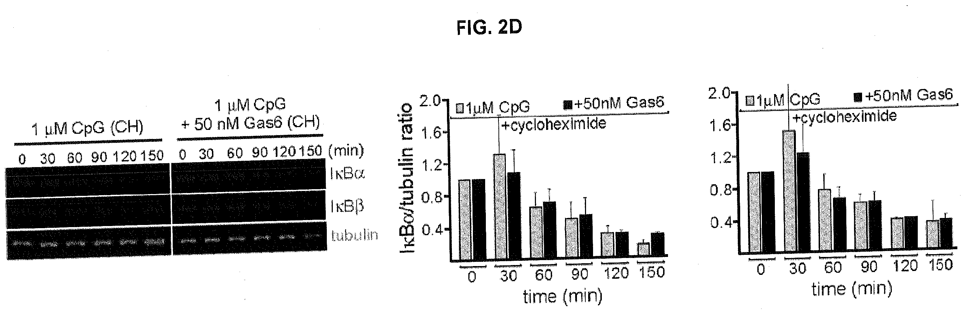

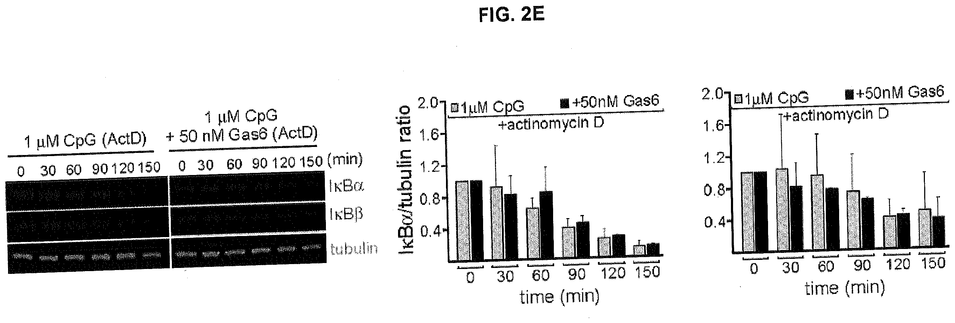

FIG. 2 includes several panels showing that TAM receptor activation inhibits conserved components of the TLR9 signaling pathway. Whole cell lysates were prepared from bone marrow derived dendritic cells (BM-DCs) activated for the indicated minutes with CpG alone, or after a 2-hour pre-incubation with 50 nM Gas6. FIG. 2A shows Western blots probed for activated, phospho-p38 Thr180/Tyr182 (top blot) and total p38 (bottom blot). FIG. 2B shows Western blots probed for activated, phospho-ERK 1/2 Thr183/Tyr185 (top blot) and total ERK 1/2 (bottom blot). FIGS. 2C-E show Quantitative Li-Cor Odyssey Western blots probed for IκBα, IκBβ, and tubulin (left), and measured IκBα/tubulin and IκBβ/tubulin signal ratios (right, mean ± S.D.; n=3). DCs were activated as described. In addition, DCs were pretreated with 10 µg/ml cycloheximide (FIG. 2D), or with 1 µg/ml actinomycin D (FIG. 2E), for 30 minutes prior to the addition of Gas6.

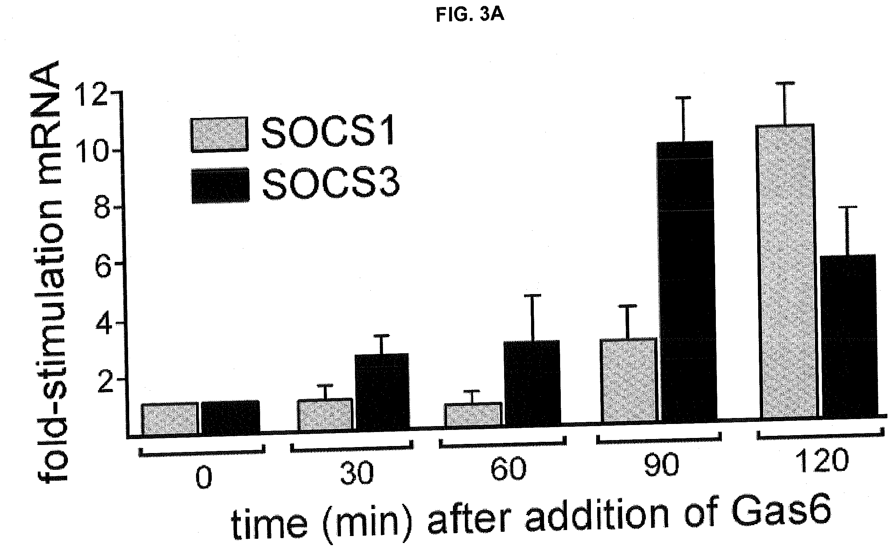

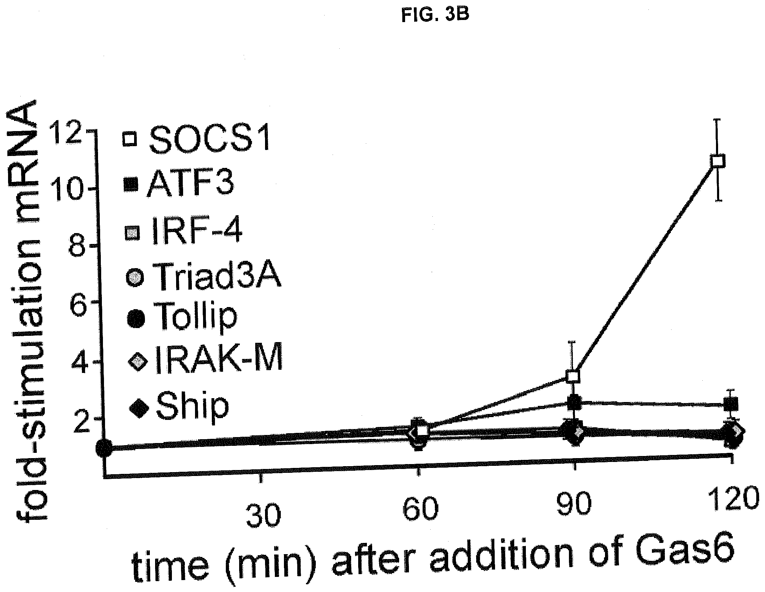

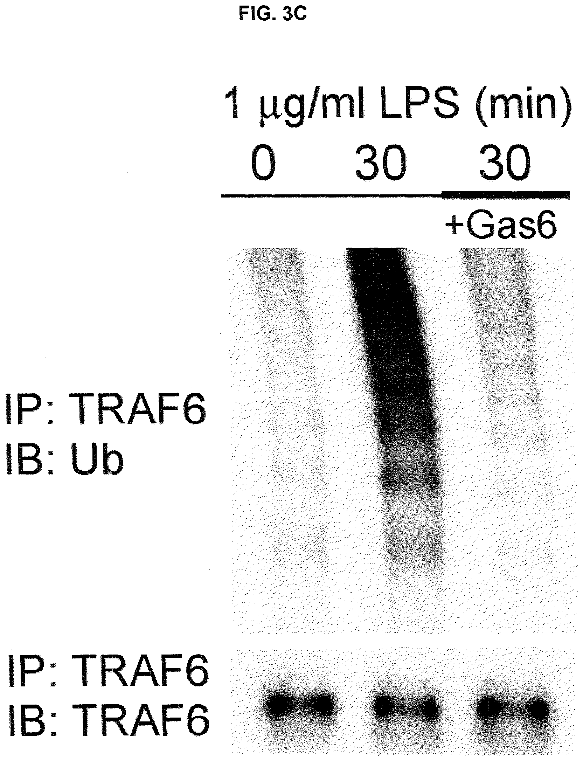

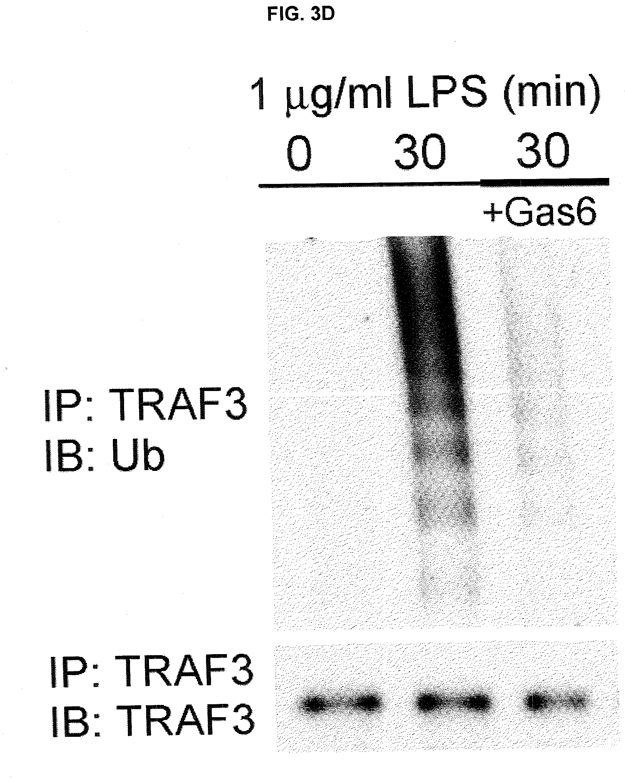

FIG. 3 includes several panels showing that TAM activation induces SOCS1/3 and inhibits TLR4-induced ubiquitylation of TRAF3/6. FIG. 3A is a bar graph showing the results of activating BM-DCs for the indicated time with 50 nM Gas6, and then assaying for expression of *SOCS1* and *SOCS3* mRNA by Q-PCR. *SOCS1*/*3* mRNA levels relative to β-actin were normalized to those of unstimulated cells. Error bars: mean ± S.D (n=4 for SOCS1; n=3 for SOCS3). FIG. 3B shows the results of treating the cells as in FIG. 3A, and then assaying for expression of the indicated inhibitor mRNAs by Q-PCR. Error bars represent the mean ± S.D (n=2). In FIG. 3C, BM-DCs were incubated for 0 or 30 minutes with 1 µg/ml LPS alone, or for 30 minutes with LPS after a 2-hour preincubation with 50 nM Gas6. TRAF6 was immunoprecipitated from whole cells lysates, and immunoprecipitates were analyzed by immunoblotting with ubiquitin (top blot) and TRAF6 (bottom blot) antibodies. In FIG. 3D, cells were treated as in FIG. 3C. TRAF3 was immunoprecipitated from whole cell lysates and its ubiquitylation was assessed by immunoblotting with ubiquitin (top blot) and TRAF3 (bottom blot) antibodies.

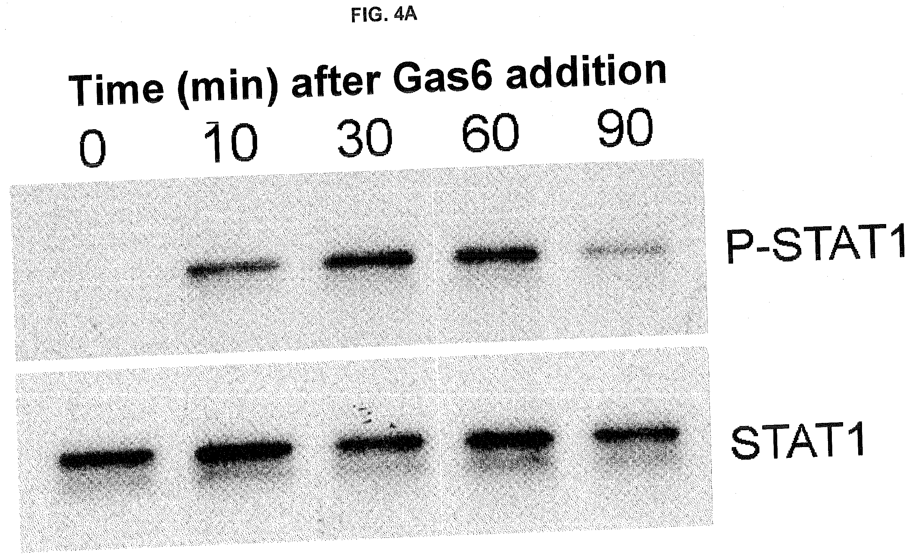

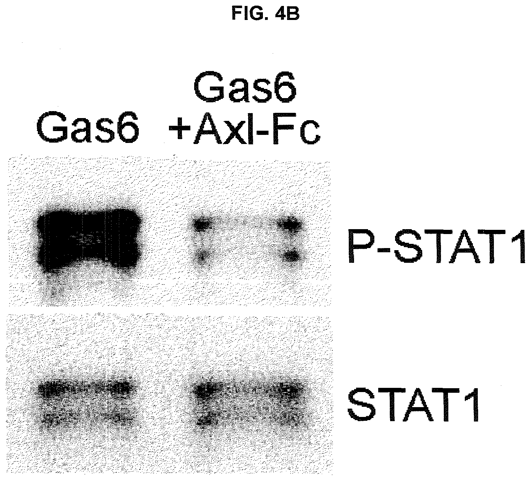

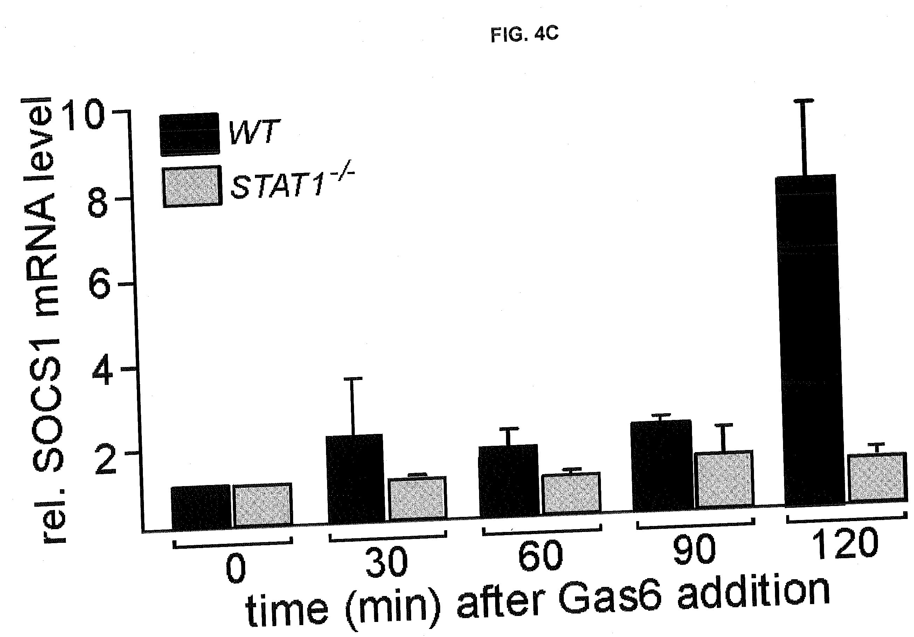

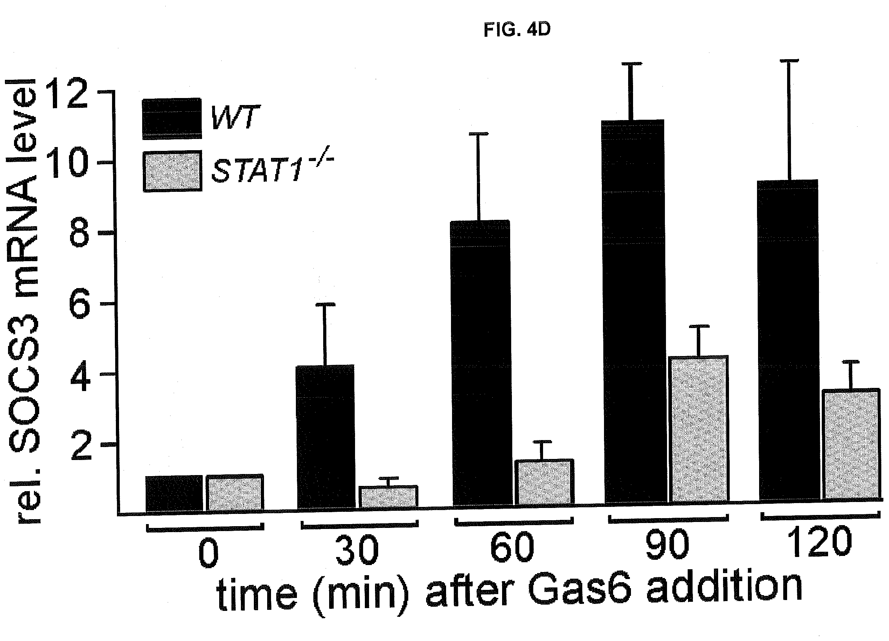

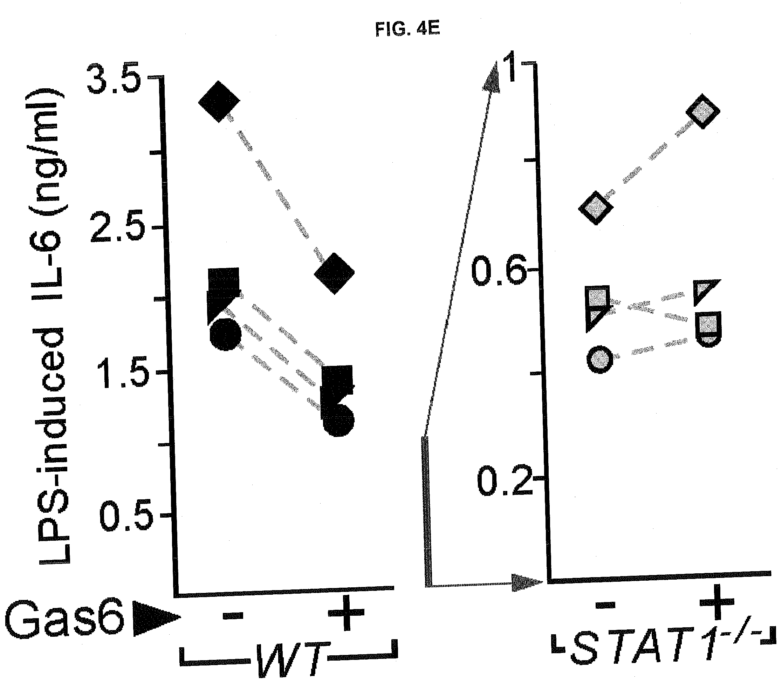

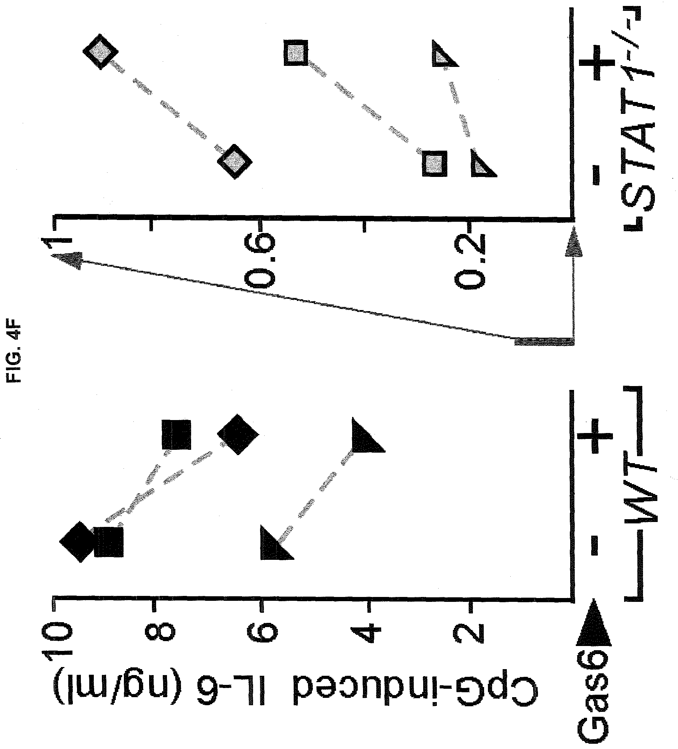

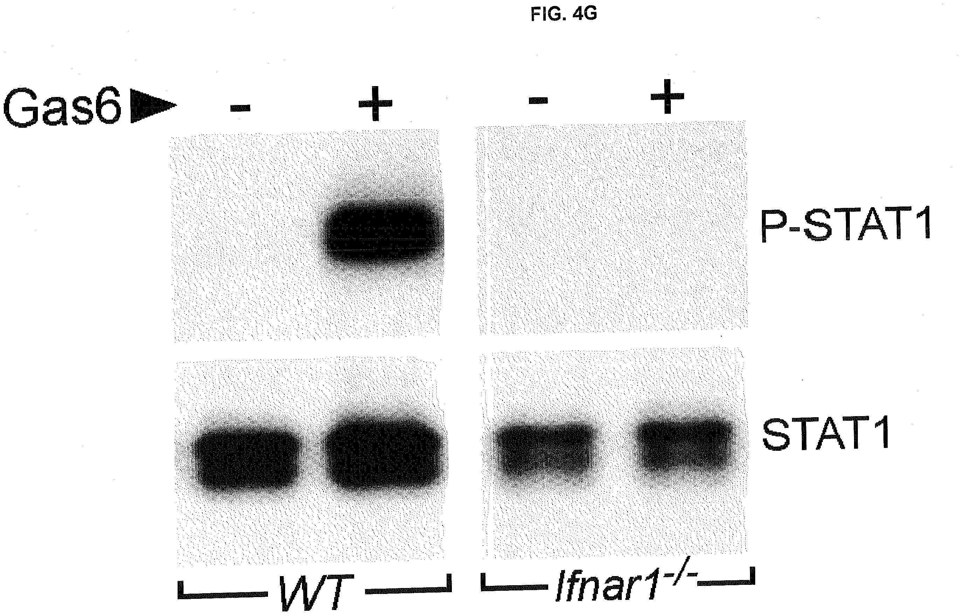

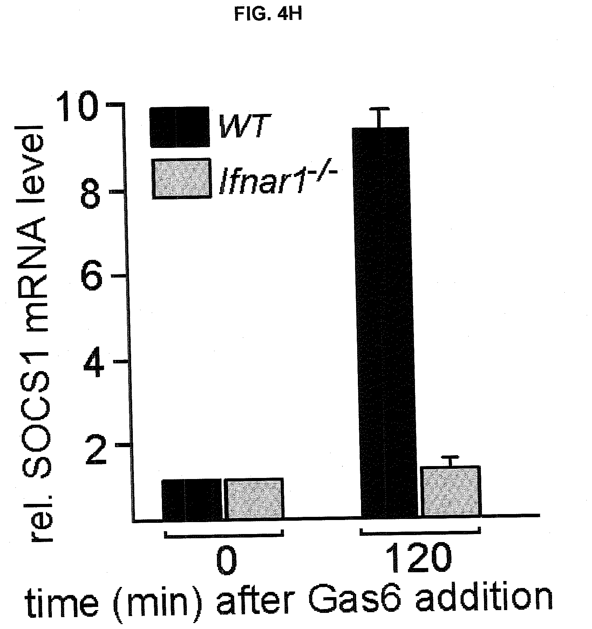

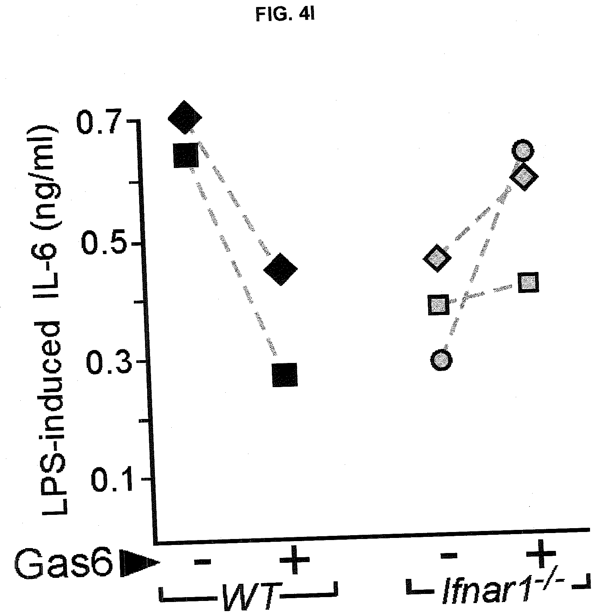

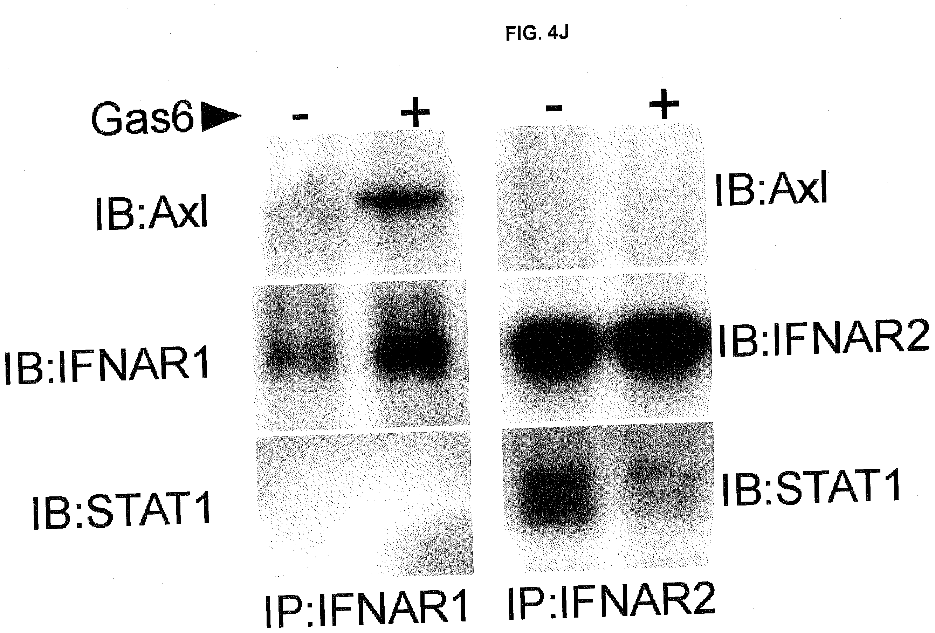

FIG. 4 includes several panels showing that IFNAR/STAT1 signaling is activated by TAM receptor activation, and is required for TAM induction of *SOCS* genes and inhibition of cytokine production. In FIG. 4A, splenic DCs were incubated for the indicated time with 50 nM Gas6, and whole cell lysates were then analyzed by immunoblotting for activated phospho-STAT1 Tyr 701 (top blot) and total STAT1 (bottom blot). In FIG. 4B, BM-DCs were incubated with 50 nM Gas6 alone or after a preincubation of 10 minutes with 100 nM Axl-FC. Whole cell lysates were then analyzed as in FIG. 4A. In FIGS. 4C and 4D, BM-DCs from *WT* and *STAT1* knock-out mice were incubated for the indicated time with 50 nM Gas6, and expression of *SOCS1* (FIG. 4C) and *SOCS3* (FIG. 4D) mRNAs was determined by Q-PCR. mRNA levels relative to β-actin expression were normalized to unstimulated cells. Error bars represent the mean ± S.D, n=2. In FIGS. 4E and 4F, BM-DCs from *WT* and *STAT1*^{*-*/*-*} mice were stimulated with 10 ng/ml LPS (FIG. 4E) or 3 nM CpG (FIG. 4F), either alone (-) or concomitantly with (+) 50 nM Gas6. After 15 hours, levels of secreted IL-6 were determined by ELISA, whose detection limit is approximately 4 pg/ml. Note the expansion of the y-axis for the *STAT1*^{*-*/*-*} measurements in FIGS. 4E and 4F. In FIG. 4G, whole cell lysates of BM-DCs from either WT (left) or IFNA receptor knock-outs (*Ifnar1*^{*-*/*-*}; right), either untreated (-) or treated with (+) 50 nM Gas6 for 60 minutes, were analyzed by immunoblotting for activated phospho-STAT1 (top blots) and total STAT1 (bottom blots). In FIG. 4H, BM-DCs from *WT* and *Ifnar1*^{*-*/*-*} mice were incubated for the indicated minutes with 50 nM Gas6, and expression of *SOCS1* mRNA was determined by Q-PCR. mRNA levels relative to β-actin expression were normalized to unstimulated cells. Error bars represent the mean ± S.D, n=2. In FIG. 4I, BM-DCs from *WT* and *Ifnar1*^{*-*/*-*} mice were stimulated with 10 ng/ml LPS, either alone (-) or concomitantly with (+) 50 nM Gas6. After 15 hours, levels of secreted IL-6 were determined by ELISA. In FIG. 4J, whole cell BM-DC lysates, either untreated (-) or treated for 20 minutes with 50 nM Gas6 (+), were immunoprecipitated with antibodies to the R1 (left blots) or R2 (right blots) chain of the type I IFN receptor. These immunoprecipitates were resolved on SDS gels, which were then immunoblotted with antibodies against Axl (top blots), the IFN receptor R1 chain (middle left blot), the IFN receptor R2 chain (middle right blot), or STAT1 (bottom blots). An equivalent association of Axl with the R1 chain of the IFN receptor was also observed in IPs of Axl blotted with anti-IFNARl.

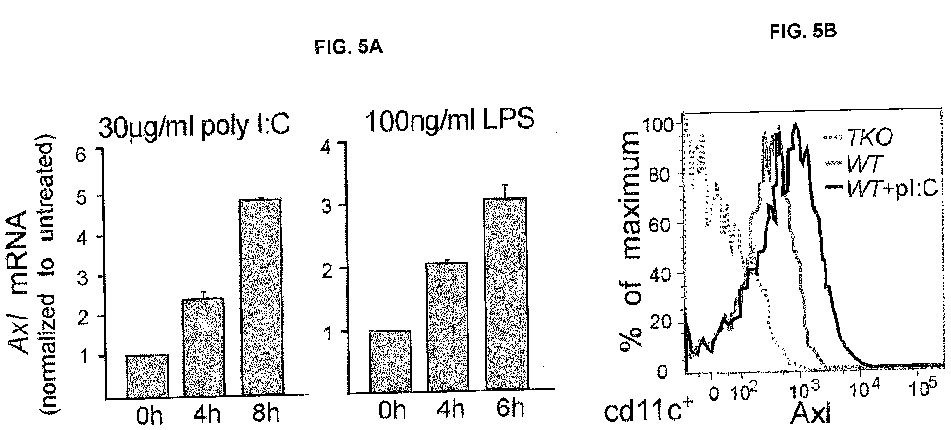

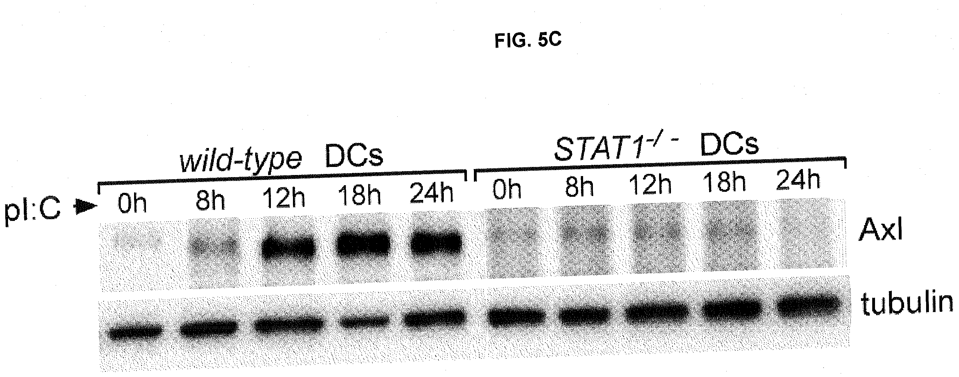

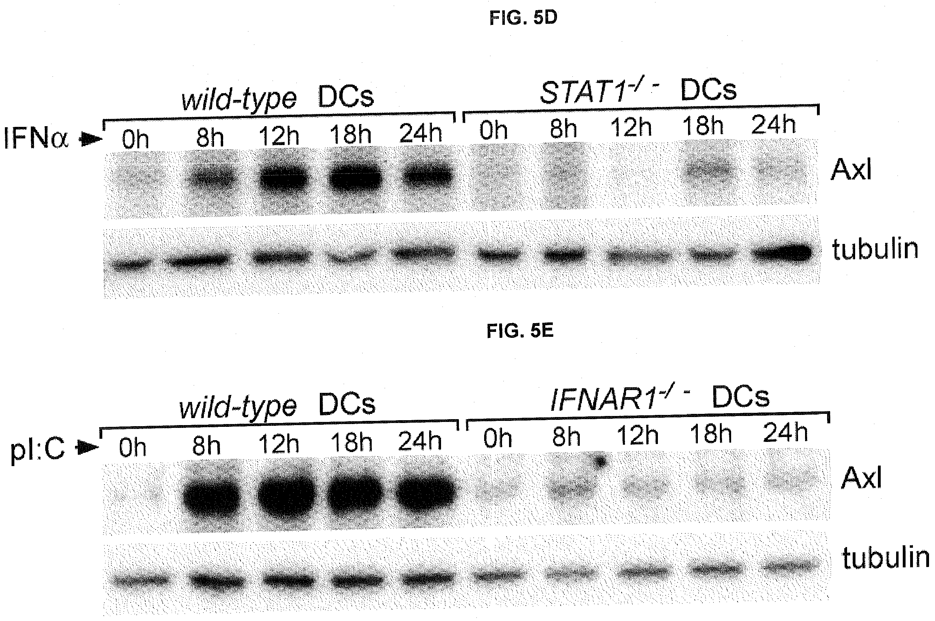

FIG. 5 includes several panels showing that TLR induction of TAM signaling is activated in an IFNAR/STAT1-dependent fashion. In FIG. 5A, BM-DCs were incubated for the indicated hours with 30 µg/ml Poly I:C or 100 ng/ml LPS and expression of *Axl* mRNA was then determined by Q-PCR. mRNA levels relative to β-actin expression were normalized to unstimulated cells. Error bars represent the mean ± S.D (n=2). FIG. 5B shows representative FACS histograms of Axl expression on unstimulated WT CD11c⁺ cells, CD11c⁺ cells stimulated with 30 µg/ml Poly I:C for 12 hours or in a *TAM* TKO control. In FIGS. 5C - 5E, BM-DCs - prepared from wild-type, *STAT1*^{*-*/}*⁻,* and *IFNAR*^{*-*/*-*} mice - were incubated for the indicated hours with 30 µg/ml Poly I:C (FIGS. 5C, 5E) or 3000 U/ml IFNα (FIG. 5D) and whole cell lysates were then analyzed by immunoblotting for Axl (top blots) and tubulin expression (bottoms blots). Wild-type controls are from strain 129 mice for FIGS. 5C and 5D, and from C57Bl/6 for FIG. 5E.

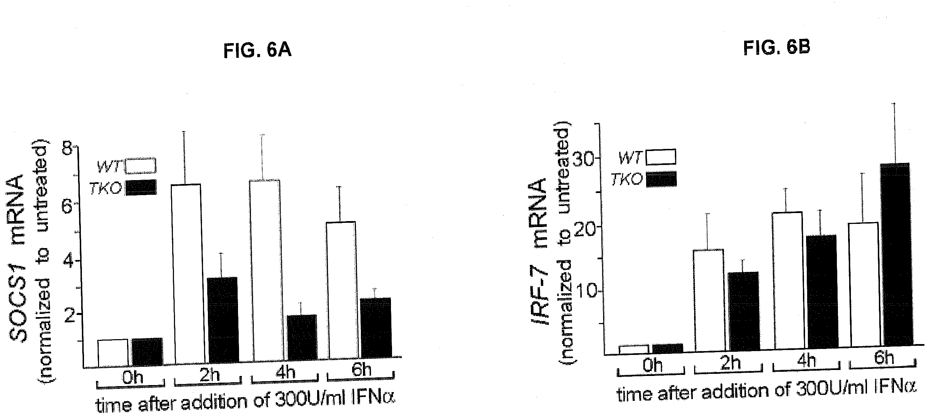

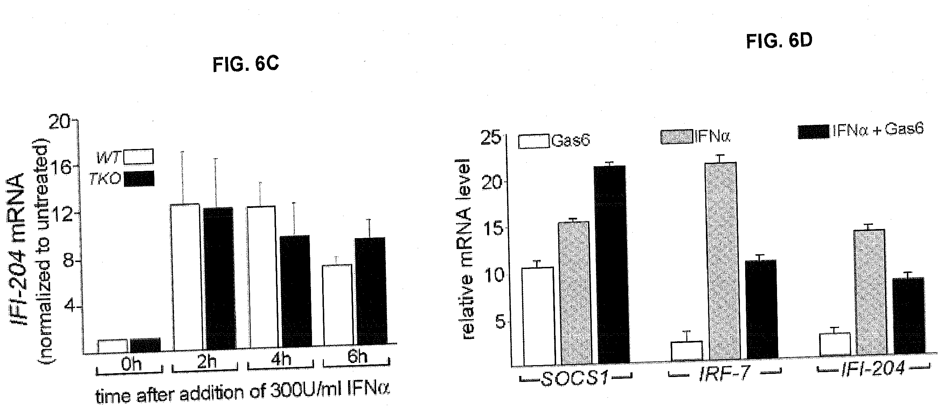

FIG. 6 includes several panels showing integration of TAM and IFN receptor signaling. In FIG. 6A, splenic CD11c⁺ cells from WT and TAM TKO mice were incubated for the indicated hours with 300 U/ml IFNα and expression of *SOCS1* mRNA was then determined by Q-PCR. mRNA levels relative to β-actin were normalized to unstimulated cells. Error bars represent the mean ± S.D (n=4). In FIGS. 6B and 6C, the same mRNA samples used in FIG. 6A were analyzed for expression of *IRF-7* mRNA (FIG. 6B) and *IFI-204* mRNA (FIG. 6C) by Q-PCR. mRNA levels relative to β-actin were normalized to unstimulated cells. Error bars represent the mean ± S.D (n=4). In FIG. 6D, RNA samples prepared from BM-DCs that were incubated for 2 hours with 50 nM Gas6, 30 U/ml IFNα, or Gas6 plus IFNα together were analyzed for expression *SOCS1* mRNA (left), *IRF-7* mRNA (middle), and *IFI-204* mRNA (right) by Q-PCR. mRNA levels relative to β-actin were normalized to unstimulated cells.

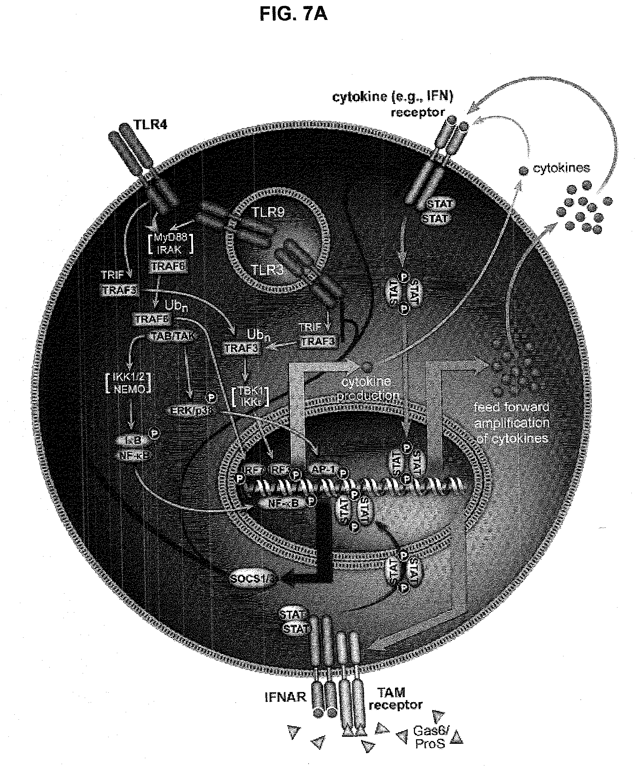

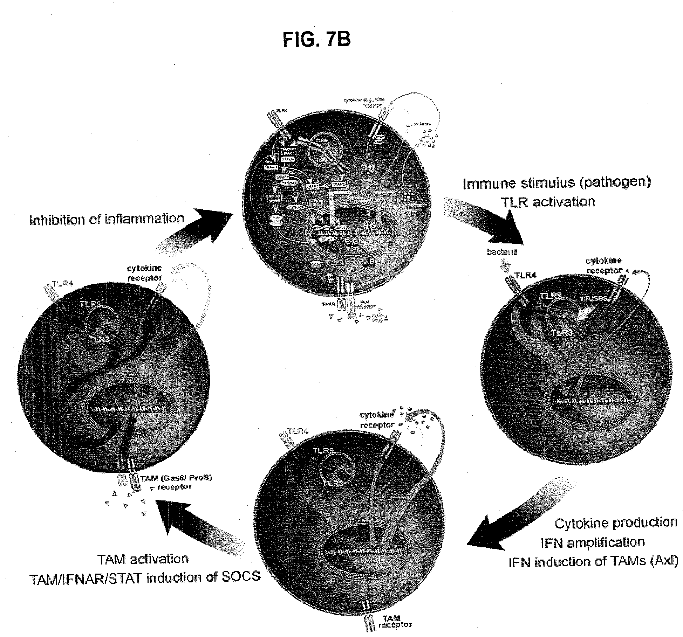

FIG. 7 includes several panels showing a cycle of inflammation and inhibition, regulated by TAM receptor signaling. FIG. 7A is a schematic representation of the sequential engagement of TLR, cytokine receptor, and TAM receptor signaling pathways in DCs. FIG. 7B is a schematic representation of the inflammatory cycle initiated by TLR ligation. Activation of TLRs leads to an initial burst of cytokines. This burst is then amplified in a second stage, via a feed-forward loop through cytokine receptors. At the same time, activation of cytokine receptors leads to an IFNAR/STAT1-dependent induction of Axl. The final stage of the inflammatory cycle involves the engagement of TAM signaling, the transcription of *SOCS* genes, and the pleiotropic inhibition of both cytokine receptor and TLR signaling pathways. This final TAM-driven inhibitory phase of the cycle is also dependent on the IFNAR/STAT1 signaling cassette, which is physically associated with TAM receptors.

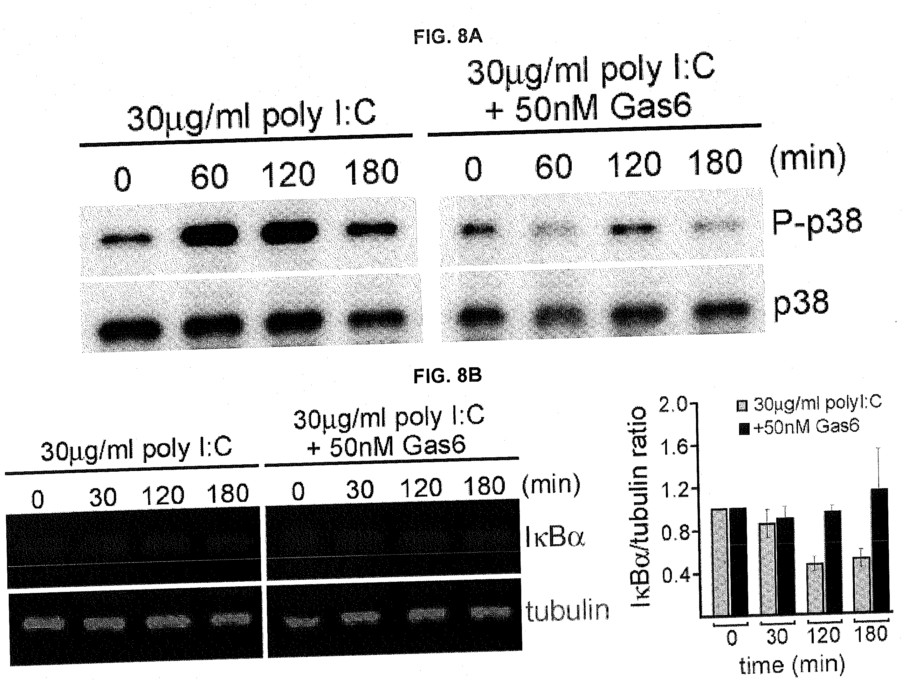

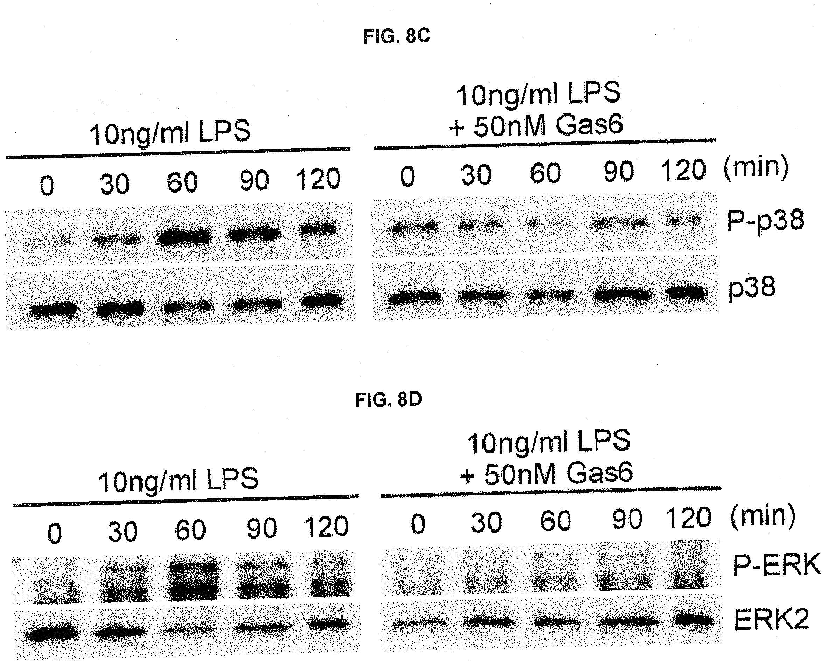

FIG. 8 includes several panels showing that TAM receptor activation by Protein S inhibits cytokine production and conserved components of the TLR9 signaling pathway. FIG. 8A shows a quantitative representation of the relative production of IFNα from DCs following 15 hours of stimulation with 10 nM CpG alone (-) or concomitantly with (+) 3 nM ProS. Cytokine production was measured as described in detail in Example 1. The results were normalized to the production of IFNα in the presence of CpG alone. Error bars represent the mean ± S.D (n=3 p, p<0.01). FIG. 8B shows Western blot analyses of the expression of phospho-p38 Thr180/Tyr182 (top blot), phospho-ERK 1/2 Thr183/Tyr185 (middle blot) and tubulin (lower blot) in whole cell lysates from BM-DCs activated for the indicated minutes with CpG alone or after a 2-hour pre-incubation with ProS. FIG. 8C shows quantitative Li-Cor Odyssey Western blot analyses of the expression of IκB 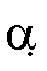 (top blot), IκB 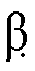 (middle blot) and tubulin (lower blot) in whole cell lysates as described in FIG. 8B. FIG. 8D shows bar graphs depicting IκBα̃/tubulin and IκBβ̃/tubulin signal ratios at the indicated minutes from western blot analyses shown in FIG. 8C.

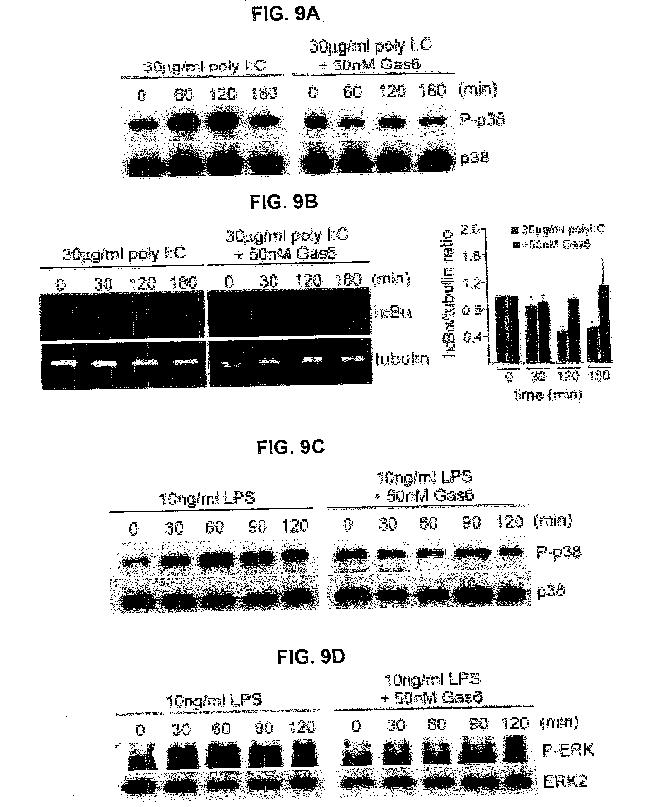

FIG. 9 includes several panels showing that TAM receptor activation inhibits TLR3 and TLR4 signaling pathways. FIG. 9A shows Western blot analyses of the expression of phospho-p38 Thr180/Thr182 (top blot) and total p38 (bottom blot) in whole cell lysates from BM-DCs activated for the indicated minutes with Poly I:C alone or after a 2-hour pre-incubation with Gas6. FIG. 9B shows quantitative Li-Cor Odyssey Western blot analyses of the expression of IκBα and tubulin, in whole cell lysates prepared as described in FIG. 9A. The bar graph on the right shows IκBα/tubulin signal ratios at the indicated time. (Error bars represent the mean ± S.D.; n=3). FIGS. 9C and 9D show Western blot analyses of the expression of phospho-p38 Thr180/Thr182 (top blot) and total p38 (bottom blot; FIG. 9C), and of phospho-ERK1/2 Thr183/Tyr185 (top blot) and total ERK 2 (bottom blot; FIG. 9D) in whole cell lysates from BM-DCs, activated for the indicated time with LPS alone or after a 2-hour pre-incubation with Gas6.

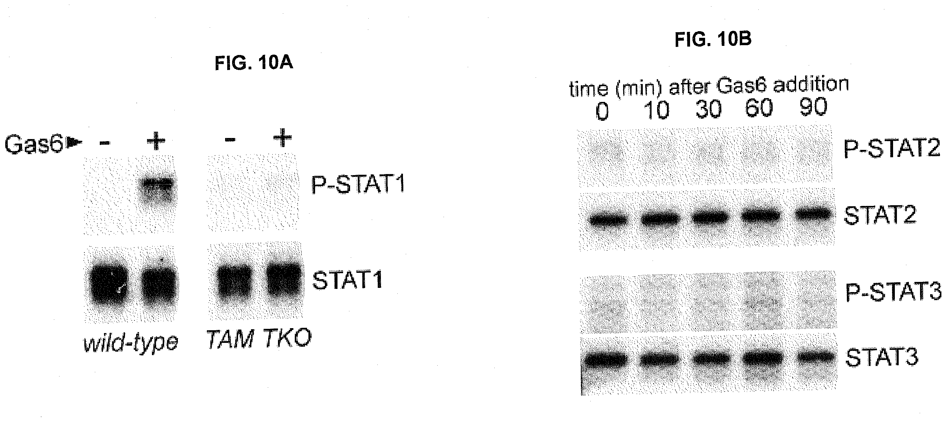

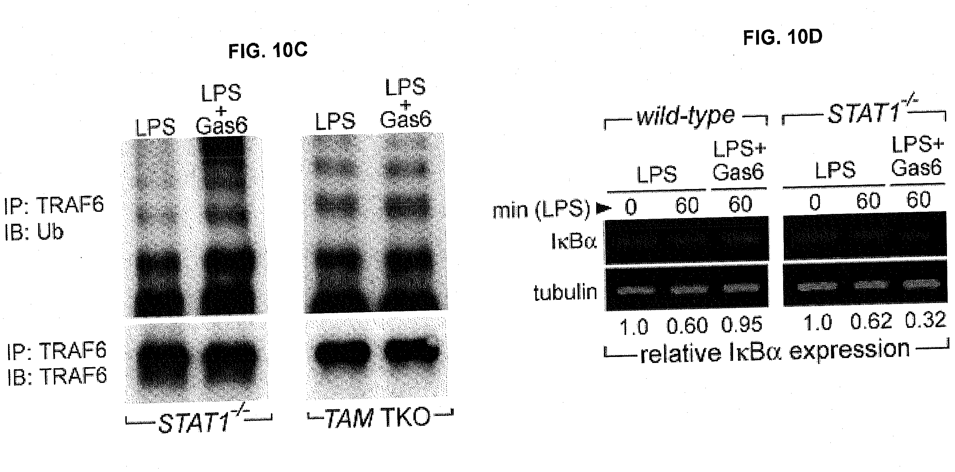

FIG. 10 includes several panels showing that TAM receptor inhibition of receptor proximal points in TLR signaling pathways requires STAT1, but does not activate STAT2 or STAT3. In FIG. 10A, BM-DCs prepared from wild-type (left blots) or *TAM* TKO mice (right blots) were incubated without (-) or with (+) 50 nM Gas6 for 60 minutes, and lysates of these cells were then analyzed by immunoblotting for expression of phospo-STAT1 (upper blots) and total STAT1 (lower blots). In FIG. 10B, splenic DCs were incubated for the indicated time with 50 nM Gas6, and whole cell lysates were then analyzed by immunoblotting for phospho-STAT2 (top blot), total STAT2 (second blot), phospho-STAT3 (third blot), and total STAT3 (bottom blot). In FIG. 10C, BM-DCs prepared from either *STAT1*^{*-*/*-*} (left blot) or *TAM* TKO mice (right blot) were incubated for 30 minutes with 1 µg/ml LPS alone, or for 30 minutes with LPS after a 2-hour preincubation with 50 nM Gas6. TRAF6 was immunoprecipitated from whole cells lysates, and immunoprecipitates were analyzed by immunoblotting with ubiquitin (top blots) and TRAF6 (bottom blots) antibodies. In FIG. 10D, whole cell lysates, prepared from either wild-type (left blots) or *STAT1*^{*-*/*-*} (right blots) BM-DCs and activated for the indicated minutes with LPS alone, or after a 2-hour pre-incubation with 50 nM Gas6, were analyzed by Li-Cor Odyssey Western blots probed for IκBα (top blots) and tubulin (bottom blots).

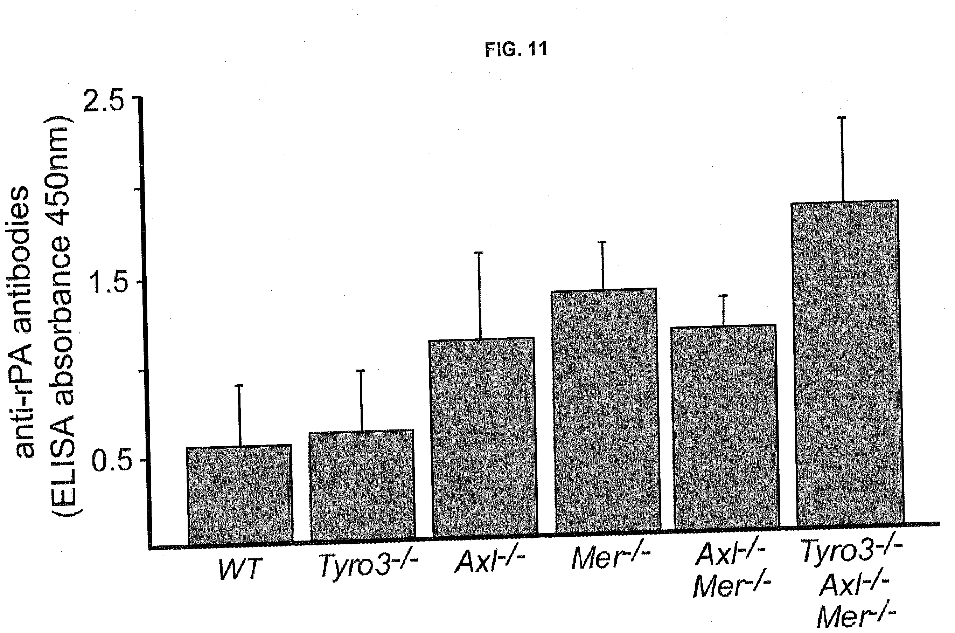

FIG. 11 is a bar graph showing TAM triple mutant mice (Tyro3^{-/-}AXL^{-/-} Mer^{-/-}) produce approximately 3.5-fold higher antibody titers at 2 weeks than wild-type (WT) mice following immunization. WT and various TAM single, double, and triple gene mutant mice (indicated) were immunized with recombinant protective antigen (rPA), the dominant immunogen in an anthrax vaccine, together with alum. Serum antibodies to rPA were assayed by ELISA at two weeks following initial immunization. (Mean antibody titer shown; n>5 mice in each cohort; error bars represent standard deviation.)

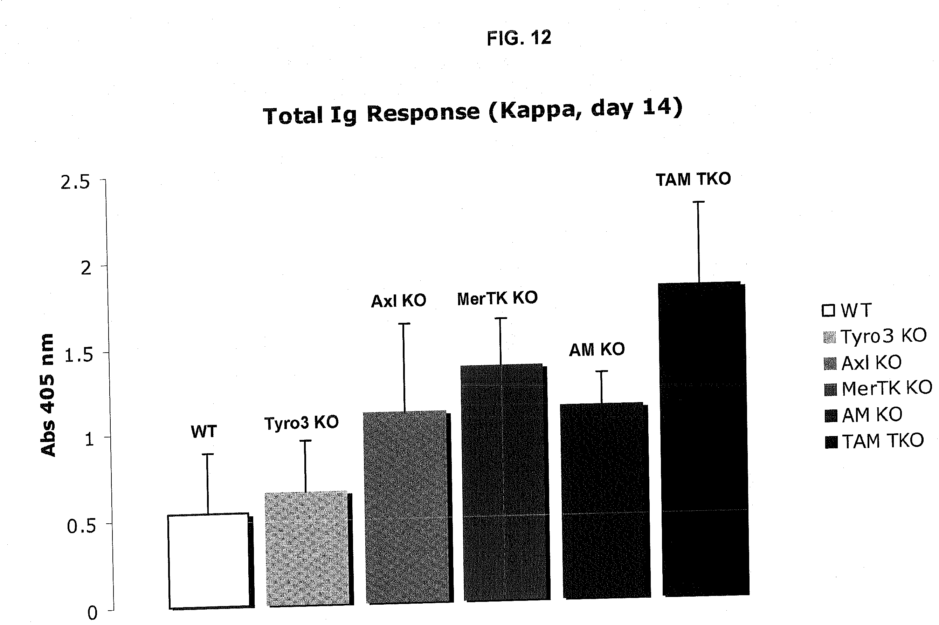

FIG. 12 is a bar graph showing that TAM triple mutant mice (Tyro3^{-/-}AXL⁻ "Mer^{-/-}) produce approximately 4-fold higher total IgG response (kappa) at 2 weeks than WT mice following immunization with 20 ug Anthrax Protective antigen.

### SEQUENCE LISTING

The nucleic acid sequences listed in the accompanying sequence listing are shown using standard letter abbreviations for nucleotide bases, as defined in 37 C.F.R. 1.822. Only one strand of each nucleic acid sequence is shown, but the complementary strand is understood as included by any reference to the displayed strand. In the accompanying sequence listing:
SEQ ID NO: 1 shows a Q-PCR forward primer for actin.
SEQ ID NO: 2 shows a Q-PCR reverse primer for actin.
SEQ ID NO: 3 shows a Q-PCR forward primer for AXL
SEQ ID NO: 4 shows a Q-PCR reverse primer for AXL.
SEQ ID NO: 5 shows a Q-PCR forward primer for BAFF (Blyss).
SEQ ID NO: 6 shows a Q-PCR reverse primer for BAFF (Blyss).
SEQ ID NO: 7 shows a Q-PCR forward primer for GAPDH.
SEQ ID NO: 8 shows a Q-PCR reverse primer for GAPDH.
SEQ ID NO: 9 shows a Q-PCR forward primer for IFN beta.
SEQ ID NO: 10 shows a Q-PCR reverse primer for IFN beta.
SEQ ID NO: 11 shows a Q-PCR forward primer for IRAK-M.
SEQ ID NO: 12 shows a Q-PCR reverse primer for IRAK-M.
SEQ ID NO: 13 shows a Q-PCR forward primer for SHIP.
SEQ ID NO: 14 shows a Q-PCR reverse primer for SHIP.
SEQ ID NO: 15 shows a Q-PCR forward primer for SOCS1.
SEQ ID NO: 16 shows a Q-PCR reverse primer for SOCS1.
SEQ ID NO: 17 shows a Q-PCR forward primer for SOCS3.
SEQ ID NO: 18 shows a Q-PCR reverse primer for SOCS3.
SEQ ID NO: 19 shows the nucleic acid sequence of CpG-ODN 1668.
SEQ ID NO 20: shows a Gla domain of human Gas6.
SEQ ID NO: 21: shows a Gla domain of human Protein S.

### DETAILED DESCRIPTION

### I. Overview

The present invention provides a TAM receptor agonist for use in a method of treating or preventing an autoimmune disease, an allergy, a graft-versus-host disease, or a transplant rejection by suppressing the immune response in a subject, wherein the TAM receptor agonist is an antibody that binds to an extracellular domain of the TAM receptor, and wherein the TAM receptor is Axl.

According to some embodiments of the invention, the TAM receptor agonist can cross-link and activate the TAM receptor in a TAM receptor dimer.

According to some embodiments of the invention, the method further comprises administering to the subject an immunosuppressive agent, thereby suppressing the immune response in the subject.

According to some embodiments of the invention, the TAM receptor agonist is administered to the subject substantially concurrently with the immunosuppressive agent.

According to some embodiments of the invention, the autoimmune disease is rheumatoid arthritis, Hashimoto's thyroiditis, pernicious anemia, Crohn's disease, ulcerative colitis, psoriasis, renal fibrosis, pulmonary fibrosis, hepatic fibrosis, Addison's disease, type I diabetes, systemic lupus erythematosus, dermatomyositis, Sjogren's syndrome, multiple sclerosis, myasthenia gravis, Reiter's syndrome, or Grave's disease.

Disclosed herein are methods that take advantage of the surprising discovery that TAM (Tyro 3, Axl, and Mer) receptor inhibitors are effective for immunoenhancement. In one aspect, methods are disclosed for enhancing an immune response in a subject. The method can include administering to a subject in need of immunoenhancement a therapeutically effective amount of a TAM receptor inhibitor, thereby enhancing the immune response in the subject. In certain examples of the method, the TAM receptor is Tyro3, Axl, or Mer. In particular examples, the TAM receptor inhibitor substantially or completely reduces the biological activity of the receptor, such as a reduction of at least 25%, at least 50%, at least 75%, at least 90%, or at least 95%. In certain examples, TAM receptor inhibitors (such as a small molecule inhibitor) bind to an intracellular domain of Tyro3, Axl, or Mer, such as an ATP binding site. In other examples, the TAM receptor inhibitor binds to an extracellular domain of the TAM receptor. In such examples, the inhibitor may interfere with binding of a TAM receptor ligand to the receptor or otherwise prevent complete activation of the receptor. Similarly, a TAM receptor inhibitor may bind to a TAM receptor ligand, such as Gas6 or Protein S, and thereby interfere with the binding of the ligand to the extracellular domain of the TAM receptor and/or activation of the TAM receptor. In yet other examples, a TAM receptor inhibitor is one that decreases or inhibits expression of a TAM receptor or ligand thereof (*e.g*., RNAi molecules). Exemplary TAM receptor inhibitors include small molecule inhibitors, antibodies, and siRNA. In yet more particular examples, the TAM inhibitor is MP470, SGI-AXL-277, AXL-1, AXL-2, AXL-3, AXL-4, AXL-5, AXL-6, AXL-7, AXL-8, or AXL-9 or derivatives thereof. In certain examples, the TAM receptor inhibitor has an IC₅₀ of less than about 50 µM, and in even more particular examples, the TAM receptor inhibitor has an IC₅₀ of from about 1 pM to about 5 µM.

Some examples of the disclosed immunoenhancement methods also include administering to the subject a therapeutically effective amount of a vaccine, thereby enhancing the immune response to the vaccine. In some examples, the TAM receptor inhibitor is administered to the subject substantially concurrently with the vaccine, and in particular examples, the vaccine includes dendritic cells (DCs) activated against a tumor in the subject.

Other examples of the disclosed immunoenhancement methods are methods of treating a tumor in a subject, methods of treating a disease of immunosuppression, and methods of treating sepsis, for instance, septic shock, severe inflammatory response syndrome (SIRS), or severe SIRS. In some examples, the method is a method of treating an infection in a subject in need thereof, and in particular examples, the subject is immunocompromised or immunosuppressed.

Also disclosed herein is the surprising discovery that TAM receptor agonists are useful for immunosuppression, for example, for the treatment or prevention of autoimmune diseases, allergies, graft-versus-host disease or transplant rejection. One embodiment of the disclosure is a TAM receptor agonist for use in a method of suppressing an immune response in a subject. The method includes administering to a subject in need of immunosuppression a therapeutically effective amount of a TAM receptor agonist, thereby suppressing the immune response in the subject. In particular examples, the TAM receptor agonist substantially increases the biological activity of the receptor, such as an increase of at least 25%, at least 50%, at least 75%, at least 90%, at least 100%, or at least 200%. In certain examples, the TAM receptor is Tyro3, Axl, or Mer. Exemplary TAM receptor agonists include TAM ligands, such as GAS6 and Protein S, as well as an amino-terminally truncated forms of GAS6 and Protein S, such as those that do not have a Gla or EGF domain or have a partial Gla or EGF domain. Other exemplary TAM receptor agonists include those agents that can specifically bind to an extracellular domain of the TAM receptor and activate the receptor, for example by crosslinking the receptor into a TAM receptor dimer. In certain examples, the TAM agonist has an EC₅₀ of less than about 50 µM, for instance, from about 1 pM to about 5 µM.

Other examples of the disclosed immunosuppression methods further include administering to the subject a therapeutically effective amount of an additional immunosuppressive agent, thereby suppressing the immune response in the subject. In some examples, the TAM receptor agonist is administered to the subject substantially concurrently with the immunosuppressive agent. In certain examples, the subject is immunocompromised.

The invention provides a TAM receptor agonist for use in methods of treating an autoimmune disease in a subject, in a method of treating an allergy, in a method of treating graft-versus-host disease or in a method of treating or preventing a transplant rejection. In some embodiments, the autoimmune disease is rheumatoid arthritis, Hashimoto's thyroiditis, pernicious anemia, Crohn's disease, ulcerative colitis, psoriasis, renal fibrosis, pulmonary fibrosis, hepatic fibrosis, Addison's disease, type I diabetes, systemic lupus erythematosus, dermatomyositis, Sjogren's syndrome, multiple sclerosis, myasthenia gravis, Reiter's syndrome, or Grave's disease. In still other embodiments, the method is a method of treating graft-versus-host disease in a subject in need thereof or a method of treating transplant rejection in a subject at risk of rejecting a transplant.

Also disclosed are methods of screening for an immunomodulator. Some examples of the method include contacting a cell expressing a TAM receptor with a test agent; and determining whether the test agent alters TAM receptor activity or the test agent inhibits binding of a ligand to the TAM receptor. Test agents that increase TAM receptor activity or enhance binding of a ligand to the TAM receptor are identified as immunosuppressive agents and wherein test agents that inhibit TAM receptor activity or reduce or inhibit binding of a ligand to the TAM receptor are identified as immunoenhancing agents. In certain examples, the TAM receptor is Tyro3, Axl, or Mer. In some examples, the method can include selecting a test agent indicated to be an immunoenhancing agent or an immunosuppressive agent for further analysis. In particular examples, the cell is in a laboratory mammal, and contacting the cell with the test agent includes administering the test agent to the mammal.

In some examples, the method is a method of screening for an immunoenhancing agent and determining whether the test agent significantly reduces or inhibits TAM receptor activity. The method can include contacting the cell with the test agent and determining whether the test agent: i. alters TAM autophosphorylation as compared to a control (such as a cell not contacted with the test agent), wherein a significant reduction in TAM autophosphorylation in the presence of the test agent relative to the control indicates that the test agent significantly reduces or inhibits TAM receptor activity; ii. alters TLR-induced cytokine production as compared to a control; wherein an increase in TLR-induced cytokine production in the presence of the test agent relative to the control indicates that the test agent inhibits TAM receptor activity; iii. alters TLR-induced stimulation of MAP kinase activation as compared to a control (such as a cell not contacted with the test agent), wherein an increase in TLR-induced stimulation of MAP kinase activation in the presence of the test agent relative to the control indicates that the test agent significantly reduces or inhibits TAM receptor activity; and/or iv. alters TLR-induced NF-kB activation as compared to a control (such as a cell not contacted with the test agent); wherein an increase in TLR-induced NF-kB activation in the presence of the test agent relative to the control indicates that the test agent significantly reduces or inhibits TAM receptor activity. In some examples, the control is a value obtained for a cell not contacted with the test agent or is a reference value (or range of values) expected when TAM receptor activity is increased, decreased, or unchanged.

In still other examples, the method is a method of screening for an immunosuppressive agent and determining whether the test agent increases TAM receptor activity. The method can include contacting the cell with the test agent and determining whether the test agent: i. alters TAM autophosphorylation as compared to a control level of TAM autophosphorylation, wherein an increase in TAM autophosphorylation in the presence of the test agent relative to a control indicates that the test agent increases TAM receptor activity; ii. alters TLR-induced cytokine production as compared to a control, wherein a decrease in TLR-induced cytokine production in the presence of the test agent relative to a control indicates that the test agent increases TAM receptor activity; iii. alters TLR-induced stimulation of MAP kinase activation as compared to a control, wherein decrease in TLR-induced stimulation of MAP kinase activation in the presence of the test agent relative to a control indicates that the test agent increases TAM receptor activity; and/or iv. alters TLR-induced NF-kB activation as compared to a control, wherein a decrease in TLR-induced NF-kB activation in the presence of the test agent relative to the control indicates that the test agent increases TAM receptor activity. In some examples, the control is a value obtained for a cell not contacted with the test agent or is a reference value (or range of values) expected when TAM receptor activity is increased, decreased, or unchanged.

In still further examples, the method can include determining if the test agent alters TAM receptor activity by (a) determining a control level of SOCS1 and SOCS 3 expression before contacting the cell with the test agent; (b) contacting the cell with the test agent; and (c) determining whether contacting the cell with the test agent alters SOCS1 and SOCS 3 expression as compared to the control level of SOCS1 and SOCS 3 expression. A reduction in SOCS1 and SOCS 3 expression in the presence of the test agent relative to the control level indicates that the test agent inhibits TAM receptor activity and such test agents are identified as immunoenhancing agents. An increase in SOCS1 and SOCS 3 expression in the presence of the test agent relative to the control level indicates that the test agent increases TAM receptor activity and such test agents are identified as immunosuppressive agents.

In certain examples, the method is a method of screening for an immunosuppressive agent and contacting the cell expressing the TAM receptor with the test agent includes contacting the cell with the test agent in the presence of a TAM receptor agonist. For example, a TAM receptor agonist, such as a TAM receptor ligand (*e.g.,* Gas6 or Protein S), amino-terminally-truncated ligands (*e.g.,* Gas6 or Protein S not having a full or only a partial Gla or EGF domain), or an antibody that binds to the extracellular domain of the TAM receptor and crosslinks and activates the TAM receptor in a TAM receptor dimer can be used.

### II Abbreviations

- AIDS: acquired immunodeficiency syndrome
- APC: antigen-presenting cell
- BM: bone marrow
- BSA: bovine serum albumen
- CMV: cytomegalovirus
- DC: dendritic cell
- DTT: dithiothreitol
- ELISA: enzyme-linked immunosorbent assay
- FACS: fluorescence-activated cell sorting
- FIV: feline immunodeficiency virus
- GAS6: growth-arrest-specific protein 6
- HIB: *Haemophilus influenzae* B
- HIV: human immunodeficiency virus
- HPV: human papiloma virus
- HSV: herpes simplex virus
- HZV: herpes zoster
- IFN: interferon
- IL: interleukin
- IRF: interferon response factor
- LDS: lithium dodecyl sulfate
- NHL: non-Hodgkin's lymphoma
- OHL: oral hairy leukoplakia
- PCP: *Pneumocystis Carinii* pneumonia
- PML: progressive multifocal leukoencephalopathy
- PS: phosphatidylserine
- PTK: protein-tyrosine kinase
- PVDF: polyvinyldifluoride
- Q-PCR: quantitative polymerase chain reaction
- RIA: radioimmunoassay
- rPA: recombinant protective antigen
- SCID: severe combined immune deficiency
- SDS: sodium dodecyl sulfate
- SHBG: sex hormone binding globulin
- SIRS: Severe Inflammatory response syndrome
- SIV: simian immunodeficiency virus
- SLE: systemic lupus erythematosus
- SOCS: suppressor of cytokine signaling
- TAM: Tyro 3, Axl, and Mer
- *TAM* TKO: *TAM* triple mutant
- TBS: tris buffered saline
- TLR: Toll-like receptor
- TNF: tumor necrosis factor
- TRAF: TNF receptor associated factor
- WT: wild-type

### III Terms

In order to facilitate review of the various embodiments of the disclosure, the following explanations of specific terms are provided:
**Adjuvant:** A vehicle or component of a vehicle used to enhance antigenicity, for example in a subject to whom it is administered. Specific, non-limiting examples of adjuvants include a suspension of minerals (*e.g*., alum, aluminum hydroxide, or phosphate) on which antigen is adsorbed; a water-in-oil emulsion in which antigen solution is emulsified in mineral oil (for instance, Freund's incomplete adjuvant), sometimes with the inclusion of killed mycobacteria (for instance, Freund's complete adjuvant) to further enhance antigenicity (substantially reduces or inhibits degradation of antigen and/or causes influx of macrophages); and immunstimulatory oligonucleotides, such as those including a CpG motif (for example see U.S. Patent No. 6,194,388; U.S. Patent No. 6,207,646; U.S. Patent No. 6,214,806; U.S. Patent No. 6,218,371; U.S. Patent No. 6,239,116; U.S. Patent No. 6,339,068; U.S. Patent No. 6,406,705; and U.S. Patent No. 6,429,199). Other non-limiting examples of adjuvants include keyhole limpet hemocyanin (KLH), bacillus Calmette Guerin (BCG), Interleukin - 2 (IL-2), Granulocyte Monocyte-Colony Stimulating Factor (GM-CSF), QS21, Montanide ISA-51, and, as described herein, TAM receptor inhibitors.

**Administration:** Includes both oral and parenteral administration, as well as vaginal and rectal administration. Generally, parenteral formulations are those that are administered through any possible mode except ingestion. This term also refers to injections, whether administered intravenously, intrathecally, intramuscularly, intraperitoneally, intra-articularly, or subcutaneously, and various surface applications including intranasal, inhalational, intradermal, and topical application, for instance.

**Allergy:** A collection of symptoms caused by an exaggerated immune response or reaction to substances that do not trigger an immune response in most people, and thus is an example of an immune-mediated disorder. The term "allergy" has become synonymous with Type I hypersensitivity (IgE-mediated allergy). Four different types of hypersensitivity were described by Coomb and Gell (Types I, II, III and IV), as a pedagogical way to increase the understanding of different immune reactions which could be provoked by many antigens. In practice, these types do not necessarily occur in isolation from each other.

Allergic diseases generally begin in childhood, although they can arise at any age. Development of allergic disease is associated with an allergic constitution due to heredity and to environmental and health factors. An allergic response involves an increased production of allergen-specific IgE antibodies, which may lead to clinical symptoms such as rhinitis, asthma, eczema, colic pains, or diarrhea. A state of hyperreactivity often accompanies an allergic reaction. If this hyperreactivity occurs in the respiratory tract, everyday stimuli like dust, tobacco smoke, cold air and perfumes may lead to allergy-like symptoms.

**Anti-infectious agent:** A substance (such as a chemical compound, protein, antisense or RNAi oligonucleotide, or other molecule) of use in treating infection of a subject. Anti-infectious agents include, but are not limited to, anti-fungal compounds, anti-viral compounds, and antibiotics. Antibiotics include, but are not limited to, amoxicillin, clarithromycin, cefuroxime, cephalexin ciprofloxacin, doxycycline, metronidazole, terbinafine, levofloxacin, nitrofurantoin, tetracycline, and azithromycin. Anti-fungal compounds include, but are not limited to, clotrimazole, butenafine, butoconazole, ciclopirox, clioquinol, clioquinol, clotrimazole, econazole, fluconazole, flucytosine, griseofulvin, haloprogin, itraconazole, ketoconazole, miconazole, naftifine, nystatin, oxiconazole, sulconazole, terbinafine, terconazole, tioconazole, and tolnaftate. Anti-viral compounds include, but are not limited to, zidovudine, didanosine, zalcitabine, stavudine, lamivudine, abacavir, tenofovir, nevirapine, delavirdine, efavirenz, saquinavir, ritonavir, indinavir, nelfinavir, saquinavir, amprenavir, and lopinavir. Anti-infectious agents also include hyper-immune globulin. Hyperimmune globulin is gamma globulin isolated from a donor, or from a pool of donors, that have been immunized with a substance of interest. Specifically, hyper-immune globulin is antibody purified from a donor who was repeatedly vaccinated against a pathogen.

**Arthritis:** An inflammatory disease that affects the synovial membranes of one or more joints in the body. It is the most common type of joint disease, and it is characterized by the inflammation of the joint. The disease is usually oligoarticular (affects few joints), but may be generalized. The joints commonly involved include the hips, knees, lower lumbar and cervical vertebrae, proximal and distal interphangeal joints of the fingers, first carpometacarpal joints, and first tarsometatarsal joints of the feet.

One type of arthritis is reactive arthritis, which is an acute nonpurulent arthritis secondary to a urinary tract or gastrointestinal infection with a variety of microorganisms, including *Chlamydia trachomatis, Yersinia, Salmonella, Shigella,* and *Campylobacter.* Microbial components are found in the affected joints. The arthritis appears abruptly and tends to involve the knees and ankles, but sometimes involves the wrists, fingers, and/or toes. Untreated, the arthritis lasts for about a year, then generally abates and only rarely is accompanied by ankylosing spondylitis. Despite evidence of disease being triggered by bacterial infection, viable bacteria are rarely present in affected joints and antibiotic treatment seldom provides relief.

Another type of arthritis is rheumatoid arthritis. Rheumatoid arthritis is a chronic, systemic, inflammatory disease that affects the synovial membranes of multiple joints in the body. Because the disease is systemic, there are many extra-articular features of the disease as well. For example, neuropathy, scleritis, lymphadenopathy, pericarditis, splenomegaly, arteritis, and rheumatoid nodules are frequent components of the disease. In most cases of rheumatoid arthritis, the subject has remissions and exacerbations of the symptoms. Rheumatoid arthritis considered an autoimmune disease that is acquired and in which genetic factors appear to play a role.

**Autoimmune disorder:** A disorder in which the immune system produces an immune response (for instance, a B cell or a T cell response) against an endogenous antigen, with consequent injury to tissues. For example, rheumatoid arthritis is an autoimmune disorder, as are Hashimoto's thyroiditis, pernicious anemia, inflammatory bowel disease (Crohn's disease and ulcerative colitis), psoriasis, renal, pulmonary, and hepatic fibroses, Addison's disease, type I diabetes, systemic lupus erythematosus, dermatomyositis, Sjogren's syndrome, multiple sclerosis, myasthenia gravis, Reiter's syndrome, and Grave's disease, among others.

**Bioterrorism agents:** Any pathogen (such as bacteria and viruses) or toxin that can be dispersed deliberately to cause disease or death to humans or animals. Examples of bioterrorism agents include *Bacillus anthracis,* which causes anthrax, *Yersinia pestis,* which causes plague, and *Variola major,* which causes smallpox, tick-borne encephalitis virus (TBEV), which causes tick-borne encephalitis, and Ebola virus, which causes Ebola. Bioterrorism agents also include biotoxins, which are toxins produced by certain biological organisms. Exemplary biotoxins are botulinum toxin, produced by the bacterium *Clostridium botulinum,* and ricin, which can be isolated from castor oil seeds. Western counter-proliferation agencies currently recognize 23 types of bacteria, 43 types of viruses, and 14 types of biotoxins as potential bioterrorism agents.

Other examples of bioterrorism agents include, but are not limited to, *Escherichia coli, Haemophilus influenzae,* cobra venom, shellfish toxin, botulinum toxin, saxitoxin, ricin toxin, *Shigella flexneri, S. dysenteriae* (*Shigella bacillus*)*, Salmonella, Staphylococcus* enterotoxin B, *Histoplasma capsulatum,* tricothecene mycotoxin, aflatoxin. Bioterrorism agents can also result in cryptococcosis, brucellosis (undulant fever), coccidioidomycosis (San Joaquin Valley or desert fever), psittacosis (parrot fever), bubonic plague, tularemia (rabbit fever), malaria, cholera, typhoid, hemorrhagic fever, tick-borne encephalitis, Venezuelan equine encephalitis, pneumonic plague, Rocky Mountain spotted fever, dengue fever, Rift Valley fever, diphtheria, melioidosis, glanders, tuberculosis, infectious hepatitis, encephalitides, blastomycosis, nocardiosis, yellow fever, typhus, and Q fever.

**Cancer:** A malignant neoplasm that has undergone characteristic anaplasia with loss of differentiation, increase rate of growth, invasion of surrounding tissue, and that is capable of metastasis. For example, thyroid cancer is a malignant neoplasm that arises in or from thyroid tissue, and breast cancer is a malignant neoplasm that arises in or from breast tissue (such as a ductal carcinoma). Residual cancer is cancer that remains in a subject after any form of treatment given to the subject to reduce or eradicate thyroid cancer. Metastatic cancer is a cancer at one or more sites in the body other than the site of origin of the original (primary) cancer from which the metastatic cancer is derived.

**Chemotherapy; chemotherapeutic agents**: As used herein, includes agents with therapeutic usefulness in the treatment of diseases characterized by abnormal cell growth (*e.g.,* an anti-neoplastic agent). Such diseases include tumors, neoplasms, and cancer, as well as diseases characterized by hyperplastic growth such as psoriasis. In one example, a chemotherapeutic agent is an agent of use in treating neoplasms such as solid tumors. In one example, a chemotherapeutic agent is radioactive molecule. One of skill in the art can readily identify a chemotherapeutic agent (for instance, see Slapak and Kufe, Principles of Cancer Therapy, Chapter 86 in Harrison's Principles of Internal Medicine, 14th edition; Perry et al., Chemotherapy, Ch. 17 in Abeloff, Clinical Oncology 2nd ed., © 2000 Churchill Livingstone, Inc; Baltzer L, Berkery R (eds): Oncology Pocket Guide to Chemotherapy, 2nd ed. St. Louis, Mosby-Year Book, 1995; Fischer DS, Knobf MF, Durivage HJ (eds): The Cancer Chemotherapy Handbook, 4th ed. St. Louis, Mosby-Year Book, 1993).

**Control:** A reference standard. In some examples, a control can be a known value (or range of values) indicative of activity of a molecule, such as TAM receptor activity or TAM ligand binding activity, in a sample not treated with a test agent. A difference between a test sample and a control can be an increase or conversely a decrease. The difference can be a qualitative difference or a quantitative difference, for example a statistically significant difference. In some examples, a difference is an increase or decrease, relative to a control, of at least about 10%, such as at least about 20%, at least about 30%, at least about 40%, at least about 50%, at least about 60%, at least about 70%, at least about 80%, at least about 90%, at least about 100%, at least about 150%, at least about 200%, at least about 250%, at least about 300%, at least about 350%, at least about 400%, at least about 500%, or greater then 500%.

In some examples, the amount of phosphorylated protein and/or unphosphorylated protein detected can be compared to a control. In several examples, the control is a known value indicative of the amount of phosphorylated protein, such as TAM receptor phosphorylation, formed from basal phosphorylation of the protein (*e.g.,* TAM receptor). In other examples, the control is a value indicative of the amount of activity, such as phosphorylation or biological activity, in the presence of a known amount of TAM receptor ligand. In still other examples, the control is the amount of phosphorylated peptide formed in a sample not contacted with the test agent. One of skill in the art will understand that the amount of unphosphorylated peptide can be determined from the difference between the total amount of peptide used and the amount of phosphorylated peptide detected. A difference between the amount of activity in the presence of a test agent relative to a control can indicate that the test agent can be of use as a TAM receptor inhibitor (such as for immunoenhancement) or a TAM receptor agonist (such as for immunosuppression).

**Dendritic cell (DC):** The principal antigen presenting cell (APC) involved in primary immune responses. Dendritic cells include plasmacytoid dendritic cells and conventional dendritic cells. Their major function is to obtain antigen in tissues, migrate to lymphoid organs and present the antigen in order to activate T cells. Immature dendritic cells originate in the bone marrow and reside in the periphery as immature cells.

**EC₅₀:** A measure of concentration used in pharmacology. EC₅₀ (the half maximal effective concentration) refers to the concentration of an agent (such as a ligand or antibody) which induces a response halfway between the baseline and maximum. It is commonly used as a measure of drug potency. The EC₅₀ of a graded dose response curve therefore represents the concentration of a compound where 50% of its maximal effect is observed. The EC₅₀ of a quantal dose response curve represents the concentration of a compound where 50% of the population exhibit a response.

**Graft-versus-host disease:** Tissue rejection, also called graft-versus-host disease, is a consequence of organ or tissue transplantation caused by the transplant recipient's (host's) immune response to the transplanted organ/tissue which can damage or destroy it. Ordinarily, the immune response protects the body from potentially harmful substances (antigens) such as microorganisms, toxins, and cancer cells. The immune system distinguishes "self" from "foreign" by reacting to proteins on the surfaces of cells. It reacts against substances it recognizes as foreign (antigens). The presence of foreign blood or tissue in the body triggers an immune response that can result in blood transfusion reactions and transplant rejection when antibodies are formed against foreign antigens on the transplanted or transfused material.

**HIV (human immunodeficiency virus):** A retrovirus that causes immunosuppression in humans (HIV disease) and leads to a disease complex known as the acquired immunodeficiency syndrome (AIDS). "HIV disease" refers to a well-recognized constellation of signs and symptoms (including the development of opportunistic infections) in persons who are infected by an HIV virus (such as HIV-1 or HIV-2), for example as determined by antibody or Western blot studies. Laboratory findings associated with this disease include a progressive decline in T-helper cells.

**IC₅₀:** A measure of concentration used in pharmacology. IC₅₀, or the half maximal inhibitory concentration, represents the concentration of an inhibitor that is required for 50% inhibition of its target (for instance, an enzyme, a cell, a receptor, or a microorganism). Generally, an IC₅₀ value is a measure of how much of a particular composition is needed to inhibit some biological process by 50%. IC₅₀ is commonly used as a measure of drug affinity, and represents the concentration of a composition that is required to obtain 50% of the maximum effect *in vivo.*

**Immune-mediated disorder:** A disorder that involves an unwanted immune response. Although immune recognition of "non-self' proteins is essential to avoid and eliminate infection, the immune response can sometimes be unwanted. Autoimmune diseases, such as rheumatoid arthritis, multiple sclerosis or insulin dependent diabetes mellitus, are the result of a pathological immune response against self antigens, and T cells are the primary mediators of autoimmunity.

Rejection of transplanted organs and tissues is a further example of an immune-mediated disorder, and can often result in damage to and/or rejection of the transplant. Tissue rejection, also called graft-versus-host disease, is a consequence of organ or tissue transplantation caused by the transplant recipient's (host's) immune response to the transplanted organ/tissue which can damage or destroy it. No two people (except identical twins) have identical tissue antigens. Therefore, in the absence of immunosuppressive drugs, organ and tissue transplantation would almost always causes an immune response against the foreign tissue (rejection), which would result in destruction of the transplant. Though tissue typing ensures that the organ or tissue is as similar as possible to the tissues of the recipient, unless the donor is an identical twin, no match is perfect and the possibility of organ/tissue rejection remains. Immunosuppressive therapy is used to prevent organ rejection. Allergy is another example of an immune-mediated disorder.

**Immune response:** A response of a cell of the immune system, such as a B cell or T cell, to a stimulus. In one embodiment, the response is specific for a particular antigen (an "antigen-specific response"). A "parameter of an immune response" is any particular measurable aspect of an immune response, including, but not limited to, cytokine secretion (IL-6, IL-10, IFN-α, etc.), immunoglobulin production, dendritic cell maturation, and proliferation of a cell of the immune system. "Enhancing an immune response" includes the use of any composition or method that results in an increase in any of these parameters. One of ordinary skill in the art can readily determine an increase in any one of these parameters using known laboratory assays. In one specific non-limiting example, incorporation of ³H-thymidine can be measured to assess cell proliferation. A "substantial" increase in a parameter of the immune response is a significant increase in this parameter as compared to a control. Specific, non-limiting examples of a substantial increase are at least about a 50% increase, at least about a 75% increase, at least about a 90% increase, at least about a 100% increase, at least about a 200% increase, at least about a 300% increase, and at least about a 500% increase.

One of ordinary skill in the art can readily identify a significant increase using known statistical methods. One, specific, non-limiting example of a statistical test used to assess a substantial increase is the use of a Z test to compare the percent of samples that respond to a vaccine alone as compared to the percent of samples that respond using a vaccine administered in conjunction with a TAM receptor inhibitor. A non-parametric ANOVA can be used to compare differences in the magnitude of the response induced by vaccine alone as compared to the percent of samples that respond using vaccine administered in conjunction with a TAM receptor inhibitor. In this example, *p* ≤ 0.05 is significant, and indicates a substantial increase in the parameter of the immune response. One of skill in the art can readily identify other statistical assays of use.

An "immunoprotective response" is an immune response that results in a decrease of symptoms upon infection or results in a delay, amelioration, or prevention of a disease associated with infection. "Enhancing the immunogenicity of a vaccine" is an example of an increase in an immune response.

"Inhibiting an immune response" includes the use of any composition or method that results in a decrease in any of these parameters. One of ordinary skill in the art can readily determine a decrease in any one of these parameters using known laboratory assays. In one specific non-limiting example, incorporation of ³H-thymidine can be measured to assess cell proliferation. A "substantial" decrease in a parameter of the immune response is a significant decrease in this parameter as compared to a control. Specific, non-limiting examples of a substantial decrease are at least about a 5% decrease, at least about a 10% decrease, at least about a 20% decrease, at least about a 30% decrease, at least about a 40% decrease, at least about a 50% decrease, at least about a 60% decrease, at least about a 70% decrease, at least about a 80% decrease, and at least about a 90% decrease.

One of skill in the art can readily identify a significant decrease using known statistical methods. One, specific, non-limiting example of a statistical test used to assess a substantial decrease is the use of a Z test to compare the percent of samples that respond to a TAM receptor agonist as compared to the percent of samples that respond in the absence TAM receptor agonist. For example, a response could be the level of one or more circulating inflammatory cytokines in subjects that receive or do not receive the TAM receptor agonist. A non-parametric ANOVA can be used to compare differences in the magnitude of the response induced by a TAM receptor agonist as compared to the percent of samples that respond using no TAM receptor agonist. In this example, *p* ≤ 0.05 is significant, and indicates a substantial decrease in the parameter of the immune response. One of skill in the art can readily identify other statistical assays of use.

Assays for immunosuppression will vary as a function of the disease application of interest. For some applications, such as potential treatment of systemic lupus erythematosus (SLE) and rheumatoid arthritis (RA), the serum levels of circulating inflammatory cytokines will be measured using routine methods, such as ELISA or other immunoassay. These inflammatory cytokines include tumor necrosis factor alpha (TNFα) and type I interferons (IFNs), such as IFNα and IFNβ. For example, a significant decrease in circulating levels of one or more of these inflammatory cytokines as measured by ELISA or other immunoassays following treatment with a TAM receptor activator indicates a successful immunosuppression treatment. For example, the circulating levels of one or more TNFα, IFNα and IFNβ can be measured prior to and following administration of the TAM receptor activator. The circulating levels can then be compared to determine if immunosuppression has occurred. In other examples, the level of these cytokines following treatment with a TAM receptor activator can be compared to a reference value (such as a value that has been previously determined to be present in a subject in need of immunosuppression).

**Immune system deficiency:** A disease or disorder in which the subject's immune system is not functioning in normal capacity or in which it would be useful to enhance a subject's immune response. Immune system deficiencies include those diseases or disorders in which the immune system is not functioning at normal capacity, or in which it would be useful to enhance the immune system response.

**Immunocompromised:** An immunocompromised subject is a subject who is incapable of developing or unlikely to develop a robust immune response, usually as a result of disease (such as infection), malnutrition, or immunosuppressive therapy. An immunocompromised immune system is an immune system that is functioning below normal levels. Immunocompromised subjects are more susceptible to opportunistic infections, for example viral, fungal, protozoan, or bacterial infections, and certain neoplasms. Those who can be considered to be immunocompromised include, but are not limited to, subjects with AIDS (or HIV positive), subjects with severe combined immune deficiency (SCID), diabetics, subjects who have had transplants and who are taking immunosuppressives, and those who are receiving chemotherapy for cancer. The term "immunocompromised individuals" also includes subjects with most forms of cancer (other than skin cancer), sickle cell anemia, cystic fibrosis, those who do not have a spleen, subjects with end stage kidney disease (dialysis), and those who have been taking corticosteroids on a frequent basis by pill or injection within the last year. Subjects with severe liver, lung, or heart disease also can be immunocompromised.

**Immunomodulator:** A molecule, such as a chemical compound, small molecule, steroid, nucleic acid molecule, or other biological agent, that can modulate an immune response. In some examples, an immunomodulator increases an immune response and has immunoenhancing activity. In additional examples, an immunomodulator decreases or suppresses an immune response and has immunosuppressant activity. Specific, non-limiting examples of immunomodulator are molecules that either decrease or increase an immune response by at least about a 10%, at least about a 20%, at least about 30%, at least about 40%, at least about 50%, at least about 60%, at least about 70% decrease, at least about 80%, or at least about 90%, for example relative to a control in the absence of the immunomodulator.

**Immunosuppressive agent:** A molecule, such as a chemical compound, small molecule, steroid, nucleic acid molecule, or other biological agent, that can decrease an immune response such as an inflammatory reaction. Specific, non-limiting examples of immunosuppressive agents are non-steroidal anti-inflammatory agents, cyclosporine A, FK506, and anti-CD4. In additional examples, the agent is a biological response modifier, such as Kineret® (anakinra), Enbrel® (etanercept), or Remicade® (infliximab), a disease-modifying antirheumatic drug (DMARD), such as Arava® (leflunomide), a nonsteroidal anti-inflammatory drug (NSAIDs), specifically a Cyclo-Oxygenase-2 (COX-2) inhibitor, such as Celebrex® (celecoxib) and Vioxx® (rofecoxib), or another product, such as Hyalgan® (hyaluronan) and Synvisc® (hylan G-F20).

**Infectious agent:** An agent that can infect a subject, including, but not limited to, viruses, bacteria, and fungi.

Examples of infectious viruses include, but are not limited to, enveloped viruses such as members of the following viral families: *Retroviridae* (*e.g.,* HIV (such as HIV1 and HIV2), MLV, SIV, FIV, Human T-cell leukemia viruses 1 and 2, XMRV, and Coltiviruses (such as CTFV or Banna virus)); *Togaviridae* (for example, alphaviruses (such as Ross River virus, Sindbis virus, Semliki Forest Virus, O'nyong'nyong virus, Chikungunya virus, Eastern equine encephalitis virus, Western equine encephalitis virus, Venezuelan equine encephalitis virus) or rubella viruses); *Flaviridae* (for example, dengue viruses, encephalitis viruses (such as West Nile virus or Japanese encephalitis virus), yellow fever viruses); *Coronaviridae* (for example, coronaviruses such as SARS virus or Toroviruses); *Rhabdoviridae* (for example, vesicular stomatitis viruses, rabies viruses); *Paramyxoviridae* (for example, parainfluenza viruses, mumps virus, measles virus, respiratory syncytial virus, sendai virus, and metopneumovirus); *Orthomyxoviridae* (for example, influenza viruses); *Bunyaviridae* (for example, Hantaan virus, bunya viruses (such as La Crosse virus), phleboviruses, and Nairo viruses); *Hepadnaviridae* (Hepatitis B viruses); *Herpesviridae* (herpes simplex virus (HSV) 1 and HSV-2, varicella zoster virus, cytomegalovirus (CMV), HHV-8, HHV-6, HHV-7, and pseudorabies virus); *Filoviridae* (filoviruses including Ebola virus and Marburg virus) and *Poxviridae* (variola viruses, vaccinia viruses, pox viruses (such as small pox, monkey pox, and Molluscum contagiosum virus), yatabox virus (such as Tanapox and Yabapox)). Non-enveloped infectious viruses include such as members of the following families: *Calciviridae* (such as strains that cause gastroenteritis); *Arenaviridae* (hemorrhagic fever viruses such as LCMV, Lassa, Junin, Machupo and Guanarito viruses); *Reoviridae* (for instance, reoviruses, orbiviruses and rotaviruses); *Birnaviridae; Parvoviridae* (parvoviruses, such as Human bocavirus adeno-associated virus); *Papillomaviridae* (such as papillomaviruses); *Papovaviridae* (papilloma viruses, polyoma viruses); *Adenoviridae* (adenoviruses); *Picornaviridae* (enteroviruses, enteric viruses, Poliovirus, coxsackieviruses, hepatoviruses, cardioviruses, aptoviruses, echoviruses, hepatitis A virus, Foot and mouth disease virus, and rhinovirus) and *Iridoviridae* (such as African swine fever virus). Other infectious viruses include unclassified viruses (for example, the etiological agents of Spongiform encephalopathies, the agent of delta hepatitis (thought to be a defective satellite of hepatitis B virus), the agents of non-A, non-B hepatitis (class 1 = internally transmitted; class 2 = parenterally transmitted (for instance, Hepatitis C); calciviruses (such as Norovirus, Norwalk and related viruses); Hepeviruses (such as Hepatitis E, JC and BK viruses) and astroviruses).

Examples of infectious bacteria that can be treated with the methods provided herein include any type of Gram-positive (such as Streptococcus, Staphylococcus, Corynebacterium, Listeria, Bacillus and Clostridium) or Gram-negative bacteria (such as Salmonella, Shigella, Enterobacteriaceae, Pseudomonas, Moraxella, Helicobacter, Stenotrophomonas, Bdellovibrio, acetic acid bacteria, and alpha-proteobacteria), *Escherichia coli, Neisseria gonorrhoeae, Neisseria meningitidis, Moraxella catarrhalis, Hemophilus influenzae, Klebsiella pneumoniae, Legionella pneumophila, Pseudomonas aeruginosa, Proteus mirabilis, Enterobacter cloacae, Serratia marcescens*). Exemplary infectious bacteria include, but are not limited to: *Helicobacter pyloris, Borelia burgdorferi, Legionella pneumophilia, Mycobacteria sps* (such as *M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes* (Group A *Streptococcus*), *Streptococcus agalactiae* (Group B *Streptococcus), Streptococcus* (*viridans* group), *Streptococcus faecalis, Streptococcus bovis, Streptococcus (anaerobic sps.), Streptococcus pneumoniae, pathogenic Campylobacter sp., Enterococcus sp., Haemophilus influenzae, Bacillus anthracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, and Actinomyces israelli.*

Examples of infectious fungi include, but are not limited to, *Cryptococcus neoformans, Histoplasma capsulatum, Coccidioides immitis, Blastomyces dermatitidis, Chlamydia trachomatis,* and *Candida albicans.*

Examples of infectious organisms (such as parasites) include, but are not limited to *Plasmodium falciparum* and *Toxoplasma gondii.*

**Inflammation:** When damage to tissue occurs, the body's response to the damage is usually inflammation. The damage may be due to trauma, lack of blood supply, hemorrhage, autoimmune attack, transplanted exogenous tissue or infection. This generalized response by the body includes the release of many components of the immune system (for instance, IL-1 and TNF), attraction of cells to the site of the damage, swelling of tissue due to the release of fluid and other processes.

**Isolated:** An "isolated" biological component (such as a nucleic acid molecule, peptide, or cell) has been purified away from other biological components in a mixed sample (such as a cell extract). For example, an "isolated" peptide or nucleic acid molecule is a peptide or nucleic acid molecule that has been separated from the other components of a cell in which the peptide or nucleic acid molecule was present (such as an expression host cell for a recombinant peptide or nucleic acid molecule).

**Opportunistic infection:** An infection that occurs in an immunocompromised subject. An opportunistic infection can result from medical treatments or from alterations in the immune system. Opportunistic infections can include, but are not limited to, bacterial infections such as salmonellosis, syphilis and neurosyphilis, tuberculosis (TB), atypical mycobacterial infection, and bacillary angiomatosis (cat scratch disease), fungal infections such as aspergillosis, candidiasis (thrush, yeast infection), coccidioidomycosis, cryptococcal meningitis, and histoplasmosis, protozoal infections such as cryptosporidiosis, isosporiasis, microsporidiosis, *Pneumocystis Carinii* pneumonia (PCP), and toxoplasmosis, viral infections such as *Cytomegalovirus* (CMV), hepatitis, herpes simplex (HSV, genital herpes), herpes zoster (HZV, shingles), human papiloma virus (HPV, genital warts, cervical cancer), *Molluscum Contagiosum,* oral hairy leukoplakia (OHL), and progressive multifocal leukoencephalopathy (PML), and neoplasms, such as Kaposi's sarcoma, systemic non-Hodgkin's lymphoma (NHL), and primary CNS lymphoma, among others.

**Parenteral:** Administered outside of the intestine, for instance, not via the alimentary tract. Generally, parenteral formulations are those that will be administered through any possible mode except ingestion. This term especially refers to injections, whether administered intravenously, intrathecally, intramuscularly, intraperitoneally, intra-articularly, or subcutaneously, and various surface applications including intranasal, intradermal, and topical application, for instance.

**Pharmaceutical agent or drug:** A chemical compound or composition capable of inducing a desired therapeutic or prophylactic effect when properly administered to a subject. Pharmaceutical agents include, but are not limited to, TAM receptor inhibitors, TAM receptor agonists, anti-infective agents, such as antibiotics, anti-fungal compounds, anti-viral compounds, and hyper-immune globulin, anti-cancer agents, for instance, chemotherapeutics, immunosuppressive agents, for instance, non-steroidal anti-inflammatory agents, biological response modifiers, and disease-modifying antirheumatic drugs.

**Pharmaceutically acceptable carriers:** The pharmaceutically acceptable carriers useful in this disclosure are conventional. Remington's Pharmaceutical Sciences, by E. W. Martin, Mack Publishing Co., Easton, PA, 15th Edition (1975), describes compositions and formulations suitable for pharmaceutical delivery of the inhibitors herein disclosed.

In general, the nature of the carrier will depend on the particular mode of administration being employed. For instance, parenteral formulations usually include injectable fluids that include pharmaceutically and physiologically acceptable fluids such as water, physiological saline, balanced salt solutions, aqueous dextrose, glycerol or the like as a vehicle. For solid compositions (for instance, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Retroviruses:** RNA viruses wherein the viral genome is RNA. When a host cell is infected with a retrovirus, the genomic RNA is reverse transcribed into a DNA intermediate which is integrated very efficiently into the chromosomal DNA of infected cells. The integrated DNA intermediate is referred to as a provirus. The term "lentivirus" is used in its conventional sense to describe a genus of viruses containing reverse transcriptase. The lentiviruses include the "immunodeficiency viruses" which include human immunodeficiency virus (HIV) type 1 and type 2 (HIV-1 and HIV-2), simian immunodeficiency virus (SIV), and feline immunodeficiency virus (FIV).

HIV is a retrovirus that causes immunosuppression in humans (HIV disease), and leads to a disease complex known as acquired immunodeficiency syndrome (AIDS). "HIV disease" refers to a well-recognized constellation of signs and symptoms (including the development of opportunistic infections) in persons who are infected by an HIV virus, as determined by antibody or western blot studies. Laboratory findings associated with this disease include a progressive decline in T-helper cells.

**Sepsis:** A serious medical condition characterized by a whole-body inflammatory state caused by infection. As used here, the term "infection" means a pathological process induced by a microorganism. Such an infection can be caused by pathogenic gram-negative and gram-positive bacteria, anaerobic bacteria, fungi, yeast, or polymicrobial organisms. Exemplary non-limiting sites of such infections are respiratory tract infections, peritonitis, genitourinary infections, and intraabdoiminal infections. As used herein, "sepsis" includes all stages of sepsis including, but not limited to, the onset of sepsis, severe sepsis, septic shock and multiple organ dysfunction associated with the end stages of sepsis.

The "onset of sepsis" refers to an early stage of sepsis, for example, prior to a stage when conventional clinical manifestations are sufficient to support a clinical suspicion of sepsis. "Severe sepsis" refers to sepsis associated with organ dysfunction, hypoperfusion abnormalities, or sepsis-induced hypotension. Hypoperfusion abnormalities include, but are not limited to, lactic acidosis, oliguria, or an acute alteration in mental status. "Septic shock" in adults refers to a state of acute circulatory failure characterized by persistent arterial hypotension unexplained by other causes. Hypotension is defined by a systolic arterial pressure below 90 mm Hg (or, in children, below normal for their age), a MAP below 60, or a reduction in systolic blood pressure of greater than 40 mm Hg from baseline, despite adequate volume resuscitation, in the absence of other causes for hypotension.

Symptoms of sepsis are often related to the underlying infectious process. When the infection crosses into sepsis, the resulting symptoms are that of systemic inflammatory response syndrome (SIRS): general inflammation, fever, elevated white blood cell count (leukocytosis), and raised heart rate (tachycardia) and breathing rate (tachypnea). The immunological response that causes sepsis is a systemic inflammatory response causing widespread activation of inflammation and coagulation pathways. This can progress to dysfunction of the circulatory system and, even under optimal treatment, can result in the multiple organ dysfunction syndrome and eventually death.

In the United States, sepsis is the leading cause of death in non-coronary intensive care unit (ICU) patients, and the tenth most common cause of death overall. Sepsis is common and also more dangerous in elderly, immunocompromised, and critically ill patients. It occurs in 1%-2% of all hospitalizations and accounts for as much as 25% of ICU bed utilization. It is a major cause of death in intensive care units worldwide, with mortality rates that range from 20% for sepsis to 40% for severe sepsis to >60% for septic shock. Patients are defined as having "severe sepsis" if they have sepsis plus signs of systemic hypoperfusion; either end organ dysfunction or a serum lactate greater then 4 mmol/dL.

Current therapies for sepsis include antibiotics, surgical drainage of infected fluid collections, fluid replacement and appropriate support for organ dysfunction. This can include hemodialysis in kidney failure, mechanical ventilation in pulmonary dysfunction, transfusion of blood products, and drug and fluid therapy for circulatory failure. Ensuring adequate nutrition, if necessary by parenteral nutrition, can be important during prolonged illness.

**siRNA:** Double stranded RNAs (dsRNAs) that can induce gene-specific inhibition of expression in invertebrate and vertebrate species are provided. These RNAs are suitable for interference or inhibition of expression of a target gene *(e.g.,* a TAM receptor or ligand thereof) and comprise double stranded RNAs of about 15 to about 40 nucleotides containing a 3' and/or 5' overhang on each strand having a length of 0- to about 5-nucleotides, wherein the sequence of the double stranded RNAs is substantially identical to a portion of a mRNA or transcript of the target gene for which interference or inhibition of expression is desired. The double stranded RNAs can be formed from complementary ssRNAs or from a single stranded RNA that forms a hairpin or from expression from a DNA vector.

In addition to native RNA molecules, RNA suitable for inhibiting or interfering with the expression of a target sequence include RNA derivatives and analogs. For example, a non-natural linkage between nucleotide residues can be used, such as a phosphorothioate linkage. The RNA strand can be derivatized with a reactive functional group or a reporter group, such as a fluorophore. Particularly useful derivatives are modified at a terminus or termini of an RNA strand, typically the 3' terminus of the sense strand. For example, the 2'-hydroxyl at the 3' terminus can be readily and selectively derivatized with a variety of groups.

Other useful RNA derivatives incorporate nucleotides having modified carbohydrate moieties, such as 2'-O-alkylated residues or 2'-deoxy-2'-halogenated derivatives. Particular examples of such carbohydrate moieties include 2'-O-methyl ribosyl derivatives and 2'-O-fluoro ribosyl derivatives.

The RNA bases may also be modified. Any modified base useful for inhibiting or interfering with the expression of a target sequence can be used. For example, halogenated bases, such as 5-bromouracil and 5-iodouracil can be incorporated. The bases can also be alkylated, for example, 7-methylguanosine can be incorporated in place of a guanosine residue. Non-natural bases that yield successful inhibition can also be incorporated.

**Subject:** Living multi-cellular vertebrate organisms, a category that includes both human and non-human mammals. The methods and compositions disclosed herein have equal applications in medical and veterinary settings. Therefore, the general term "subject" is understood to include all animals, including, but not limited to, humans or veterinary subjects, such as other primates, dogs, cats, horses, and cows.

**TAM receptor:** The TAM family (Tyro 3, Axl, and Mer) was first identified as a distinct receptor protein-tyrosine kinase (PTK) family (Lai & Lemke, (1991) Neuron. 6(5):691-704). Designated Tyro 3, Tyro 7, and Tyro 12 at that time, the kinase domains of these proteins clearly segregated into a separate family based on sequence conservation (Lai & Lemke, (1991) Neuron. 6(5):691-704). Subsequent isolation of full-length cDNAs by multiple groups confirmed this segregation, and also resulted in multiple names for the receptors. Tyro 3, Axl, and Mer are now the consensus, assigned gene designations. An analysis of the mouse and human 'kinomes' indicates that Tyro 3, Axl, and Mer constitute the full TAM family. (There are 58 receptor PTK genes in the human and mouse genomes.) Specific examples of Axl receptor amino acid sequences include, but are not limited to Genbank Accession Nos. NP_001690 (invariant ATP binding Lysine (K) 558) and NP_068713 (as of November 9,2007). Specific examples of Tyro 3 receptor amino acid sequences include, but are not limited to Genbank Accession Nos. NP_006284 (invariant ATP binding Lysine (K) 550), EAW92506, and EAW92507 (as of November 9,2007). Specific examples of Mer receptor amino acid sequences include, but are not limited to Genbank Accession Nos. AAK54121, AAI14918 (invariant ATP binding Lysine (K) 443), and AAI14918 (as of November 9,2007). The invariant ATP binding site Lysine (K) is located in the sequence VAVKTM.

The TAM receptors share an arrangement of sequence motifs in their extracellular regions in which two tandem immunoglobulin (Ig)-related domains are immediately followed by two fibronectin type III (FNIII)-related repeats. These receptors are the only receptor PTKs to display this particular array of Ig and FNIII domains. The ectodomains of Tyro 3, Axl, and Mer are all followed closely by a single transmembrane domain, a relatively large cytoplasmic juxtamembrane region, and a split tyrosine kinase domain. For example, the extracellular domain of human Axl (Genbank Accession No. NP_068713.2 as of November 7, 2008) spans amino acid positions from about position 1 to about position 445 amino and contains two Ig domains and two FNIII domains. The first Ig domain, denoted herein as IgI, includes from about position 33 to about position 137. The second Ig domain, denoted herein as Ig2, includes from about position 139 to about position 222 of SEQ ID NO:2. The first FNIII domain, denoted herein as FNIII(a), includes from about position 225 to about position 328. The second FNIII domain, denoted herein as FNIII(b), includes from about position 337 to about position 418. Further, the intracellular domain, such as the intracellular domain of Mer (such as the amino acid sequence of Mer Genbank Accession No. NP_032613.1 as of November 7, 2008) spans amino acid positions from about position 521 to about position 994.

The positions of each of the domains of each of the TAM receptors, including their ligand binding domains and the ATP and substrate binding sites of the protein-tyrosine kinase domains, are known in the art and are readily accessible in public NCBI and National Library of Medicine (NLM) databases. For example, the extracellular domain of the human Tyro3 protein (Genbank Accession No. EAW92508 as of November 7, 2008), contains a first Ig domain from about position 41 to about position 120, a second Ig domain from about position 130 to about position 205, a first FNIII domain from about position 215 to about position 305, and a second FNIII domain from about position 315 to about position 397. The Tyro3 protein-tyrosine kinase domain extends from about position 510 to about position 730. The extracellular domain of the human c-Mer protein (GenBank Accession No. EAW52097 as of November 7, 2008) contains a first Ig domain from about position 115 to about position 187, a second Ig domain from about position 195 to about position 280, a first FNIII domain from about position 285 to about position 375, and a second FNIII domain from about position 387 to about position 478. The c-Mer substrate binding site extends from about position 725 to about position 750.

The TAM receptor ligands include Protein S and Gas6. "TAM receptor activity" includes any biological activity of the TAM receptor, for instance an activity that is enhanced or induced by the binding of a TAM receptor ligand *(e.g.,* Gas6 or Protein S) to the receptor. An "enhancer of TAM receptor activity" includes any composition that increases TAM receptor activity. Examples of an inecrease in TAM receptor activity include, but are not limited to a an increase in TAM autophosphorylation, a decrease in TLR-induced cytokine production, a decrease in TLR-induced stimulation of MAP kinase activation, a decrease in TLR-induced NF-kB activation, and an increase in SOCS1 and/or SOCS 3 expression. An "inhibitor of TAM receptor activity" includes any composition that decreases TAM receptor activity. Examples of a decrease in TAM receptor activity include, but are not limited to a decrease in TAM autophosphorylation, an increase in TLR-induced cytokine production, an increase in TLR-induced stimulation of MAP kinase activation, an increase in TLR-induced NF-kB activation, and a decrease in SOCS1 and/or SOCS 3 expression. Exemplary methods for measuring such activity are provided herein.

**Therapeutically effective amount:** An amount of a therapeutic agent (such as a TAM receptor inhibitor or TAM receptor agonist), alone or in combination with other agents (such as a vaccine or dendritic cells or an immunosuppressive agent) sufficient to prevent advancement of a disease, to cause regression of the disease, or which is capable of relieving symptoms caused by the disease, such as fever, respiratory symptoms, pain or swelling.

**Treating a disease:** "Treatment" refers to a therapeutic intervention that ameliorates a sign or symptom of a disease or pathological condition (*e.g*., an autoimmune disorder, sepsis or other infection) after it has begun to develop. As used herein, the term "treatment" also encompasses "prevention," which refers to inhibiting the full development of a disease, for example in a person who is known to have a predisposition to a disease such as a person who has been or is at risk for being exposed to an infective agent or who has been or is at risk for developing an autoimmune disease.. Examples of persons at risk for being exposed to an infective agent include, but are not limited to, military personnel, medical personnel, travelers, and caregivers of adults and children, as well as those with weakened immune systems, for example, the elderly, people on immunosuppressive drugs, subjects with cancer, and subjects infected with HIV. For treatment of sepsis, in one embodiment, treatment is for severe sepsis, septic shock, or treatment of sepsis where SOCS inhibitor levels are high, or, in some instances, maximal. Such a treatment is less effective early in infection, when SOCS inhibitor levels (elevated by TAM signaling) are not yet maximal. In some embodiments, measurement of Gas6 in plasma is a biomarker for sepsis (see, for instance, Gibot et al., (2007) Crit Care;11(1):R8; Borgel et al., (2006) Crit Care Med. 34(1):219-22). Examples of persons at risk for developing an autoimmune disease include, but are not limited to, people with a family history of one or more autoimmune disease, those who are at risk of exposure to certain environmental triggers, subjects producing antibodies to self-antigens, and subjects who have undergone transplantation.

**Vaccine:** A preparation of antigen, DNA, protein subunit, peptide, attenuated microorganisms (including but not limited to bacteria and viruses), dendritic cells activated against a tumor (*e.g*., cancer), living microorganisms, or killed microorganisms, administered for the prevention, amelioration or treatment of a disease, for instance, cancer, or an infectious disease.

Generally, the first step in making a vaccine is to isolate or create an organism, or part of one, which is unable to cause full blown disease, but that still retains the antigens responsible for inducing the host's immune response. This can be done in many ways. One way is to kill the organism using heat or formalin; vaccines produced in this way are called "inactivated" or "killed" vaccines. Examples of killed vaccines in common use today are the typhoid vaccine and the Salk poliomyelitis vaccine.

Another way to produce a vaccine is to use only the antigenic part of the disease-causing organism, for example the capsule, the flagella, or part of the protein cell wall; these types of vaccines are called "acellular vaccines." An example of an acellular vaccine is the *Haemophilus influenzae* B (HIB) vaccine. Acellular vaccines exhibit some similarities to killed vaccines: neither killed nor acellular vaccines generally induce the strongest immune responses and can therefore require a "booster" every few years to insure their continued effectiveness. In addition, neither killed nor acellular vaccines can cause disease and are therefore considered to be safe for use in immunocompromised individuals.

A third way of making a vaccine is to "attenuate" or weaken a live microorganism by mutating the organism to alter its growth capabilities. In one embodiment an attenuated vaccine is not replication competent or lacks essential proteins. Examples of attenuated vaccines are those that protect against measles, mumps, and rubella. Immunity is often life-long with attenuated vaccines and does not require booster shots.

Vaccines also can be produced from a toxin. In these cases, the toxin is often treated with aluminum or adsorbed onto aluminum salts to form a "toxoid." Examples of toxoids are the diphtheria and the tetanus vaccines. Vaccines made from toxoids often induce low-level immune responses and are therefore sometimes administered with an adjuvant. For example, the diphtheria and tetanus vaccines are often combined with the pertussis vaccine and administered together as a DPT immunization. The pertussis acts as an adjuvant in this vaccine. When more than one vaccine is administered together it is called a "conjugated vaccine."

Another way of making a vaccine is to use an organism that is similar to the virulent organism, but that does not cause serious disease, such as using the cowpox virus to protect against infection with smallpox virus, or BCG vaccine, an attenuated strain of *Mycobacterium bovis,* used to protect against *Mycobacterium tuberculosis.*

"Subunit vaccines" are vaccines which use a polypeptide from an infectious organism to stimulate a strong immune response. An "antigen vaccine" uses an immunogenic epitope of a polypeptide to induce a protective immune response. A "DNA vaccine" uses a nucleic acid encoding an antigen to induce a protective immune response.

Specific, non-limiting examples of vaccines that can be used with the TAM receptor inhibitors and methods of the present disclosure include the BioThrax Anthrax vaccine currently in use by the U.S. military and the DTP (diptheria-tetanus-pertussis) vaccine, the HIB (Haemophilus influenzae type B) vaccine, the Pneumococcal conjugate, the Hepatitis A vaccine, the Hepatitis B vaccine, the Human papillomavirus vaccine, and the Rabies vaccines.

Other vaccines that can be used according to the methods described herein are dendritic cell (DC)-based vaccines, for instance cancer vaccines. In general, a dendritic cell-based vaccine involves obtaining dendritic cells from a subject's blood via leukapheresis. The DCs are then stimulated *ex vivo* with antigens, for instance the subject's own cancer antigens, cultured, and re-administered (for instance, subcutaneously) into the subject. Once administered, DC vaccines activate the immune system's T cells. Activation by DCs causes the T cells to multiply and attack tumor cells that express that antigen. DC-based vaccines are discussed at greater length below.

Unless otherwise explained, all technical and scientific terms used herein have the same meaning as commonly understood by one of ordinary skill in the art to which this disclosure belongs. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: A Comprehensive Desk Reference, published by VCH Publishers, Inc., 1995 (ISBN 1-56081-569-8).

The singular terms "a," "an," and "the" include plural referents unless context clearly indicates otherwise. "Comprising" means "including." "Comprising A or B" means "including A," "including B," or "including A and B." It is further to be understood that all base sizes or amino acid sizes and all molecular weight or molecular mass values given for nucleic acids or peptides are approximate, and are provided for description.

Suitable methods and materials for the practice or testing of the disclosure are described below. However, the provided materials, methods, and examples are illustrative only and are not intended to be limiting. Accordingly, except as otherwise noted, the methods and techniques of the present disclosure can be performed according to methods and materials similar or equivalent to those described and/or according to conventional methods well known in the art and as described in various general and more specific references that are cited and discussed throughout the present specification (see, for instance, Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2000); Ausubel et al., Short Protocols in Molecular Biology: A Compendium of Methodsfrom Current Protocols in Molecular Biology, 4th ed., Wiley & Sons, 1999).

### IV. Use of TAM Receptor Inhibitors as Immunoenhancers and TAM Receptor Agonists as Immunosuppressors

### A. Overview

The innate immune response to pathogens represents the first line of defense against infectious disease. Among the most potent mediators of this response are the Toll-like receptors (TLRs), a set of receptors that activate host defenses responsible for local inflammation, the recruitment of effector cells, and the secretion of cytokines that modulate both the innate and adaptive immune responses (Akira (2006) Curr Top Microbiol Immunol 311, 1-16; Beutler et al., (2006) Annu Rev Immunol 24, 353-389). The twelve TLRs encoded in mammalian genomes are 'pattern recognition receptors,' which detect a diverse set of largely invariant molecular signatures displayed by invading pathogens. TLR-4, for example, recognizes bacterial lipopolysaccharide (LPS; Poltorak et al., (1998) Science 282, 2085-2088). Similarly, TLR-9 recognizes bacterial DNA that contains unmethylated CpG dinucleotides (Hemmi et al., (2000) Nature 408, 740-745), and TLR-3 and TLR-7 are activated by double and single stranded RNAs, respectively (Alexopoulou et al., (2001) Nature 413, 732-73; Heil et al., (2004) Science 303, 1526-1529).

TLRs are prominently expressed in sentinel cells, such as DCs and macrophages, which drive the innate immune response (Iwasaki & Medzhitov (2004) Nat Immunol 5, 987-995). Activation of TLRs engages multiple intracellular adaptor and signaling proteins, including MyD88, TRIF, and TRAF6 (Akira (2006) Curr Top Microbiol Immunol 311, 1-16). Subsequently, evolutionarily ancient signal transduction cascades, centered on the MAP kinase pathways and the NF-κB and interferon response factor (IRF) transcription factors, are activated, resulting in the induction of pro-inflammatory cytokines such as interleukin 6 (IL-6), IL-12, tumor necrosis factor (TNF) α, and type 1 interferons (IFNs). Additionally, DCs are the professional antigen-presenting cells that bridge the innate and adaptive immune responses (Steinman & Hemmi (2006) Curr Top Microbiol Immunol 311, 17-58). TLR activation in DCs induces the secretion of cytokines and the up-regulation of co-stimulatory molecules that subsequently orchestrate the adaptive response (Iwasaki & Medzhitov (2004) Nat Immunol 5, 987-995).

Although innate immunity is essential for the protection of organisms, it must be properly regulated, since unrestrained DC activation and the resulting inflammatory environment can lead to the development of chronic inflammation and a response against self (Marshak-Rothstein (2006) Nat Rev Immunol 6, 823-835). The sustained stimulation of DC maturation that is brought on by elevated levels of type I IFNs and other pro-inflammatory cytokines, for example, is associated with the development of autoimmune diseases such as systemic lupus erythematosus (Banchereau & Pascual (2006) Immunity 25, 383-392), Sjögren's syndrome (Gottenberg et al. (2006) Proc Natl Acad Sci USA 103, 2770-2775), and psoriasis (Lowes et al., (2005) Proc Natl Acad Sci USA 102, 19057-19062). Consistent with a requirement for the eventual inhibition of an inflammatory response, it is now increasingly evident that TLR activation in DCs drives, and is itself modulated by, the production of negative regulators that feed back upon and inhibit this activation (Liew et al., (2005) Nat Rev Immunol 5, 446-458). Prominent among such TLR-driven inhibitors are the SOCS proteins, whose importance in maintaining immune homeostasis is highlighted by the phenotype of SOCS1-deficient mice, which exhibit hyperactivation of DCs and develop lupus-like disease (Hanada et al., (2003) Immunity 19, 437-450). Their importance notwithstanding, prior to this disclosure, how the induction of negative regulators is integrated with the TLR-activated inflammation response remained to be elucidated.

Described herein is a novel negative regulatory pathway driven by receptor tyrosine kinases of the Tyro3/TAM family (Lai & Lemke (1991) Neuron. 6(5):691-704), and establish its role as a pleiotropic inhibitor of both TLR- and cytokine-driven immune responses. Previously, it was demonstrated that loss of function of the three TAM receptors, Tyro 3, Axl, and Mer, results in profound dysregulation of the immune response (Caraux et al., (2006) Nat Immunol 7, 747-754; Lemke & Lu (2003) Curr Opin Immunol 15, 31-36; Lu & Lemke (2001) Science 293, 306-311). Tyro 3^{-/-}Axl^{-/-}Mer^{-/-} triple mutant mice (*TAM* TKOs) display massive splenomegaly and lymphadenopathy, lymphocyte infiltration of essentially all tissues, high circulating autoantibody titers (to phospholipids, collagen, ribonucleoproteins, and double-stranded DNA), and broad spectrum autoimmune disease (Lu & Lemke (2001) Science 293, 306-311). Even Mer-/- single mutants develop appreciable splenomegaly and are hypersensitive to LPS-induced endotoxic shock (Camenisch et al. (1999) J Immunol 162, 3498-3503). These phenotypes are cell non-autonomous with respect to lymphocytes, and, without being bound by theory, are thought to result from the loss of TAM signaling in antigen-presenting cells (APCs; Lu & Lemke (2001) Science 293, 306-311; Lemke & Lu (2003) Curr Opin Immunol 15, 31-36). Described herein is a comprehensive analysis of TAM function in the DC subset of APCs. Also disclosed is a previously unrecognized pathway of TAM-mediated negative regulation of both TLR activation and cytokine production in these cells. This pathway controls the phased attenuation of DC activation.

As described herein, TAM receptor inhibitors can be used as immunoenhancers, for example as a vaccine adjuvant, in the treatment of sepsis, and in the treatment of immunodeficiency. One example of the disclosure is a method of enhancing an immune response in a subject, which can include administering to a subject in need of immunoenhancement (*e.g.*, a subject with severe sepsis, an immunocompromised subject, or a subject to whom a vaccine is administered) one or more TAM receptor inhibitors. The method can be accomplished by administering a therapeutically effective amount of a TAM receptor inhibitor to a subject in need of immunoenhancement, thereby enhancing the immune response in the subject. The immune response to a TAM receptor inhibitor can be measured using routine methods. For example, in the context of vaccine adjuvants, patient response can be monitored by the measurement of circulating serum IgG antibodies to the vaccine target (immunogen), as measured by routine methods such as ELISA or other immunoassay. Humoral (circulating antibody) immunity is linked to the presence of antibodies to the immunogen in the vaccine. In particular examples, such as for use of TAM inhibitors as a vaccine against anthrax, antibodies against recombinant protective antigen (rPA) of the anthrax bacillus can be monitoreds..

Other examples described herein include methods of screening for an immunoenhancing agent. In some examples, these methods include contacting a cell expressing a TAM receptor with a test agent, and determining whether the test agent inhibits TAM receptor activity. These methods are described in greater detail below.

Also as described herein, TAM receptor agonists can be used as immunosuppressors, for example, in the treatment of autoimmune disorders, to prevent or transplant rejection, or treat or prevent graft-versus-host disease following transplant. One embodiment of the disclosure is a method of suppressing an immune response in a subject, which can include administering a TAM receptor agonist to a subject in need of immunosuppression (for instance, a subject with an autoimmune disease, a subject with an allergy, or a subject who has received (or will receive) an organ or tissue transplant). The method can be accomplished by administering a therapeutically effective amount of a TAM receptor agonist to a subject in need of immunosuppression, thereby suppressing the immune response in the subject. The immune response to a TAM receptor agonist can be measured using routine methods. For example, in the treatment of autoimmune disease, patient response can be monitored by, for example, the measurement of circulating levels of pro-inflammatory cytokines such as TNFα and/or IFNs, including IFNα and IFNβ, as measured by routine methods such as ELISA or other immunoassay. A successful response to the immunosuppression is demonstrated as lowered levels of one or more of these cytokines. Patient response can also be monitored by any of several well-described and frequently-employed alternatives, including body temperature: inflammation is associated with elevated body temperature, and immunosuppression restores temperature to normal levels (∼98.6°F in humans).

Other examples described herein include methods of screening for an immunosuppressive agent. In some examples, these methods include contacting a cell expressing a TAM receptor with a test agent, and determining whether the test agent increases TAM receptor activity. These methods are also described in greater detail below.

### B. TAM receptors and ligands

TAM receptors are receptor tyrosine kinases. These cell surface receptor proteins include an extracellular ligand-binding domain (*e.g.,* a domain that binds Gas6 or Protein S ligands), a transmembrane spanning domain, and an intracellular domain responsible for kinase activity. The TAM receptors share an arrangement of sequence motifs in their extracellular regions in which two tandem immunoglobulin (Ig)-related domains are immediately followed by two fibronectin type III (FNIII)-related repeats. These receptors are the only receptor PTKs to display this particular array of Ig and FNIII domains. The ectodomains of Tyro3, Axl, and Mer are all followed closely by a single transmembrane domain, a relatively large cytoplasmic juxtamembrane region, and a split tyrosine kinase domain. Specific examples of Axl receptor amino acid sequences include, but are not limited to Genbank Accession Nos. NP_001690 and NP_068713 (as of November 9, 2007). Specific examples of Tyro3 receptor amino acid sequences include, but are not limited to Genbank Accession Nos. NP_006284, EAW92506, and EAW92507 (as of as of November 9, 2007). Specific examples of Mer receptor amino acid sequences include, but are not limited to Genbank Accession Nos. AAK54121, AAI14918, and AAI14918 (as of as of November 9, 2007).

The extracellular domain of human Axl (NP_068713.2) spans amino acid positions from about position 1 to about position 445 amino and contains two Ig domains and two FNIII domains. The first Ig domain, denoted herein as IgI, includes from about position 33 to about position 137. The second Ig domain, denoted herein as Ig2, includes from about position 139 to about position 222 of SEQ ID NO:2. The first FNIII domain, denoted herein as FNIII(a), includes from about position 225 to about position 328. The second FNIII domain, denoted herein as FNIII(b), includes from about position 337 to about position 418. Further, the intracellular domain, such as the intracellular domain of Mer (such as the amino acid sequence of Mer Genbank Accession No. NP_032613.1 as of November 7, 2008) spans amino acid positions from about position 521 to about position 994.

The positions of each of the domains of each of the TAM receptors are are known. For example, the extracellular domain of the human Tyro3 protein (Genbank Accession No. EAW92508 as of November 7, 2008), contains a first Ig domain from about position 41 to about position 120, a second Ig domain from about position 130 to about position 205, a first FNIII domain from about position 215 to about position 305, and a second FNIII domain from about position 315 to about position 397. The Tyro3 protein-tyrosine kinase domain extends from about position 510 to about position 730. The extracellular domain of the human c-Mer protein (GenBank Accession No. EAW52097 as of November 7, 2008) contains a first Ig domain from about position 115 to about position 187, a second Ig domain from about position 195 to about position 280, a first FNIII domain from about position 285 to about position 375, and a second FNIII domain from about position 387 to about position 478. The c-Mer substrate binding site extends from about position 725 to about position 750.

The TAM receptor ligands include Protein S and Gas6. Protein S (ProS) is an anticoagulant in the blood coagulation cascade. It acts as a co-factor for activated protein C, a protease that degrades Factor V and Factor VIII and thereby inhibits blood coagulation. Gas6, an acronym for growth-arrest-specific protein 6, was originally identified in a screen for mRNAs that were induced when fibroblasts were growth arrested in culture. Gas6 is expressed in discrete cellular loci in a variety of adult tissues, very often in cell layers that are apposed to or intermingled with cells that express Tyro3, Axl, or Mer (Lu & Lemke, (2001) Science. 293(5528):306-11). Many cell types co-express both Gas6 and Protein S, and at the same time also express one or more TAM receptors (Lu & Lemke, (2001) Science. 293(5528):306-11).

Gas6 and Protein S exhibit 44% amino acid sequence identity overall, share the same complex multi-domain structure, and are the only two proteins encoded in the mouse and human genomes that display this configuration of domains. The amino-terminal segments of both proteins contain long strings of glutamic acids residues that are carboxylated on their γ carbons, in a vitamin K-dependent reaction in the Golgi. These so called 'Gla-domains', whose γ carboxylation is essential to both Ca⁺² binding and full biological activity, are common to a number of proteins that bind polar phospholipids such as phosphatidylserine (PS). The extracellularly-displayed PS is a signature of apoptotic cells. In Gas6 and ProS, the Gla domain (such as amino acids 49-90 of Gas6) is closely followed by a loop domain (*e.g*., amino acids 91-117 of human Gas6), and then by four tandem EGF-related domains (*e.g*., amino acids 118-278 of human Gas6). These are in turn followed by a carboxy-terminal domain that contains two laminin G repeats (*e.g.,* amino acids 279-678 of human Gas6) that are structurally related to those of the sex hormone binding globulin (SHBG). The SHBG-related domains of Gas6 and ProS account for both ligand binding and receptor activation, and will fully activate the TAM receptors in the absence of the Gla domain and/or the EGF domain.

### C. TAM receptor inhibitors

TAM receptor inhibitors include agents that significantly reduce or even inhibit the biological activity of a TAM receptor in a cell. Such agents need not inhibit TAM receptor activity by 100%, lesser amounts can be effective in the methods provided herein. For example, a TAM receptor inhibitor may decrease the biological activity by at least 25%, at least 50%, at least 70%, at least 80%, at least 90%, at least 95%, or even at least 99%. Methods of measuring such activity are known in the art. In some examples, a decrease in biological activity is indicated by a decrease in expression of Tyro3, Axl, or Mer or combinations thereof (at the DNA, RNA, or protein level). In other examples, a decrease in biological activity is indicated by a change in a downstream effect, such a reduction in TAM autophosphorylation, increase in TLR-induced cytokine production, increase in TLR-induced stimulation of MAP kinase activation, increase in TLR-induced NF-kB activation, or reduction in SOCS1 and SOCS 3 expression. Methods of detecting such alternations in expression or activity (which in some examples are quantified) are routine, and can include Western blotting, ELISA, flow cytometry, northern blotting, PCR, RT-PCR, and the like.

In some examples, TAM receptor inhibitors bind with high specificity to the TAM receptor extracellular domain or to a Tyro3, Axl, or Mer ligand. Such agents therefore substantially decrease or prevent the interaction between the ligand and the receptor and for example can significantly reduce or inhibit receptor activation, for example by preventing signaling from the receptor and reducing or inhibiting downstream biological effects. Examples of such inhibitors can include antibodies (*e.g*., monoclonal antibodies, for example humanized monoclonal antibodies) or other small molecules that bind to a Tyro3, Axl, or Mer ligand, or a Tyro3, Axl, or Mer receptor, and prevent or significantly reduce the interaction of the ligand binding to the receptor.
In another example, such inhibitors target a Tyro3, Axl, or Mer intracellular domain, such as a kinase domain or ATP binding site, and thus for example significantly reduce or inhibit receptor activation, for example by preventing signaling from the receptor and reducing or inhibiting downstream biological effects. Examples of such inhibitors can include small molecule inhibitors, for example those that are membrane permeable, for example AXL-1, AXL-2, AXL-3, AXL-4, AXL-5, AXL-6, AXL-7, AXL-8, AXL-9, MP470, and SGI-AXL-277. In some examples, the AXL inhibitor is a triazole derivative. Examples of AXL inhibitors are disclosed in U.S. Patent Publication 2007/0213375, filed September 13, 2007. In certain examples, the AXL inhibitor is a triazole derivative with one of the following general structures: 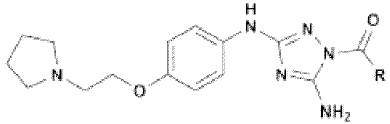 or 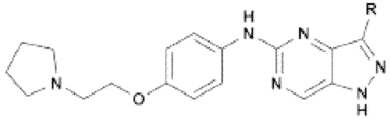 or 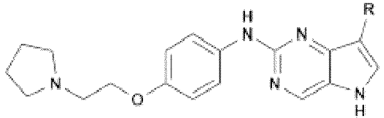 wherein R can be H or CH₃. One skilled in the art will appreciate that other derivatives can be made.

In yet other examples, TAM receptor inhibitors substantially decrease or inhibit expression of a TAM receptor, such as RNAi molecules that significantly decrease or inhibit expression of Tyro3, Axl or Mer. In yet other examples, TAM receptor inhibitors substantially decrease or inhibit expression of a TAM receptor ligand, such as RNAi molecules that significantly decrease or inhibit expression of a Tyro3, Axl or Mer ligand, thereby decreasing the amount of ligand available to activate the TAM receptor.

In some examples, a TAM receptor inhibitor has an IC₅₀ of less than about 50 µM, for instance, less than about 50 nM, or less than 1 pM. In particular examples, a TAM receptor inhibitor has an IC₅₀ of about 10 µM or 20 µM, 0.05 µM to about 5 µM, 0.1 nM to 20 nM, 1 pM to about 10 µM, or 0.1 pM to 50 pM, and in particular examples, a TAM receptor inhibitor has an IC₅₀ of less than from about 0.005 nM to about 50 nM or from about 0.05 nM to about 50 nM. In addition to the known TAM receptor inhibitors, higher potency inhibitors are generated by chemical modification of the existing inhibitors. For instance, the known compounds generally work in the low micromolar or low nanomolar range, however chemical modification makes them, in some examples, more potent and more specific (*e.g.*, work in the low picomoloar range). In one example, QSAR analysis is performed using the solved Kinase Domain Crystal Structure of MERTK. Axl and Tyro3 kinases also may be modeled upon this crystal structure (see, for instance, Walker, Huang, Finerty Jr., Weigelt, Sundstrom, Arrowsmith, Edwards, Bochkarev, Dhe-Paganon, Human Proto-oncogene Tyrosine-protein Kinase MER; PDB (protein data base) 2P0C). These more potent compositions will have lower IC₅₀ values.

In some examples, a TAM receptor inhibitor specifically binds to a target (such as a extracellular binding domain, ligand, or intracellular kinase domain of Tyro, Axl, or Mer) with a binding constant that is at least 10³ M⁻¹ greater, 10⁴ M⁻¹ greater or 10⁵ M⁻¹ greater than a binding constant for other molecules in a sample. In some examples, a TAM receptor inhibitor (such as an aptamer, antibody (*e.g*., monoclonal antibody) or fragments thereof) has an equilibrium constant (K_{d}) of 1 nM or less. For example, TAM receptor inhibitors are provided that bind to a TAM receptor (such as a extracellular binding domain, ligand, or intracellular kinase domain of Tyro, Axl, or Mer) with a binding affinity of at least about 0.1 x 10⁻⁸ M, at least about 0.3 x 10⁻⁸ M, at least about 0.5 x 10⁻⁸ M, at least about 0.75 x 10⁻⁸ M, at least about 1.0 x 10⁻⁸ M, at least about 1.3 x 10⁻⁸ M at least about 1.5 x 10⁻⁸M, or at least about 2.0 x 10⁻⁸ M. Kd values can, for example, be determined by competitive ELISA (enzyme-linked immunosorbent assay) or using a surface-plasmon resonance device such as the Biacore T100, which is available from Biacore, Inc., Piscataway, NJ.

The ability of a TAM receptor inhibitor (*e.g*., RNAi, aptamer, antibody, or membrane permeable small molecule) to function as an immunoenhancer can be performed using the methods described herein. For example, potential TAM receptor inhibitors can be screened for their ability to function as an immunoenhancer. In some examples, the ability of potential TAM receptor inhibitors to enhance a subject's response to a vaccine or treat a disease of immunosuppressio or sepsis is tested.

### 1. Membrane-permeable small molecules

In some examples, TAM receptor inhibitors are small molecule inhibitors that bind to an ATP binding site of Tyro3, Axl, or Mer. Specific examples of Axl receptor amino acid sequences include, but are not limited to Genbank Accession Nos. NP_001690 (invariant ATP binding Lysine (K) 558) and NP_068713 (as of November 9,2007). Specific examples of Tyro 3 receptor amino acid sequences include, but are not limited to Genbank Accession Nos. NP_006284 (invariant ATP binding Lysine (K) 550), EAW92506, and EAW92507 (as of November 9,2007). Specific examples of Mer receptor amino acid sequences include, but are not limited to Genbank Accession Nos. AAK54121, AAI14918 (invariant ATP binding Lysine (K) 443), and AAI14918 (as of November 9,2007). The invariant ATP binding site Lysine (K) is located in the sequence VAV**K**TM.

In some examples, small molecule inhibitors that bind to an intracellular kinase domain (such as an ATP binding site) of Tyro3, Axl, or Mer, can be used to decrease the biological activity of a TAM receptor in a cell. In particular examples, the small molecule inhibitor is membrane permeable. In some examples, a TAM receptor inhibitor is a triazole compound or derivative thereof, such as an inhibitor of Axl catalytic activity (particular examples can be found in US Patent Publication Nos. 20070213375 and 20080153815). Several small molecule TAM receptor inhibitors are known, for instance AXL-1, AXL-2, AXL-3, AXL-4, AXL-5, AXL-6, AXL-7, AXL-8, AXL-9, MP470, and SGI-AXL-277. Other small molecule TAM receptor inhibitors can be obtained, for example, from Rigel Pharmaceuticals, Inc., San Francisco, CA and SuperGen, Inc., Dublin, CA. Other specific examples of TAM receptor inhibitors can be found in PCT Publication Nos: WO07030680A3, WO06052936A3, WO04092735A3, WO07056151A2, and U.S. Patent Publication No: US20070142402 In some examples, the AXL inhibitor is a triazole derivative. Examples of AXL inhibitors are disclosed in U.S. Patent Publication 2007/0213375, filed September 13, 2007, In certain examples, the AXL inhibitor is a triazole derivative with one of the following general structures: 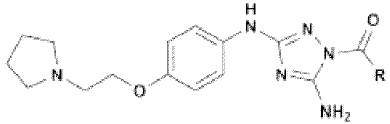 or 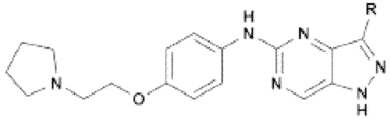 or 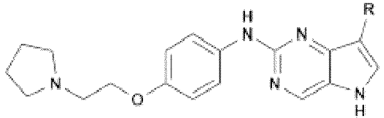 wherein R can be H or CH₃.

In addition to the known TAM receptor inhibitors, higher potency inhibitors can be generated by chemical modification of the existing inhibitors. For instance, the known compounds generally work in the low micromolar range, however chemical modification makes them, in some examples, more potent and more specific. In one example, QSAR analysis is performed using the solved Kinase Domain Crystal Structure of MERTK. Axl and Tyro3 kinases also may be modeled upon this crystal structure (see, for instance, Walker, Huang, Finerty Jr., Weigelt, Sundstrom, Arrowsmith, Edwards, Bochkarev, Dhe-Paganon, Human Proto-oncogene Tyrosine-protein Kinase MER (available on the world wide web at www.rcsb.org/pdb/explore.do?structureId=3BRB-;PDB (protein data base) 2P0C). These more potent compositions will have lower IC₅₀ values.

### 2. Antibodies

In some examples, a TAM receptor inhibitor is an anti- Mer, anti-Tyro3, or anti-Axl antibody, for instance, an anti-human Mer, Tyro3, or Axl monoclonal antibody. Examples of anti-TAM receptor antibodies can be found in, for example, Varnum et al., (1995) Nature, 373: 623-626, and Gallicchio et al., (2005) Blood, Vol. 105, No. 5, pp. 1970-1976. Anti-TAM receptor antibodies can be obtained from Novartis Novartis International AG, Basel, Switzerland. The antibodies encompassed by the present disclosure include any antibody that selectively binds to a conserved binding surface or epitope of a Tyro3, Axl, or Mer protein, for instance, a conserved binding surface or epitope in the extracellular domain of a Tyro3, Axl, or Mer protein, or an antibody that is able to bind to a TAM receptor ligand (*e.g.,* Gas6 or Protein S) and impair the interaction between the ligand and the TAM receptor. An "epitope" of a given protein or peptide or other molecule is a part of or a site on a larger molecule to which an antibody or antigen-binding fragment thereof will bind, and against which an antibody will be produced. An epitope can be defined by both the amino acid residues involved in antibody binding and also by their conformation in three dimensional space (for instance, a conformational epitope or the conserved binding surface). An epitope can be included in peptides as small as about 4-6 amino acid residues, or can be included in larger segments of a protein (*e.g.,* 7-12 amino acids), and need not be comprised of contiguous amino acid residues when referring to a three dimensional structure of an epitope, particularly with regard to an antibody-binding epitope. For example, an epitope of an extracellular domain of a TAM receptor or a TAM ligand can be used to generate antibodies useful for the disclosed methods. Antibody-binding epitopes are frequently conformational epitopes rather than a sequential epitopes, or in other words, an epitope defined by amino acid residues arrayed in three dimensions on the surface of a protein or polypeptide to which an antibody binds.

Disclosed TAM receptor inhibitors include antibodies. The term "antibody" refers to an immunoglobulin molecule (or combinations thereof) that specifically binds to, or is immunologically reactive with, a particular antigen, and includes polyclonal, monoclonal, genetically engineered and otherwise modified forms of antibodies, including but not limited to chimeric antibodies, humanized antibodies, heteroconjugate antibodies (*e.g*., bispecific antibodies, diabodies, triabodies, and tetrabodies), single chain Fv antibodies (scFv), polypeptides that contain at least a portion of an immunoglobulin that is sufficient to confer specific antigen binding to the polypeptide, and antigen binding fragments of antibodies. Antibody fragments include proteolytic antibody fragments [such as F(ab')2 fragments, Fab' fragments, Fab'-SH fragments, Fab fragments, Fv, and rIgG], recombinant antibody fragments (such as sFv fragments, dsFv fragments, bispecific sFv fragments, bispecific dsFv fragments, diabodies, and triabodies), complementarity determining region (CDR) fragments, camelid antibodies (see, for example, U.S. Patent Nos. 6,015,695; 6,005,079; 5,874,541; 5,840,526; 5,800,988; and 5,759,808), and antibodies produced by cartilaginous and bony fishes and isolated binding domains thereof (see, for example, International Patent Application No. WO03014161).

A Fab fragment is a monovalent fragment consisting of the VL, VH, CL and CHI domains; a F(ab')₂ fragment is a bivalent fragment comprising two Fab fragments linked by a disulfide bridge at the hinge region; an Fd fragment consists of the VH and CHI domains; an Fv fragment consists of the VL and VH domains of a single arm of an antibody; and a dAb fragment consists of a VH domain (see, *e.g.,* Ward et al., Nature 341:544-546, 1989). A single-chain antibody (scFv) is an antibody in which a VL and VH region are paired to form a monovalent molecule via a synthetic linker that enables them to be made as a single protein chain (see, *e.g.,* Bird et al., Science, 242: 423-426, 1988; Huston et al., Proc. Natl. Acad. Sci. USA, 85:5879-5883, 1988). Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see, *e.g.,* Holliger et al., Proc. Natl. Acad. Sci. USA, 90:6444-6448, 1993; Poljak et al., Structure, 2:1121-1123, 1994). A chimeric antibody is an antibody that contains one or more regions from one antibody and one or more regions from one or more other antibodies. An antibody may have one or more binding sites. If there is more than one binding site, the binding sites may be identical to one another or may be different. For instance, a naturally occurring immunoglobulin has two identical binding sites, a single-chain antibody or Fab fragment has one binding site, while a "bispecific" or "bifunctional" antibody has two different binding sites.

As used herein, the term "selectively binds to" refers to the specific binding of one protein to another (for instance, an antibody, fragment thereof, or binding partner to an antigen), wherein the level of binding, as measured by any standard assay (for example, an immunoassay), is statistically significantly higher than the background control for the assay. For example, when performing an immunoassay, controls typically include a reaction well/tube that contain antibody or antigen binding fragment alone (for instance, in the absence of antigen), wherein an amount of reactivity (for instance, non-specific binding to the well) by the antibody or antigen binding fragment thereof in the absence of the antigen is considered to be background.

In some examples, an antibody specifically binds to the extracellular domain of a TAM receptor (*e.g.,* Tyro3, Axl, or Mer) or ligand thereof (*e.g.,* Gas6 or Protein S) with a binding constant that is at least 10³ M⁻¹ greater, 10⁴ M⁻¹ greater or 10⁵ M⁻¹ greater than a binding constant for other molecules in a sample. In some examples, such antibodies (*e.g*., monoclonal antibody) or fragments thereof has an equilibrium constant (K_{d}) of 1 nM or less. For example, antibodies that bind to a TAM receptor or ligand thereof with a binding affinity of at least about 0.1 x 10⁻⁸ M, at least about 0.3 x 10⁻⁸M, at least about 0.5 x 10⁻⁸M, at least about 0.75 x 10⁻⁸ M, at least about 1.0 x 10⁻⁸M, at least about 1.3 x 10⁻⁸ M at least about 1.5 x 10⁻⁸M, or at least about 2.0 x 10⁻⁸ M. Kd values can, for example, be determined by competitive ELISA (enzyme-linked immunosorbent assay) or using a surface-plasmon resonance device such as the Biacore T100, which is available from Biacore, Inc., Piscataway, NJ.

Binding can be measured using a variety of methods standard in the art, including, but not limited to: Western blot, immunoblot, enzyme- linked immunosorbant assay (ELISA), radioimmunoassay (RIA), immunoprecipitation, surface plasmon resonance, chemiluminescence, fluorescent polarization, phosphorescence, immunohistochemical analysis, matrix-assisted laser desorptionlionization time-of-flight mass spectrometry, microcytometry, microarray, microscopy, fluorescence activated cell sorting (FACS), and flow cytometry.

In some examples, an anti-TAM receptor antibody or antigen binding fragment thereof is a competitive inhibitor of the binding of a Tyro3, Axl, or Mer ligand (for instance, Gas6 or Protein S). A competitive inhibitor is an inhibitor (for instance, a small molecule inhibitor, antibody, or antigen binding fragment or polypeptide) that binds to Tyro3, Axl, or Mer that is expressed by a cell, and that significantly reduces or inhibits the binding of a Tyro3, Axl, or Mer ligand (for instance, Gas6, Protein S, amino-terminally truncated Gas6, or an amino-terminally truncated Protein S) to the Tyro3, Axl, or Mer that is expressed by the cell. A competitive inhibitor can bind to the target with a greater affinity for the target than the Tyro3, Axl, or Mer ligand. Competition assays can be performed using standard techniques in the art (for instance, competitive ELISA or other binding assays). For example, competitive inhibitors can be detected and quantified by their ability to inhibit the binding of Tyro3, Axl, or Mer to another, labeled anti-Tyro3, Axl, or Mer antibody or ligand.

Isolated antibodies can include serum containing such antibodies, or antibodies that have been purified to varying degrees. Whole antibodies can be polyclonal or monoclonal. Alternatively, functional equivalents of whole antibodies, such as antigen binding fragments in which one or more antibody domains are truncated or absent (for instance, Fv, Fab, Fab', or F(ab)2 fragments), as well as genetically- engineered antibodies or antigen binding fragments thereof, including single chain antibodies, humanized antibodies, antibodies that can bind to more than one epitope (for instance, bi-specific antibodies), or antibodies that can bind to one or more different antigens (for instance, bi- or multi-specific antibodies), also can be used.

In one embodiment, an anti-Axl antibody (or an antibody to a ligand thereof) is a humanized antibody. Humanized antibodies are molecules having an antigen binding site derived from an immunoglobulin from a non-human species, the remaining immunoglobulin-derived parts of the molecule being derived from a human immunoglobulin. The antigen binding site can include either complete variable regions fused onto human constant domains or only the complementarity determining regions (CDR5) grafted onto appropriate human framework regions in the variable domains. Humanized antibodies can be produced, for example, by modeling the antibody variable domains, and producing the antibodies using genetic engineering techniques, such as CDR grafting. A description various techniques for the production of humanized antibodies can be found, for example, in Morrison et al. (1984) Proc. Natl. Acad. Sci. USA 81:6851-55; Whittle et al. (1987) Prot. Eng. 1:499-505; Co et al. (1990) J. Immunol. 148:1149-1154; Co et al. (1992) Proc. Natl. Acad. Sci. USA 88:2869-2873; Carter et al. (1992) Proc. Natl. Acad. Sci. 89:4285-4289; Routledge et al. (1991) Eur. J. Immunol. 21:2717- 2725 and PCT Patent Publication Nos. WO 91/09967; WO 91/09968 and WO 92/113831.

Other embodiments include fully human antibodies. One method to produce such antibodies having a particular binding specificity includes obtaining human antibodies from immune donors (for instance, using EBV transformation of B-cells or by PCR cloning and phage display). In addition, and more typically, synthetic phage libraries have been created that use randomized combinations of synthetic human antibody V-regions. By selection on the antigen, fully human antibodies can be made in which the V-regions are very human-like in nature. Finally, fully human antibodies can be produced from transgenic mice. Specifically, transgenic mice have been created which have a repertoire of human immunoglobulin germline gene segments. Therefore, when immunized, these mice produce human-like antibodies. All of these methods are known in the art.

Genetically engineered antibodies include those produced by standard recombinant DNA techniques involving the manipulation and re- expression of DNA encoding antibody variable and/or constant regions. Particular examples include, chimeric antibodies, where the VH and/or VL domains of the antibody come from a different source as compared to the remainder of the antibody, and CDR grafted antibodies (and antigen binding fragments thereof), in which at least one CDR sequence and optionally at least one variable region framework amino acid is derived from one source, and the remaining portions of the variable and the constant regions (as appropriate) are derived from a different source. Construction of chimeric and CDR-grafted antibodies is described, for example, in European Patent Applications EP-A 0194276, EP- A 0239400, EP- A 0451216 and BP-A0460617. In one example, chimeric antibodies are produced that include antibody variable domains that bind to Tyro3, Axl, or Mer, and fused to these domains is a protein that serves as a second targeting moiety. For example, the targeting moiety can include a protein that is associated with the cell or tissue to be targeted or with a particular system in the animal.

Methods of generating antibodies (such as monoclonal or polyclonal antibodies) are well established in the art (for example, see Harlow and Lane, Antibodies: A Laboratory Manual, Cold Spring Harbor Laboratory, New York, 1988). Generally, in the production of a polyclonal antibody, a suitable experimental animal, such as, for example, a rabbit, a sheep, a hamster, a guinea pig, a mouse, a rat, or a chicken, is exposed to an antigen against which an antibody is desired (*e.g*., against an extracellular TAM receptor domain or ligand thereof). Typically, an animal is immunized with an effective amount of antigen that is injected into the animal. An effective amount of antigen refers to an amount needed to induce antibody production by the animal. The animal's immune system is then allowed to respond over a pre-determined period of time. The immunization process can be repeated until the immune system is found to be producing antibodies to the antigen. In order to obtain polyclonal antibodies specific for the antigen, serum is collected from the animal that contains the desired antibodies (or in the case of a chicken, antibody can be collected from the eggs). Such serum is useful as a reagent. Polyclonal antibodies can be further purified from the serum (or eggs) by, for example, treating the serum with ammonium sulfate.

Monoclonal antibodies can be produced according to the methodology of Kohler & Milstein (Nature 256:495-497, 1975), or using the human B- cell hybridoma method (Kozbor (1984) Immunol, 133:3001; Brodeur et al., Monoclonal Antibody Production Techniques and Applications, pp. 51-63 (Marcel Dekker, Inc., New York, 1987). For example, B lymphocytes are recovered from the spleen (or any suitable tissue) of an immunized animal and then fused with myeloma cells to obtain a population of hybridoma cells capable of continual growth in suitable culture medium. Hybridomas producing the desired antibody are selected by testing the ability of the antibody produced by the hybridoma to bind to the desired antigen. The hybridomas can be cloned and the antibodies can be produced by and then isolated from the hybridomas. An exemplary method for producing a monoclonal TAM receptor (or TAM receptor ligand) antibody includes (a) administering to an animal an effective amount of a protein or peptide (for instance, a Tyro3, Axl, or Mer extracellular domain or ligand thereof) to produce the antibodies, and (b) recovering the antibodies. As used herein, the term "monoclonal antibody" includes chimeric, humanized, and human forms of a monoclonal antibody. Monoclonal antibodies often are synthesized in the laboratory in pure form by a single clone (population) of cells. These antibodies can be made in large quantities and have a specific affinity for certain target antigens which can be found on the surface of cells.

In one example, monoclonal antibody to a TAM receptor (*e.g.,* an epitope of the extracellular domain) or TAM receptor ligand (or epitope of the ligand) can be prepared from murine hybridomas according to the classical method of Kohler and Milstein (Nature, 256:495, 1975) or derivative methods thereof. In one exemplary method, a mouse (such as Balb/c 6-8 weeks old) are immunized is repetitively inoculated (*e.g.,* 3-6 times) with a few micrograms of the selected peptide or carrier conjugate thereof over a period of a few weeks. In some examples, mice can be injected three times intradermally into the base of the tail on days 0, 10, and 20 using an insulin syringe with a 28-gauge needle attached. Serum can be drawn on days 30 and 45 for evaluation of the anti-serum titer. The mouse is then sacrificed, and the antibody-producing cells of the spleen isolated. Spleens can be harvested about 80-90 hours after the last cell boost for cell fusion. The spleen cells are fused by means of polyethylene glycol with mouse myeloma cells, and the excess unfused cells destroyed by growth of the system on selective media comprising aminopterin (HAT media). For example, cell fusions of the splenocytes can be performed according to the protocol of Oi and Herzenberg (Selected Methods in Cellular Immunology, Freeman Press, San Francisco, 1980). Splenocytes and SP2/0 cells are mixed, for example at a 4:1 ratio. The mixed cells are centrifuged and the cell pellet resuspended in polyethylene glycol (such as 40% -50% (w/v) polyethylene glycol) and appropriate medium. The resulting suspension is centrifuged and the cell pellet resuspended in HAT medium, and seeded in 96-well plates at 100 µl/ well (2.5x10⁵ cells/well) and cultured in a CO₂ incubator. On the day after fusion, 100 µl of fresh HAT medium containing 500 µg/ml geneticin (Invitrogen) is added. On days 4 and 7, half of the spent medium is replaced by fresh HAT medium containing 250 µg/ml geneticin. On day 8, the growth of the hybridoma in each well is checked under a microscope. The successfully fused cells are diluted and aliquots of the dilution placed in wells of a microtiter plate where growth of the culture is continued. Antibody-producing clones are identified by detection of antibody in the supernatant fluid of the wells by immunoassay procedures, such as ELISA, as originally described by Engvall (Enzymol., 70:419, 1980), and derivative methods thereof. For example, mAb production in culture supernatants can be assayed on day 10 by ELISA assay or days 9 and 10 by FACS sorter. Positive clones can be expanded and the specific hybridomas cloned by a limiting dilution method. Selected positive clones can be expanded and their monoclonal antibody product harvested for use.

In another example, an anti-Tyro3, Axl, or Mer (or ligand thereof) monoclonal antibody is produced recombinantly. For example, once a cell line expressing an antibody, for example a hybridoma, has been obtained, it is possible to clone therefrom the cDNA and to identify the variable region genes encoding the desired antibody, including the sequences encoding the CDRs. Then, antibodies and antigen binding fragments can be obtained by preparing one or more replicable expression vectors containing at least the DNA sequence encoding the variable domain of the antibody heavy or light chain and optionally other DNA sequences encoding remaining portions of the heavy and/or light chains as desired, and transforming/transfecting an appropriate host cell, in which production of the antibody will occur. Suitable expression hosts include bacteria, (for example, an *E. coli* strain), fungi, (in particular yeasts (for instance, members of the genera *Pichia, Saccharomvces,* or *Kluyverornyces*), and mammalian cell lines, (for example, a non-producing myeloma cell line, such as a mouse NSO line, or CHO cells). In order to obtain efficient transcription and translation, the DNA sequence in each vector includes appropriate regulatory sequences, particularly a promoter and leader sequence operably linked to the variable domain sequence. Particular methods for producing antibodies in this way are known and routinely used. For example, basic molecular biology procedures are described by Maniatis et al. (Molecular Cloning, Cold Spring Harbor Laboratory, New York, 1989); DNA sequencing can be performed as described in Sanger et al. (PNAS 74, 5463, (1977)) and the Amersham International plc sequencing handbook; and site directed mutagenesis can be carried out according to the method of Kramer et al. (Nucleic Acids Res. 1:9441, (1984)) and the Anglian Biotechnology Ltd. handbook. Additionally, there are numerous publications, including patent specifications, detailing techniques suitable for the preparation of antibodies by manipulation of DNA, creation of expression vectors and transformation of appropriate cells, for example, as reviewed by Mountain & Adair in Biotechnology and Genetic Engineering Reviews (ed. Tombs, M P, 10, Chapter 1, 1992, Intercept, Andover, UK) and in the aforementioned European Patent Applications.

In another example, monoclonal antibody to a TAM receptor (*e.g.,* an epitope of the extracellular domain) or TAM receptor ligand can be prepared from rabbit hybridomas as described in U.S. Pat. Nos. 7,148,332, 5,675,063, or 4,859,595.

In yet another example, monoclonal antibodies to a TAM receptor (*e.g.,* an epitope of the extracellular domain) or TAM receptor ligand can be prepared by repetitively inoculating a non-human mammal (such as a mouse or rabbit) with one or more plasmids encoding a TAM receptor (*e.g.,* an epitope of the extracellular domain) or TAM receptor ligand (or fragment thereof). For example, pcDNA3 (Invitrogen, Carlsbad, CA) or a vector derived there from, can be manipulated using standard molecular biology methods to include a coding sequence for a peptide fragment of a TAM receptor (*e.g.,* an epitope of the extracellular domain) or TAM receptor ligand. In one exemplary method, Balb/c mice (6-8 weeks old) are immunized three times with the appropriate plasmid (20 µg in phosphate-buffered saline), and one boost can be given with cells before fusion. Mice can be injected three times intradermally into the base of the tail on days 0, 10, and 20 using an insulin syringe with a 28-gauge needle attached. Serum can be drawn on days 30 and 45 for evaluation of the anti-serum titer. To boost the immunized mice, cells expressing the desired plasmid are injected (for example on day at least 50). These injections can be intravenous and intraperitoneal. Spleens are harvested about 80-90 hours after the last cell boost for cell fusion. Cell fusions of the splenocytes can be performed according to the protocol of Oi and Herzenberg (Selected Methods in Cellular Immunology, Freeman Press, San Francisco, 1980). Splenocytes and SP2/0 cells are mixed, for example at a 4:1 ratio. The mixed cells are centrifuged and the cell pellet resuspended in polyethylene glycol (such as 40% -50% (w/v) polyethylene glycol) and appropriate medium. The resulting suspension is centrifuged and the cell pellet resuspended in HAT medium, and seeded in 96-well plates at 100 µl/ well (2.5x10⁵ cells/well) and cultured in a CO₂ incubator. On the day after fusion, 100 µl of fresh HAT medium containing 500 µg/ml geneticin (Invitrogen) is added. On days 4 and 7, half of the spent medium is replaced by fresh HAT medium containing 250 µg/ml geneticin. On day 8, the growth of the hybridoma in each well is checked under a microscope. mAb production in culture supernatants can be assayed on day 10 by ELISA assay or days 9 and 10 by FACS sorter. Positive clones can be expanded and the specific hybridomas cloned by a limiting dilution method.

In addition, protocols for producing humanized forms of monoclonal antibodies and fragments of monoclonal antibodies are known in the art (see, *e.g.,* U.S. Pat. Nos. 6,054,297, 6,407,213, 6,639,055, 6,800,738, and 6,719,971 and U.S. Pat. Appl. Pub. Nos. 2005/0033031, and 2004/0236078). Similarly, methods for producing single chain antibodies have been described and can be useful for the making of TAM receptor inhibitors (see, Buchner et al., Anal. Biochem. 205:263-270, 1992; Pluckthun, Biotechnology 9:545, 1991; Huse et al., Science 246:1275, 1989 and Ward et al., Nature 341:544, 1989).

Methods known to those of ordinary skill in the art (such as those described in detail herein, including Section J) can be used to screen such antibodies to identify those that are TAM receptor inhibitors, in contrast to those that are TAM receptor agonists.

### 3. Inhibitory RNA molecules (RNAi)

In yet another example, TAM receptor inhibitors are siRNAs or other inhibitory RNAs (RNAi) that can decrease or eliminate the biological activity of a TAM receptor, for example by decreasing translation of a TAM receptor or ligand thereof. One of ordinary skill in the art can readily generate siRNAs, which specifically bind to a nucleic acid encoding the TAM receptor (*e.g*., Tyro3, Axl, or Mer) or ligand thereof (*e.g.,* Gas6 or Protein S). In an example, commercially available kits, such as siRNA molecule synthesizing kits from PROMEGA® (Madison, WI) or AMBION® (Austin, TX) may be used to synthesize siRNA molecules. In another example, siRNAs are obtained from commercial sources, such as from QIAGEN® Inc (Germantown, MD), INVITROGEN® (Carlsbad, CA), AMBION (Austin, TX), DHARMACON® (Lafayette, CO), SIGMA-ALDRICH® (Saint Louis, MO) or OPENBIOSYSTEMS® (Huntsville, AL).

siRNAs are double stranded RNAs (dsRNAs) that can induce gene-specific inhibition of expression are provided. These RNAs are suitable for interference or inhibition of expression of a target TAM receptor and comprise double stranded RNAs of about 15 to about 40 nucleotides (such as 19 to 23 nucleotides) containing a 3' and/or 5' overhang on each strand having a length of 0- to about 5-nucleotides, wherein the sequence of the double stranded RNAs is substantially identical to a portion of a mRNA or transcript of the target TAM receptor or ligand thereof for which interference or inhibition of expression is desired. For example, using TAM receptor nucleic acid sequences known in the art (*e.g.,* see GenBank Accession Nos. NM_006293.2, NM_021913.3, and NM_006343.2 for Tyro3, Axl, and Mer, respectively), or using Gas6 or Protein S sequences known in the art (see for example, Genbank™ Nos: NM_000820.1 for Gas6 and NM_000313.1 for Protein S) siRNA sequences specific for such sequences can be generated using routine methods. The double stranded RNAs can be formed from complementary ssRNAs or from a single stranded RNA that forms a hairpin or from expression from a DNA vector.

In addition to native RNA molecules, RNA suitable for inhibiting or interfering with the expression of a TAM receptor or ligand thereof include RNA derivatives and analogs. For example, a non-natural linkage between nucleotide residues can be used, such as a phosphorothioate linkage. The RNA strand can be derivatized with a reactive functional group or a reporter group, such as a fluorophore. Particularly useful derivatives are modified at a terminus or termini of an RNA strand, typically the 3' terminus of the sense strand. For example, the 2'-hydroxyl at the 3' terminus can be readily and selectively derivatized with a variety of groups. Other useful RNA derivatives incorporate nucleotides having modified carbohydrate moieties, such as 2'-O-alkylated residues or 2'-deoxy-2'-halogenated derivatives. Particular examples of such carbohydrate moieties include 2'-O-methyl ribosyl derivatives and 2'-O-fluoro ribosyl derivatives. The RNA bases may also be modified. Any modified base useful for inhibiting or interfering with the expression of a TAM receptor or TAM receptor ligand can be used. For example, halogenated bases, such as 5-bromouracil and 5-iodouracil can be incorporated. The bases can also be alkylated, for example, 7-methylguanosine can be incorporated in place of a guanosine residue. Non-natural bases that yield successful inhibition can also be incorporated.

In certain examples, expression vectors are employed to express the at least one siRNA molecule. For example, siRNA molecules can be expressed within cells from eukaryotic promoters. Those skilled in the art will recognize that any nucleic acid can be expressed in eukaryotic cells using the appropriate DNA/RNA vector. The activity of such nucleic acids can be augmented by their release from the primary transcript by an enzymatic nucleic acid (see, for instance, Draper et al., PCT WO 93/23569, and Sullivan et al., PCT WO 94/02595).

In some examples, siRNA molecules are expressed from transcription units (see for example, Couture et al., 1996, TIG 12:510) inserted into DNA or RNA vectors. The recombinant vectors can be DNA plasmids or viral vectors. siRNA expressing viral vectors can be constructed based on, for example, but not limited to, adeno-associated virus, retrovirus, adenovirus, lentivirus or alphavirus. In another example, pol III based constructs are used to express siRNA nucleic acid molecules (see, for example, Thompson, U.S. Pat. Nos. 5,902,880 and 6,146,886).

In another example, an expression vector includes a nucleic acid sequence encoding at least one siRNA molecule specifically designed to inhibit the TAM receptor or TAM receptor ligand. In a particular example, the vector contains a sequence(s) encoding both strands of a siRNA molecule comprising a duplex. In another example, the vector also contains sequence(s) encoding a single nucleic acid molecule that is self-complementary and thus forms a siRNA molecule. Once delivered, the recombinant vectors capable of expressing the siRNA molecules persist in target cells. Alternatively, viral vectors can be used that provide for transient expression of nucleic acid molecules. Such vectors can be repeatedly administered as necessary. Once expressed, the siRNA molecule interacts with the target mRNA and generates an RNAi response.

### 4. Aptamers

In yet another example, TAM receptor inhibitors are aptamers that can decrease or eliminate the biological activity of a TAM receptor. One of ordinary skill in the art can readily generate aptamers specific for a TAM receptor (*e.g.,* Tyro3, Axl, or Mer).

Aptamers include single-stranded nucleic acid molecules (such as, DNA or RNA) that assume a specific, sequence-dependent shape and binds to a a TAM receptor with high affinity and specificity. Aptamers generally comprise fewer than 100 nucleotides, fewer than 75 nucleotides, or fewer than 50 nucleotides (such as 10 to 100 or 10 to 50 nucleotides). In another example, a TAM receptor inhibitor is a mirror-image aptamer (also called a SPIEGELMER™). Mirror-image aptamers are high-affinity L-enantiomeric nucleic acids (for example, L-ribose or L-2'-deoxyribose units) that display high resistance to enzymatic degradation compared with D-oligonucleotides (such as, aptamers). The target binding properties of aptamers and mirror-image aptamers are designed by an *in vitro*-selection process starting from a random pool of oligonucleotides, as described for example, in Wlotzka et al., Proc. Natl. Acad. Sci. 99(13):8898-902, 2002.

In another example, an aptamer is a peptide aptamer that binds to a TAM receptor with high affinity and specificity. Peptide aptamers include a peptide loop (*e.g*., which is specific for a TAM receptor) attached at both ends to a protein scaffold. This double structural constraint greatly increases the binding affinity of the peptide aptamer to levels comparable to an antibody's (nanomolar range). The variable loop length is typically 8 to 20 amino acids (*e.g.,* 8 to 12 amino acids), and the scaffold may be any protein which is stable, soluble, small, and non-toxic (*e.g*., thioredoxin-A, stefin A triple mutant, green fluorescent protein, eglin C, and cellular transcription factor Sp1). Peptide aptamer selection can be made using different systems, such as the yeast two-hybrid system (*e.g*., Gal4 yeast-two-hybrid system) or the LexA interaction trap system.

### D. TAM receptor agonists

TAM receptor agonists include agents that significantly increase the biological activity of a TAM receptor in a cell, for instance an agent that specifically binds to and activates a TAM receptor. For example, a TAM receptor agonist may increase the biological TAM receptor activity by at least 25%, at least 50%, at least 70%, at least 80%, at least 90%, at least 95%, at least 100%, at least 200%, or even at least 500%. Methods of measuring such activity are known in the art. In some examples, an increase in biological activity is indicated by a increase in expression of Tyro3, Axl, or Mer or combinations thereof (at the DNA, RNA, or protein level). In other examples, an increase in biological activity is indicated by a change in a downstream effect, such an increase in TAM autophosphorylation, decrease in TLR-induced cytokine production, decrease in TLR-induced stimulation of MAP kinase activation, decrease in TLR-induced NF-kB activation, or increase in SOCS1 and SOCS 3 expression. Methods of detecting such alternations in expression or activity (which in some examples are quantified) are routine, and can include western blotting, ELISA, flow cytometry, northern blotting, PCR, RT-PCR, and the like.

In some examples, TAM receptor agonists are a Tyro3, Axl, or Mer ligand, such as Gas6 and Protein S, as well as mimetics of such ligands that can activate a TAM receptor. Such agents can therefore substantially increase the interaction between the ligand and the receptor and for example can significantly increase receptor activation, for example by increasing signaling from the receptor and increasing downstream biological effects. Examples of such TAM receptor agonists are recombinant versions of Gas6 or Protein S, such as variant sequences that include insertions, deletions and truncations of such proteins that retain TAM receptor agonist activity. For example, Gas6 is a peptide which is able to activate the TAM receptor and encompasses the mature, pre-, prepro- and pro- forms of Gas6 polypeptide, either purified from a natural source, chemically synthesized or recombinantly produced.

Where the Gas6 or Protein S polypeptide has the amino acid sequence of a ProteinS or Gas6 polypeptide found in nature (such as a human Gas6 or Protein sequence, *e.g*., Genbank™ Accession No. NP_000811.1 and NP_000304.2 as of November 7, 2007, respectively), it is referred to herein as a "native" regardless of the method by which it is produced (*e.g.,* it can be isolated from an endogenous source of the molecule or produced by synthetic techniques).

In some examples, Protein S and Gas6 ligands encompass variants of native sequences, as long as such molecules are functionally active, such as being capable of activating a TAM receptor. Such variants include fragments, such as a human Gas6 or Protein S amino acid sequence in which one or more amino acid residues are added at the N- or C-terminus of, or within, the sequence, one or more amino acid residues are deleted, and optionally substituted by one or more amino acid residues, and derivatives of the above proteins, polypeptides, or fragments thereof, wherein an amino acid residue has been covalently modified so that the resulting product is a non-naturally occurring amino acid. In some examples, 1 to 50, 1 to 20, or 1 to 10 amino acid residues are changed. Such variants may be made synthetically, for example, by site-directed or PCR mutagenesis, or may exist naturally, as in the case of allelic forms and other naturally occurring variants of the translated amino acid sequence that may occur in human and other animal species. In certain examples, a Protein S or Gas6 variant shares at least about 75% (such as at least 80%, at least 90%, or at least 95%) sequence identity with a native Protein S or Gas6 amino acid o rnucleic acid coding sequence after aligning the sequences to provide for maximum homology, as determined, for example, by the Fitch et al., PNAS (USA) 80:1382-1386 (1983), version of the algorithm described by Needleman et al., J. Mol. Biol. 48:443-453 (1970).

Examples of such TAM receptor agonists are recombinant versions of Gas6 or Protein S, such as an amino-terminally truncated Gas6 or Protein S. In particular examples, the recombinant agonist has an amino-terminal truncation that lacks the 'Gla' domain, which corresponds to residues 39-92 (SEQ ID NO: 20) of the human Gas6 sequence (Genbank™ Nos: NM_000820.1; NP_000811.1) and residues 23-85 (SEQ ID NO: 21) of the human ProS1 sequence (Genbank™ Nos: NM_000313.1; NP_000304.1). The Gla domain is dispensable for TAM receptor binding and activation, however, therefore recombinant versions of Gas6 and ProS lacking this domain are potent TAM activators, but do not have side effects relating to blood coagulation. Additional examples of Gas6 or ProS truncations include full or partial deletions of one, two, three, or all four of the EGF domains of either Gas6 or ProS. In some example, the TAM receptor agonist is a Gas6 or Protein S ligand that does not include a Gla domain nor one or more of the EGF domains. Other exemplary Gas6 mutants that can be used as TAM receptor agonists are provided in U.S. Patent No. 6,255,068.

In some examples, TAM receptor agonists bind with high specificity to the TAM receptor extracellular domain. Such agents may substantially increase the interaction between a TAM receptor ligand and the receptor and/or stimulate or increase receptor activation, for example by enhancing signaling from the receptor and increasing downstream biological effects. Examples of such inhibitors can include antibodies (*e.g*., monoclonal antibodies, for example humanized monoclonal antibodies, and other antibodies and fragments thereof described above) designed to bind to TAM receptor epitopes in order to cross-link the TAM receptors, and thereby activate them through receptor dimerization and cross-phosphorylation, are used as TAM agonists. Such antibodies (as well as fragments thereof) can be obtained from commercial sources or generated as described above for TAM receptor inhibitors, except that antibodies that stimulate the receptor instead of inhibit the receptor would be selected. Methods known to those of ordinary skill in the art (such as those described in detail herein, including Section J) can be used to screen such antibodies to identify those that are TAM receptor inhibitors, in contrast to those that are TAM receptor agonists.

In other embodiments, polymer beads derivatized with phosphatidylserine (PS), or liposomes composed principally of PS, are used as therapeutic TAM receptor agonists. These PS-containing agents bind to the Gla domain of endogenously-produced TAM ligands, which are normally made by macrophages, DCs, and other cells in the body, and thereby stabilize and increase the binding affinity of these endogenous ligands.

In some embodiments, a TAM receptor agonist has an EC₅₀ of less than about 50 µM, for instance, less than about 50 nM, or less than 1 pM. In particular embodiments, a TAM receptor agonist has an EC₅₀ of about 10 µM or 20 µM, 0.05 µM to about 5 µM, 0.1 nM to 20 nM, 1 pM to about 10 µM, or 0.1 pM to 50 pM, and in particular embodiments, a TAM receptor agonist has an EC₅₀ of less than from about 0.005 nM to about 50 nM or from about 0.05 nM to about 50 nM, such as about 50 pM to about 1 nM, or about 1 nM to about 50 nM. For example, a TAM receptor agonist, including murine recombinant Gas6 and full length murine Protein S, has an EC₅₀ around 50 pM to about 50 nM, such as about 50 pM to about 1 nM or about 1 nM to about 50 nM. In addition to the known TAM receptor agonists, higher potency inhibitors are generated by chemical modification of the existing agonist. For instance, the known compounds generally work in the low micromolar range, however chemical modification makes them, in some embodiments, more potent and more specific. In one embodiment, QSAR analysis is performed using the solved Kinase Domain Crystal Structure of MERTK. Axl and Tyro3 kinases also may be modeled upon this crystal structure (see, for instance, Walker, Huang, Finerty Jr., Weigelt, Sundstrom, Arrowsmith, Edwards, Bochkarev, Dhe-Paganon, Human Proto-oncogene Tyrosine-protein Kinase MER (in press); PDB (protein data base) 2P0C). These more potent compositions will have lower EC₅₀ values.

### D. Pharmaceutical Compositions including TAM receptor inhibitors or TAM receptor agonists

The TAM receptor inhibitors and TAM receptor agonists used in the methods described herein can be formulated in a variety of ways depending on the location and type of disease to be treated or prevented. Pharmaceutical compositions are thus provided for both local (for instance, topical or inhalational) use and for systemic use. Therefore, the disclosure includes within its scope pharmaceutical compositions including at least one TAM receptor inhibitor (*e.g*., one, two or three TAM receptor inhibitors) or at least one TAM receptor agonist (for instance, one, two or three TAM receptor agonists) formulated for use in human or veterinary medicine. While the TAM receptor inhibitors or TAM receptor agonists typically will be used to treat human subjects, they also can be used to treat similar or identical diseases in other vertebrates, such other primates, dogs, cats, horses, and cows.

Pharmaceutical compositions that include at least one TAM receptor inhibitor as described herein as an active ingredient, or that include both a TAM receptor inhibitor and an additional anti-infective agent, vaccine, or anti-cancer agent as active ingredients, can be formulated with an appropriate solid or liquid carrier, depending upon the particular mode of administration chosen. Further, pharmaceutical compositions that include at least one TAM receptor agonist as described herein as an active ingredient, or that include both a TAM receptor agonist and an additional immunosuppressive agent as an active ingredient, can be formulated with an appropriate solid or liquid carrier, depending upon the particular mode of administration chosen. A suitable administration format can best be determined by a medical practitioner for each subject individually. Various pharmaceutically acceptable carriers and their formulation are described in standard formulation treatises, for instance, Remington's Pharmaceutical Sciences by E. W. Martin. See also Wang & Hanson (1988) Journal of Parenteral Science and Technology, Technical Report No. 10, Supp. 42: 2S.

The dosage form of the pharmaceutical composition is determined by the mode of administration chosen. For instance, in addition to injectable fluids, inhalational, transdermal, rectal, vaginal, and oral formulations can be employed. Inhalational preparations can include aerosols, particulates, and the like. In general, the goal for particle size for inhalation is about 1 µm or less in order that the pharmaceutical reach the alveolar region of the lung for absorption. Oral formulations can be liquid (for instance, syrups, solutions, or suspensions), or solid (for instance, powders, pills, tablets, or capsules). For solid compositions, conventional non-toxic solid carriers can include pharmaceutical grades of mannitol, lactose, starch, or magnesium stearate. Actual methods of preparing such dosage forms are known, or will be apparent, to those of ordinary skill in the art.

In one embodiment, a pharmacological composition is provided that includes at least one TAM receptor inhibitor or TAM receptor agonist and a pharmacologically acceptable carrier. Pharmacologically acceptable carriers (for instance, physiologically or pharmaceutically acceptable carriers) are well known in the art. A suitable pharmacological composition can be formulated to facilitate the use of TAM receptor inhibitors or agonists *in vivo.* Such a composition can be suitable for delivery of the active ingredient to any suitable host, such as a patient for medical application, and can be manufactured in a manner that is itself known, for instance, by means of conventional mixing dissolving, granulating, dragee-making, levigating, emulsifying, encapsulating, entrapping, or lyophilizing processes.

The compositions or pharmaceutical compositions can be administered by any route, including parenteral administration, for example, intravenous, intraperitoneal, intramuscular, intraperitoneal, intrathecal, or intra-articular injection or infusion, or by sublingual, oral, topical, intra-nasal, or transmucosal administration, or by pulmonary inhalation. When TAM receptor inhibitors or agonists are provided as parenteral compositions, for instance, for injection or infusion, they are generally suspended in an aqueous carrier, for example, in an isotonic buffer solution at a pH of about 3.0 to about 8.0, for example at a pH of about 3.5 to about 7.4, 3.5 to 6.0, or 3.5 to about 5.0. Useful buffers include sodium citrate-citric acid and sodium phosphate-phosphoric acid, and sodium acetate/acetic acid buffers.

For oral administration, the pharmaceutical compositions that include a TAM receptor inhibitor or agonist can take the form of, for example, tablets or capsules prepared by conventional means with pharmaceutically acceptable excipients such as binding agents (for instance, pregelatinised maize starch, polyvinylpyrrolidone or hydroxypropyl methylcellulose); fillers (for instance, lactose, microcrystalline cellulose or calcium hydrogen phosphate); lubricants (for instance, magnesium stearate, talc or silica); disintegrants (for instance, potato starch or sodium starch glycolate); or wetting agents (for instance, sodium lauryl sulphate). The tablets can be coated by methods well known in the art. Liquid preparations for oral administration can take the form of, for example, solutions, syrups or suspensions, or they can be presented as a dry product for constitution with water or other suitable vehicle before use. Such liquid preparations can be prepared by conventional means with pharmaceutically acceptable additives such as suspending agents (for instance, sorbitol syrup, cellulose derivatives or hydrogenated edible fats); emulsifying agents (for instance, lecithin or acacia); non-aqueous vehicles (for instance, almond oil, oily esters, ethyl alcohol or fractionated vegetable oils); and preservatives (for instance, methyl or propyl-p-hydroxybenzoates or sorbic acid). The preparations can also contain buffer salts, flavoring, coloring, and sweetening agents as appropriate.

For administration by inhalation, the TAM receptor inhibitors or agonists for use according to the present disclosure are conveniently delivered in the form of an aerosol spray presentation from pressurized packs or a nebulizer, with the use of a suitable propellant, for instance, dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount. Capsules and cartridges for use in an inhaler or insufflator can be formulated containing a powder mix of the compound and a suitable powder base such as lactose or starch.

Pharmaceutical compositions that include at least one TAM receptor inhibitor or agonist as described herein as an active ingredient normally will be formulated with an appropriate solid or liquid carrier, depending upon the particular mode of administration chosen. The pharmaceutically acceptable carriers and excipients useful in this disclosure are conventional. For instance, parenteral formulations usually include injectable fluids that are pharmaceutically and physiologically acceptable fluid vehicles such as water, physiological saline, other balanced salt solutions, aqueous dextrose, glycerol or the like. Excipients that can be included are, for instance, proteins, such as human serum albumin or plasma preparations. If desired, the pharmaceutical composition to be administered can also contain minor amounts of non-toxic auxiliary substances, such as wetting or emulsifying agents, preservatives, and pH buffering agents and the like, for example sodium acetate or sorbitan monolaurate. Actual methods of preparing such dosage forms are known, or will be apparent, to those skilled in the art.

### F. Use of TAM receptor inhibitors as adjuvants

In general, the TAM receptor inhibitors described herein can be used in conjunction with any vaccine, for instance, a vaccine that is less than fully protective, that requires one or more booster administrations, or that uses an adjuvant that is less than fully-effective or that has safety concerns. For instance, the current anthrax vaccine (BioThrax) uses an aluminum-based adjuvant, for which there are multiple side effect concerns. Specific, non-limiting examples of other vaccines that use adjuvants that have safety concerns include DTP (diptheria-tetanus-pertussis), *Haemophilus influenzae* type B, Pneumococcal conjugate, Hepatitis A, Hepatitis B, Human papillomavirus, and Rabies vaccines.

Some examples of the disclosure are methods of enhancing the immunogenicity of a vaccine. In particular examples, the vaccine is a vaccine against a bioterrorism agent. The method includes administering to the subject a therapeutically effective amount of TAM receptor inhibitor in combination with a vaccine against a bioterrorism agent, thereby enhancing the efficacy of the vaccine. The vaccine can be a preparation of antigen, DNA, protein subunit, peptide, attenuated microorganisms (including but not limited to bacteria and viruses), living microorganisms, or killed microorganisms, administered for the prevention, amelioration or treatment of a disease.

In some examples, the vaccine is a heat or formalin-killed vaccine. Examples of heat-killed vaccines in common use today are the typhoid vaccine and the Salk poliomyelitis vaccine.

In other examples, the vaccine is an acellular vaccine. Acellular vaccines are made using only the antigenic part of the disease-causing organism, for example the capsule, the flagella, or part of the protein cell wall. In still other examples, the vaccine is an attenuated vaccine. Attenuated vaccines are made by "attenuating" or weakening a live microorganism by aging it or altering its growth conditions. In still further examples, the vaccine is a toxoid.

In other examples, the vaccine is made from a related, less virulent pathogen. The related pathogen does not cause serious disease, but provides protection from the more virulent pathogen. For example, the relatively mild cowpox virus is used to protect against the similar, but often lethal, smallpox virus. In still further examples, the vaccine is a subunit vaccine or a DNA vaccine.

Thus, one or more TAM receptor inhibitors can be used in conjunction with a wide variety of vaccines, including, but not limited to vaccines for protection from a bioterrorism agent. Specific, non-limiting examples of such vaccines include a heat or formalin-killed vaccine, attenuated vaccine, protein subunit vaccine, antigen vaccine, DNA vaccine, acellular vaccine, or toxoid vaccine directed against *Bacillus anthracis, Yersinia pestis, Variola major,* tick-borne encephalitis virus (TBEV), Ebola virus, *Escherichia coli, Haemophilus influenzae,* cobra venom, shellfish toxin, botulinum toxin, saxitoxin, ricin toxin, *Shigella flexneri, S. dysenteriae* (*Shigella bacillus*), *Salmonella, Staphylococcus* enterotoxin B, *Histoplasma capsulatum,* tricothecene mycotoxin, aflatoxin. The vaccine also can be directed against cryptococcosis, brucellosis (undulant fever), coccidioidomycosis (San Joaquin Valley or desert fever), psittacosis (parrot fever), bubonic plague, tularemia (rabbit fever), malaria, cholera, typhoid, hemorrhagic fever, tick-borne encephalitis, Venezuelan equine encephalitis, pneumonic plague, Rocky Mountain spotted fever, dengue fever, Rift Valley fever, diphtheria, melioidosis, glanders, tuberculosis, infectious hepatitis, encephalitides, blastomycosis, nocardiosis, yellow fever, typhus, and Q fever. In some examples, the vaccine is an antigen from *Bacillus anthracis,* Ebola virus, tick-borne encephalitis virus (TBEV), *Yersinia pestis*, *Variola major, Histoplasma capsulatum, Haemophilus influenzae, Escherichia coli, Shigella flexneri, S. dysenteriae* (*Shigella bacillus*), *Salmonella,* or *Staphylococcus.*

In particular examples, the vaccine is an anthrax vaccine, such as, but not limited to AVA, or an anthrax antigen, such as, but not limited to Protective Antigen (PA) or recombinant Protective Antigen (rPA). Primary vaccination with AVA generally consists of three subcutaneous injections at 0, 2, and 4 weeks, and three booster vaccinations at 6, 12, and 18 months. To maintain immunity, the manufacturer recommends an annual booster injection. Because of the complexity of a six-dose primary vaccination schedule and frequency of local injection-site reactions, schedules with a reduced number of doses would be desirable. Administration of AVA in conjunction with a TAM receptor inhibitor provides a better immune response to the vaccine than use of the vaccine alone, and can result in a decreased frequency of immunizations required to attain an immune protective response.

In particular, non-limiting examples, the vaccine is a DNA sequence encoding the non-toxic protective antigen (PA) from *B. anthracis* or an immunogenic fragment thereof. The sequence for PA has been determined and has been deposited in GenBank at Accession No. M22589. Other antigens of use include, but are not limited to, *B. anthracis* lethal factor or an immunogenic fragment thereof, disclosed in U.S. Patent Publication No: 2002/0051791A1, hantavirus antigens, for example those disclosed in U.S. Patent No. 5,614,193, smallpox antigens, for example those disclosed in U.S. Patent No. 4,567,147, plague antigens, for example those disclosed in PCT Publication No. WO9824912A2, Ebola virus antigens, for example those disclosed in PCT Publication No WO0000617A2, tick-borne encephalitis antigens, for example those disclosed in U.S. Patent No. 6,372,221 and European Patent Publication EP0691404B1, *Histoplasma capsulatum* antigens, for example those disclosed in PCT Publication No WO9955874A2 and U.S. Patent No. 6,391,313, *Haemophilus influenzae* antigens, for example those disclosed in U.S. Patent No. 6,342,232, and European Patent Publication EP0432220B1, *E. coli* antigens, for example those disclosed in U.S. Patent Nos. 5,370,872, 6,077,516, and 3,975,517, *Shigella* antigens, for example those disclosed in U.S. Patent Nos. 5,077,044, 5,686,580, and 5,681,736, *Salmonella* antigens, for example those disclosed in PCT Publication No WO0170247A2, U.S. Patent Publication No. US20010021386A1, and European Patent Publication EP1112747A1, and *Staphylococcus* antigens, for example those disclosed in European Patent Publication EP0694309A3 and U.S. Patent No. 6,391,315.

The method includes administering a therapeutically effective amount of a TAM receptor inhibitor to a subject in conjunction with a vaccine, for instance a vaccine against a bioterrorism agent, thereby enhancing the immunogenicity of the vaccine. In one examples, the TAM receptor inhibitor is administered locally, such as topically or by inhalation. In another examples, the TAM receptor inhibitor is administered systemically, such as by intravenous injection, intramuscular injection, or subcutaneous injection.

An effective amount of a TAM receptor inhibitor can be administered in combination with a vaccine in a single dose, or in multiple doses. For example, boosters of the vaccine and TAM receptor inhibitor can be administered periodically after the initial administration, for example, at one month, two months, or three months after the initial administration. In specific, non-limiting examples, pulse doses of a TAM receptor inhibitor, in combination with a vaccine, are administered at 2 weeks, four weeks, 6 months, 12 months, 18 months, or yearly after the initial bolus administration.

In other examples, a subject who likely has been exposed to an infectious agent can receive a vaccine against the infectious agent in conjunction with a TAM receptor inhibitor and an anti-infective agent. For example, during a course of treatment of a subject who has been, or is likely to have been exposed to an infectious agent, the vaccine and TAM receptor inhibitor can be administered daily, weekly, or every two weeks.

TAM receptor inhibitors can be administered before vaccine administration, concurrently with vaccine administration, or after vaccine administration. For example, the TAM receptor inhibitor can be administered before the vaccine is administered, for instance, two weeks, one week, one day, or one hour before the vaccine is administered to the subject. Alternatively, the TAM receptor inhibitor can be administered concurrently with vaccine administration, or, for instance, two weeks, one week, one day, or one hour after the vaccine is administered to the subject.

Thus, the TAM receptor inhibitors described herein can be administered to a subject in combination with a vaccine in order to enhance the immunogenicity of the vaccine. The effectiveness of the TAM receptor inhibitor administration can be measured by monitoring vaccine titer or avidity of antibody response, or cytotoxic T cell response, by methods known to one of skill in the art. For example, an increase in vaccine titer or avidity of antibody response over time is an indicator of efficacy of TAM receptor inhibitor treatment.

In another examples, the TAM receptor inhibitor is used in conjunction with a dendritic cell-based vaccine, for example a tumor vaccine, for instance in order to boost the resulting immune response and overcome the characteristic immunesuppression of tumor-associated DCs. Tumor vaccines are used for the treatment of various cancers, including melanoma and lymphoproliferative disorders, such as different types of leukemias, lymphomas and myelomas. A major challenge in developing effective DC vaccines that induce a strong T-cell mediated immunity against tumors relies on the generation of immunopotent DCs. Enhanced efficacy of immune-based strategies could be achieved using TAM inhibitors.

Briefly, in a dendritic cell-based vaccine protocol, DCs are isolated from a subject with a tumor, and the cells are contacted with a tumor-associated antigen or transfected with a vector *ex vivo.* The cells are then reintroduced into the subject's body, where they initiate an immune response against the tumor. The use of DC vaccines as treatments for subjects with malignancies and chronic infectious diseases is well known (Paczesny et al., (2003) Semin. Cancer Biol. 13:439; Gandhi et al., (2002) Annu Rev. Med. 53:149). Following activation by inflammatory cytokines or microbial products, DCs possess several characteristics that are implicated in the efficient stimulation of tumor-specific T lymphocytes, including enhanced homing to lymphoid tissues, high level expression of MHC class I- and II molecules in conjunction with costimulatory molecules, and secretion of immunostimulatory cytokines (Banchereau et al., (1998) Nature 392:245). The ability of DCs to prime tumor-specific T cell responses has been demonstrated in various animal models (Flamand et al., (1994) Eur. J. Immunol. 24:605; Mayordomo et al., (1995) Nat. Med. 1:1297; Celluzzi et al., (1996) J. Exp. Med. 183:283). These studies have led to several clinical trials evaluating the efficacy of DCs loaded *ex vivo* with tumor-associated antigens to initiate protective immune responses in cancer patients (Stift et al., (2003) J. Clin. Oncol. 2 1:135; Kono et al., (2002) Clin. Cancer Res. 8:3394; Murphy et al., (1999) Prostate 38:37; Fong et al., (2001) J. Immunol. 166:4254; Dees et al., (2004) Cancer Immunol. Immunother 53:777; Chang et al. (2002) Clin. Cancer Res. 8:1021 ; Banchereau et al., (2001) Cancer Res. 6 1:6451).

Dendritic cells can be obtained from a subject (for instance, a cancer patient or a person at risk for cancer), and then the DC are loaded with a tumor-associated antigen *ex vivo,* and then introduced back into the subject. Alternatively, a cell from which a DC can be derived (for instance, monocytes or CD34+ bone marrow progenitor cells) is obtained from the subject, DC are derived therefrom, and then modified by loading with the antigen and administered to the subject as described above. Alternatively, the cell (a DC cell or precursor thereof) can be obtained from a donor subject, modified, and then introduced into a recipient subject. If the DC is not from (or derived from) the recipient, it is desirable that the DC is HLA compatible with the recipient.

One particular method of deriving human DCs is from monocytes. Blood can be obtained from a human subject, peripheral blood mononuclear cells, isolated, cultured in medium containing granulocyte-macrophage colony-stimulating factor, and/or interleukin 4 (IL-4) for a suitable time. For example, cells can be harvested after approximately 4-14 days as immature DCs, or can be induced to mature, for instance, after further incubation in the presence of lipopolysaccharide and/or tumor necrosis factor-α for from about 12 or 24 hours to about 1, 2, 3 or 4 days.

Multiple techniques have been employed for loading DCs with tumor-associated antigens, including pulsing with MHC class I- and/or II-restricted peptides (Thurner et al., (1999) J. Exp. Med. 190:1669; Nestle et al., (1998) Nat. Med. 4:328; Schuler-Thurner et al., (2002) J. Exp. Med. 195:1279), incubation with tumor cell lysates (Nestle et al., (1998) Nat. Med. 4:328), and electroporation with tumor cell RNA (Heiser et al., (2002) J. Clin. Invest. 109:409). In one example, the loading of the DC with tumor-associated antigens is enhanced by carrying out the loading steps in the presence of a TAM receptor inhibitor. For instance, in one specific example, an effective amount of a TAM receptor inhibitor is added to the DC culture medium prior to, during, or after exposing the DC to antigen.

In another example, a TAM receptor inhibitor is used to enhance an immune response in the subject when the DCs are reintroduced into the subject. In one example, the cells are then reintroduced into the subject in conjunction with an effective amount of a TAM receptor inhibitor. As described above for other types of vaccines, the TAM receptor inhibitor can be administered locally or systemically, such as by intravenous injection, intramuscular injection, or subcutaneous injection. The TAM receptor inhibitor can be administered in a single dose, or in multiple doses. For example, in some examples, the TAM receptor inhibitor can be administered periodically after the initial administration, for example, days, weeks, or months after the initial administration. Administration of the TAM receptor inhibitor can occur prior to reintroduction of the DCs, substantially contemporaneously with reintroduction of the DCs, or after reintroduction of the DCs.

Furthermore, tumors have been shown to produce various factors that induce suppression of tumor associated DCs. The TAM receptors and their ligands often are highly expressed in tumor samples. Without being bound by theory, it is believed that increased TAM signaling may lead to tumor progression due to the intrinsic oncogenic potential of this RTK family, as well as the induction of pathological tumor tolerance through the inhibition of DCs. The use of TAM inhibitors in this setting constitutes a novel therapeutic approach to cancer treatments.

### G. Methods of treating sepsis with a TAM receptor inhibitor

As described herein, TAM receptor inhibitors are useful for the treatment of sepsis. Sepsis is a serious medical condition characterized by a whole-body inflammatory state caused by infection. Such an infection can be caused by pathogenic gram-negative and gram-positive bacteria, anaerobic bacteria, fungi, yeast, or polymicrobial organisms. Sepsis is characterized by several distinct stages including, but not limited to, the onset of sepsis, severe sepsis, septic shock, and multiple organ dysfunction associated with the end stages of sepsis. TAM receptor inhibitors generally are most effective when used after the early stages of sepsis, for instance, when the subject's condition progresses to "severe sepsis" or beyond. The signs and symptoms of severe sepsis can be subtle. Infection and associated organ dysfunction are two requirements for a diagnosis of severe sepsis, but other clinical indicators also can point to the development of severe sepsis. For instance, subjects in whom severe sepsis should be suspected include ICU patients receiving anti-infectives, subjects with severe community-acquired pneumonia, subjects who have had intra-abdominal surgery, subjects with meningitis, subjects with chronic diseases (including diabetes, heart failure, chronic renal failure, and hepatitis), subjects with compromised immune status (HIV/AIDS, use of cytotoxic and immunosuppressive agents, malignant neoplasms, and alcoholism), subjects with cellulitis, subjects with peritonitis, and subjects with a urinary tract infection. In children, sepsis can accompany infection of the bone (osteomyelitis). In hospitalized patients, common sites of infection include intravenous lines, surgical wounds, surgical drains, and sites of skin breakdown known as decubitus ulcers or bedsores.

The infection is often confirmed by a positive blood culture, though blood cultures can be negative in individuals who have been receiving antibiotics. In some instances, a change in mental status and hyperventilation can be the earliest signs of impending sepsis. Other signs of impending sepsis include fever or hypothermia, hyperventilation, chills, shaking, warm skin, skin rash, rapid heart beat, confusion or delirium, and decreased urine output. Test results that are indicative of sepsis include a low or high white blood cell count, a low platelet count, a blood culture that is positive for bacteria, blood gases that reveal acidosis, abnormal kidney function tests (early in the course of disease), a peripheral smear that demonstrates a low platelet count and destruction of red blood cells, elevated fibrin degradation products, and a blood differential with immature white blood cells.

The later stages of sepsis are characterized by organ dysfunction, hypoperfusion abnormalities, and sepsis-induced hypotension. Hypoperfusion abnormalities include, but are not limited to, lactic acidosis, oliguria, or an acute alteration in mental status. Hypotension is defined by a systolic arterial pressure below 90 mm Hg (or, in children, below normal for their age), a MAP below 60, or a reduction in systolic blood pressure of greater than 40 mm Hg from baseline, despite adequate volume resuscitation, in the absence of other causes for hypotension.

The traditional therapy for sepsis includes antibiotics, surgical drainage of infected fluid collections, fluid replacement, and appropriate support for organ dysfunction, for instance, hemodialysis in kidney failure, mechanical ventilation in pulmonary dysfunction, transfusion of blood products, and drug and fluid therapy for circulatory failure. Patients who succumb to sepsis typically do so due to immunosuppression that occurs in the later stages of sepsis, for instance during sever sepsis and beyond. Previous exposure to the LPS of gram-negative bacteria, for example, leads to a decreased capacity to respond to subsequent challenges, and the immune system eventually shuts down in the face of infection (Cook et al., (2004) Nat Immunol. 5(10):975-9). Multiorgan dysfunction can be caused, at least in part, by a shift from the inflammatory stage of sepsis to an anti-inflammatory phenotype. In advanced sepsis, activated helper cells evolve from a Th1 phenotype, producing proinflammatory cytokines, to a Th2 phenotype, producing antiinflammatory cytokines. In addition, apoptosis of circulation and tissue lymphocytes (B cells and CD4+ T cells) contributes to immunosuppression (Russell, (2006) N Engl J Med, 355:16). In addition, SOCS levels are maximal in advanced sepsis. It is at this point in the development of sepsis that administration of TAM receptor inhibitors is most beneficial.

Thus, disclosed in one example is a method of treating sepsis that involves administering a therapeutically effective amount of a TAM receptor inhibitor to a subject in need of immunoenhancement, for instance a subject with advanced sepsis, thereby treating the sepsis. In one example, the TAM receptor inhibitor is administered systemically, such as by intravenous injection, intramuscular injection, or subcutaneous injection. In some examples, subjects are screened to determine if they have sepsis prior to administration of the TAM receptor inhibitor.

An effective amount of a TAM receptor inhibitor can be administered in a single dose, or in multiple doses. For example, in some example, a TAM receptor inhibitor is administered periodically after the initial administration, for example, twice a day or more. In other examples, a TAM receptor inhibitor is administered as a continuous infusion. In still other examples, for a subject who is suffering from advanced sepsis, a TAM receptor inhibitor is administered in conjunction with one or more anti-infectious agents. Specific, non-limiting examples of suitable anti-infectious agents include anti-fungal compounds, anti-viral compounds, and antibiotics. Antibiotics include, but are not limited to, amoxicillin, clarithromycin, cefuroxime, cephalexin ciprofloxacin, doxycycline, metronidazole, terbinafine, levofloxacin, nitrofurantoin, tetracycline, and azithromycin. Anti-fungal compounds, include, but are not limited to, clotrimazole, butenafine, butoconazole, ciclopirox, clioquinol, clioquinol, clotrimazole, econazole, fluconazole, flucytosine, griseofulvin, haloprogin, itraconazole, ketoconazole, miconazole, naftifine, nystatin, oxiconazole, sulconazole, terbinafine, terconazole, tioconazole, and tolnaftate. Anti-viral compounds, include, but are not limited to, zidovudine, didanosine, zalcitabine, stavudine, lamivudine, abacavir, tenofovir, nevirapine, delavirdine, efavirenz, saquinavir, ritonavir, indinavir, nelfinavir, saquinavir, amprenavir, and lopinavir. Anti-infectious agents also include hyper-immune globulin. Hyperimmune globulin is gamma globulin isolated from a donor, or from a pool of donors, that have been immunized with a substance of interest. Specifically, hyper-immune globulin is antibody purified from a donor who was repeatedly vaccinated against a pathogen. TAM receptor inhibitors can be administered before administration of the anti-infectious agent, concurrently with administration of the anti-infectious agent, or after administration of the anti-infectious agent.

### H. Use of TAM receptor inhibitors in immunocompromised subjects

Another method disclosed herein is a method of using one or more TAM receptor inhibitors for increasing an immune response to an opportunistic infection in an immunocompromised subject. In some examples, administration of a TAM receptor inhibitor at a therapeutic dose treats the opportunistic infection. Immunocompromised subjects are more susceptible to opportunistic infections, for example viral, fungal, protozoan, or bacterial infections, prion diseases, and certain neoplasms. Those who can be considered to be immunocompromised include, but are not limited to, subjects with AIDS (or HIV positive), subjects with severe combined immune deficiency (SCID), diabetics, subjects who have had transplants and who are taking immunosuppressives, and those who are receiving chemotherapy for cancer. Immunocompromised individuals also include subjects with most forms of cancer (other than skin cancer), sickle cell anemia, cystic fibrosis, those who do not have a spleen, subjects with end stage kidney disease (dialysis), and those who have been taking corticosteroids on a frequent basis by pill or injection within the last year. Subjects with severe liver, lung, or heart disease also can be immunocompromised.

In some examples, the immunocompromised subject is infected with a lentivirus. Lentiviruses include, but are not limited to human immunodeficiency virus type 1 (HIV-1), human immunodeficiency virus type 2 (HIV-2), simian immunodeficiency virus agm (SIVagm), simian immunodeficiency virus mnd (SIVmnd), simian immunodeficiency virus syk (SIVsyk), simian immunodeficiency virus col (SIVcol), Visna-Maedi virus (VMV), bovine immunodeficiency virus (BIV), feline immunodeficiency virus (FIV), caprine arthritis-encephalitis virus (CAEV), and equine infectious anemia virus (EIAV). In some examples, the lentivirus is human immunodeficiency virus type 1 (HIV-1). In some examples, the lentivirus is human immunodeficiency virus type 2 (HIV-2).

In some examples, the opportunistic infection is infection with Leishmania major. In other examples, the opportunistic infection is a bacterial infection such as salmonellosis, syphilis, neurosyphilis, turberculosis, atypical mycobacterial infection, or bacillary angiomatosis (cat scratch disease); a fungal infection such as aspergillosis, candidiasis (thrush, yeast infection), coccidioidomycosis, cryptococcal meningitis, or histoplasmosis; a protozoal infection such as cryptosporidiosis, isosporiasis, microsporidiosis, *Pneumocystis Carinii* pneumonia (PCP), or toxoplasmosis; a viral infection such as *Cytomegalovirus* (CMV), hepatitis, herpes simplex (HSV, genital herpes), herpes zoster (HZV, shingles), human papiloma virus (HPV, genital warts, cervical cancer), *Molluscum Contagiosum,* oral hairy leukoplakia (OHL), or progressive multifocal leukoencephalopathy (PML); or a neoplasm, such as Kaposi's sarcoma, systemic non-Hodgkin's lymphoma (NHL), or primary CNS lymphoma, among others.

In order to increase an immune response to an opportunistic infection in a subject, a therapeutically effective amount of a TAM receptor inhibitor is administered to the subject. In one example, the TAM receptor inhibitor can be administered locally, such as by topical application or intradermal administration. TAM receptor inhibitors can be injected once, for example, or they can be injected in divided doses two or more times, for example monthly, weekly, daily, or 2-4 or more times daily. In other examples, the administration of the TAM receptor inhibitor is systemic. Oral, intravenous, intra-arterial, subcutaneous, intra-peritoneal, intra-muscular, inhalational, and even rectal administration is contemplated.

The present methods also include combinations of the TAM receptor inhibitors disclosed herein with one or more drugs useful in the treatment of an opportunistic infection. For example, the TAM receptor inhibitors disclosed herein can be administered, whether before or after exposure to a virus, in combination with effective doses of other anti-virals, immunomodulators, anti-infectives, or vaccines. The term "administration" refers to both concurrent and sequential administration of the active agents.

In one example, a combination of TAM receptor inhibitor with one or more agents useful in the treatment of a lentiviral disease is provided. In one specific, non-limiting example, the lentiviral disease is an HIV-1-induced, an HIV-2-induced, a SIV-induced, or a FIV induced disease. Specific, non-limiting examples of anti-virals include: AL-721 (from Ethigen of Los Angeles, CA), recombinant human interferon beta (from Triton Biosciences of Alameda, CA), Acemannan (from Carrington Labs of Irving, TX), gangiclovir (from Syntex of Palo Alto, CA), didehydrodeoxythymidine or d4T (from Bristol-Myers-Squibb), EL10 (from Elan Corp. of Gainesville, GA), dideoxycytidine or ddC (from Hoffman-LaRoche), Novapren (from Novaferon Labs, Inc. of Akron, OH), zidovudine or AZT (from Burroughs Wellcome), ribavirin (from Viratek of Costa Mesa, CA), alpha interferon and acyclovir (from Burroughs Wellcome), Indinavir (from Merck & Co.), 3TC (from Glaxo Wellcome), Ritonavir (from Abbott), Saquinavir (from Hoffmann-LaRoche), and others.

Specific, non-limiting examples of immuno-modulators are AS-101 (Wyeth-Ayerst Labs.), bropirimine (Upjohn), gamma interferon (Genentech), GM-CSF (Genetics Institute), IL-2 (Cetus or Hoffman-LaRoche), human immune globulin (Cutter Biological), IMREG (from Imreg of New Orleans, LA), SK&F106528, TNF (Genentech), and soluble TNF receptors (Immunex).

Specific, non-limiting examples of some anti-infectious agents used include clindamycin with primaquine (from Upjohn, for the treatment of pneumocystis pneumonia), fluconazlone (from Pfizer for the treatment of cryptococcal meningitis or candidiasis), nystatin, pentamidine, trimethaprim-sulfamethoxazole, and many others, as described above.

"Highly active anti-retroviral therapy" or "HAART" refers to a combination of drugs which, when administered in combination, inhibits a retrovirus from replicating or infecting cells better than any of the drugs individually. In one example, the retrovirus is a human immunodeficiency virus. In one example, a TAM receptor inhibitor is administered in conjunction with a highly active anti-retroviral therapy that includes the administration of 3'axido-3-deoxy-thymidine (AZT) in combination with other agents. Specific, non-limiting examples of agents that can be used in combination in HAART for a human immunodeficiency virus are nucleoside analog reverse transcriptase inhibitor drugs (NRTI), non-nucleoside analog reverse transcriptase inhibitor drugs (NNRTI), viral-entry inhibitors, integrase inhibitors, maturation inhibitors and protease inhibitor drugs (PI). One specific, non-limiting example of HAART used to suppress an HIV infection is a combination of indinavir and efavirenz, a NNRTI.

In one example, HAART is a combination of three drugs used for the treatment of an HIV infection, such as the drugs shown in Table 2 below. Examples of three-drug HAART for the treatment of an HIV infection include 1 protease inhibitor from column A plus 2 nucleoside analogs from column B in Table 1. In addition, ritonavir and saquinavir can be used in combination with 1 or 2 nucleoside analogs. As disclosed herein, all of these therapies are enhanced by combining them with administration of TAM receptor inhibitors.

| **Table 1** | |
|---|---|
| **Column A** | **Column B** |
| indinavir (Crixivan) | AZT/ddI |
| nelfinavir (Viracept) | D4T/ddI |
| ritonavir (Norvir) | AZT/ddC |
| saquinavir (Fortovase) | AZT/3TC |
| ritonavir/saquinavir | D4T/3TC |

In addition, other 3- and 4-drug combinations can reduce HIV to very low levels for sustained periods. The combination therapies that are enhanced by TAM receptor inhibitor administration are not limited to the above examples, but include any effective combination of agents for the treatment of HIV disease (including treatment of AIDS).

### I. Therapeutic Uses of TAM receptor agonists

Methods are disclosed herein for treating or preventing an immune mediated disorder in a subject. In one specific, non-limiting example, the immune mediated disorder is an autoimmune disease. In another specific, non-limiting example, the immune mediated disorder is an allergic reaction. In a further specific, non-limiting example, the immune mediated disorder is transplant rejection (for instance, graft-versus-host disease).
Autoimmune diseases include, but are not limited to inflammatory (rheumatoid) arthritis, Hashimoto's thyroiditis, pernicious anemia, inflammatory bowel disease (Crohn's disease and ulcerative colitis), psoriasis, renal, pulmonary, and hepatic fibroses, Addison's disease, type I diabetes, systemic lupus erythematosus, dermatomyositis, Sjogren's syndrome, multiple sclerosis, myasthenia gravis, Reiter's syndrome, and Grave's disease, among others. Clinical measures of response can be measured for each of these diseases. For example, a reduction in pain, reduction in inflammation of tissues (*e.g*., joints), improved tissue (kidney) function, improved ability to digest food can serve as indicators of successful immunosuppression.

In particular examples, the method includes administering a therapeutically effective amount of the TAM receptor agonist to a subject having or at risk of developing an immune-mediated inflammatory disorder, thereby treating or preventing the immune-mediated disorder. In one embodiment, the TAM receptor agonist is administered locally, such as by intra-articular injection for rheumatoid arthritis or by topical administration for psoriasis. In another embodiment, the TAM receptor agonist is administered systemically. In one embodiment, the immune-mediated disorder is an autoimmune disease, an allergy, graft-versus-host disease or a disorder associated with a transplant rejection. In particular embodiments, the immune-mediated disorder is inflammatory bowel disease (Crohn's disease and ulcerative colitis), psoriasis, renal, pulmonary, or hepatic fibrosis, type I diabetes, systemic lupus erythematosus, Sjogren's syndrome, or multiple sclerosis,.

TAM receptor agonist administration can be systemic or local. Local administration of the agonist is performed by methods well known to those skilled in the art. By way of example, for the treatment of rheumatoid arthritis, one method of administration to the knee, hip and/or shoulder of an individual is by intra-articular injection. Alternatively, in another example, for the treatment of psoriasis, a TAM receptor agonist is applied topically as an ointment or cream. In other embodiment, the administration of the TAM receptor agonist is systemic. Oral, intravenous, intra-arterial, subcutaneous, intra-peritoneal, intra-muscular, and even rectal or vaginal administration is contemplated.

In other embodiments, the method is a method of treating or preventing an autoimmune disease in a subject that involves contacting immune cells with a TAM receptor agonist ex vivo, and transferring the immune cells to a subject having or at risk of developing an autoimmune disease, thereby treating or preventing the autoimmune disease. Without being bound by theory, these immune cells act to suppress immune activation in a subject. One specific, non-limiting example is dendritic cells. In particular examples, the method is a method of treating graft-versus-host diseases or a transplant rejection. The method of treating transplant rejection includes preventing transplant rejection in a subject at risk of rejecting a transplant and treating transplant rejection in a subject in need thereof. In other examples, a TAM receptor agonist would be administered *in vivo,* such as within one day following transplant and continue thereafter, potentially for an extended period, such as for at least 3 days, at least 7 days, at least 14 days, at least 30 days as a function of any indications of transplant rejection.

In certain examples, the immune cells, such as dendritic cells, are contacted with a TAM receptor agonist, and subsequently administered to a subject. The immune cells can be delivered alone, in conjunction with a TAM receptor agonist, and/or in conjunction with an additional immunosuppressive agent. The immune cells can be delivered either systemically or locally. In specific, non-limiting examples, the cells are delivered parenterally, intravenously, intra-muscularly, subcutaneously, or intra-articularly. Precise, effective quantities of cells can be readily determined by those who are skilled in the art and will depend upon the condition being treated and the therapy being employed. Other agents, such as growth factors or immunosuppressive agents, can be administered in conjunction with the immune cells.

In another embodiment, an additional anti-inflammatory agent or immunosuppressive agent is administered in conjunction with a TAM receptor agonist. The administration of the anti-inflammatory agent or immunosuppressive agent and the TAM receptor agonist can be sequential or simultaneous. In particular examples, the immunosuppressive agent is a non-steroidal anti-inflammatory agent, such as diclofenac, diflunisal, etodolac, flurbiprofen, ibuprofen, indomethacin, ketoprofen, ketorolac, nabumetone, naproxen, oxaprozin, piroxicam, sulindac, tolmetin, celecoxib, or rofecoxib, a steroid, such as cortisone, dexamethasone, hydrocortisone, methylprednisolone, prednisolone, prednisone, or triamcinolone, or an immunosuppressive agent, for example cyclosporin, tacrolimus, mycophenolic acid, or sirolimus.

In particular examples, the immunosuppressive agent is a biological response modifier, such as Kineret® (anakinra), Enbrel® (etanercept), or Remicade® (infliximab), a disease-modifying antirheumatic drug (DMARD), such as Arava® (leflunomide), a nonsteroidal anti-inflammatory drug (NSAIDs), specifically a Cyclo-Oxygenase-2 (COX-2) inhibitor, such as Celebrex® (celecoxib) and Vioxx® (rofecoxib), or another product, such as Hyalgan® (hyaluronan) and Synvisc® (hylan G-F20).

### J. Methods of identifying immunomodulators

Other examples include methods of screening for immunomodulators, such as TAM receptor inhibitors and TAM receptor agonists. In one examples, the method includes contacting a cell expressing a TAM receptor (or one or more receptor binding domains, for instance, the SHBG domain for the ligands and the IgG domains for the receptors) with a test agent, and determining whether the test agent alters TAM receptor activity. Exemplary activities of a TAM receptor inhibitor and a TAM receptor agonist are provided in Table 2.

**Table 2: Activities of TAM receptor inhibitors or TAM receptor agonists**

| | TAM receptor inhibitor/antagonist - immunoenhancer | TAM receptor activator/agonist - immunosuppressor |
|---|---|---|
| Autophosphorylation of TAM receptor | decreases | increases |
| TLR-induced cytokine production | increases | decreases |
| TLR-induced MAP kinase | increases | decreases |
| TLR-induced NF-kB activation | increases | decreases |
| SOCS1 and SOCS3 expression | decreases | increases |

In some examples, the method is a method of screening for an immunoenhancing agent and determining whether the test agent substantially reduces or inhibits TAM receptor activity. In other examples, the method is a method of screening for an immunosuppressive agent and determining whether the test agent substantially increases TAM receptor activity. The method can include contacting the cell with the test agent and determining whether contacting the cell with the test agent: alters TAM autophosphorylation, TLR-induced cytokine production, TLR-induced stimulation of MAP kinase activation, and/or TLR-induced NF-kB activation, as compared to a control. In this example, a reduction in TAM autophosphorylation, or an increase in TLR-induced cytokine production, TLR-induced stimulation of MAP kinase activation, or TLR-induced NF-kB activation in the presence of the test agent relative to the control level indicates that the test agent inhibits TAM receptor activity, and thus is an immunoenhancer. In other examples, an increase in TAM autophosphorylation, or a decrease in TLR-induced cytokine production, TLR-induced stimulation of MAP kinase activation, or TLR-induced NF-kB activation in the presence of the test agent relative to the control level indicates that the test agent stimulates TAM receptor activity, and thus is an immunosuppressor.

Autophosphorylation assays are well known in the art. In one example, cells expressing a TAM receptor are cultured and treated with test media, for instance, for 20 minutes at 37°C. Media is aspirated off and cold lysis buffer is added to each sample. The sample is centrifuged to spin down cell nuclei, and the supernatant is mixed with protein A agarose beads and affinity purified anti-TAM receptor antibody, then incubated. Protein A beads are pelleted and washed and separated on Tris-Glycine gels, and transferred (for Western blotting) onto a PVDF membrane (Millipore). The blot is probed with anti-phosphotyrosine as the primary antibody. A substantial decrease in phosphotyrosine labeling relative to control indicates that the test agent is a TAM receptor inhibitor. A control can be a known value indicative of phosphotyrosine labeling in a sample, such as a cell, not treated with a test agent. For example, a decrease in phosphotyrosine by at least about 20%, at least about 30%, at least about 40%, at least about 50%, at least about 60%, at least about 70%, at least about 80%, or at least about 90% as compared to such a control indicates that the test agent inhibits TAM receptor activity, and thus the test agent is an immunoenhancer. In contrast, a substantial increase in phosphotyrosine labeling relative to control indicates that the test agent is a TAM receptor agonist. For example, an increase in TAM phosphotyrosine by at least about 20%, at least about 30%, at least about 40%, at least about 50%, at least about 60%, at least about 70%, at least about 80%, at least about 90%, at least about 100%, or at least about 200% as compared to such a control indicates that the test agent activates the TAM receptor and thus is an immunosuppressor.

Cytokine assays are also well known in the art. For example, cytokine assays are manufactured by Assay Designs, Inc, Ann Arbor, Michigan; AssayGate, Inc., Ijamsville, MD; and Panomics, Inc., Fremont, CA. An increase in TLR-induced cytokine production in the presence of the test agent relative to the control level indicates that the test agent inhibits TAM receptor activity. A control level can be a reference value indicative of the amount of TLR-induced cytokine production in the absence of a test agent or the amount of TLR-induced cytokine production in the absence of a test agent (such as a cell contacted with carrier alone). For example, a substantial increase in TLR-induced cytokine production by at least about 20%, at least about 30%, at least about 40%, at least about 50%, at least about 60%, at least about 70%, at least about 80%, at least about 90%, at least about 100%, or at least about 200% as compared to such a control indicates that the test agent inhibits TAM receptor activity, and thus the test agent is an immunoenhancer. In contrast, a substantial decrease in TLR-induced cytokine production relative to control indicates that the test agent is a TAM receptor agonist. For example, a decrease in TLR-induced cytokine production by at least about 20%, at least about 30%, at least about 40%, at least about 50%, at least about 60%, at least about 70%, at least about 80%, or at least about 90% as compared to such a control indicates that the test agent activates the TAM receptor, and thus the test agent is a immunosuppressor.

Further, MAP kinase activity can be determined by performing MAP kinase assays with kits that are manufactured by, for instance, MDS Analytical Technologies, Sunnyvale, CA; Millipore, Inc., Billerica, MA; Calbiochem, San Diego, CA; and Cell Signaling Technology, Danvers, MA. A substantial increase in MAP kinase activation (such as indicated by an increase in phosphorylation of p38) in the presence of the test agent relative to the control level of MAP kinase activity (such as basal levels of MAP kinase activity) indicates that the test agent inhibits TAM receptor activity. For example, an increase in MAP kinase activation by at least about 20%, at least about 30%, at least about 40%, at least about 50%, at least about 60%, at least about 70%, at least about 80%, at least about 90%, at least about 100%, or at least about 200% as compared to such a control indicates that the test agent inhibits TAM receptor activity and thus the test agent is an immunoenhancer. In contrast, a decrease in MAP kinase activity relative to control indicates that the test agent is a TAM receptor agonist. For example, a substantial decrease in MAP kinase activity by at least about 10%, at least about 20%, at least about 30%, at least about 40%, at least about 50%, at least about 60%, at least about 70%, at least about 80%, or at least about 90% as compared to such a control indicates that the test agent activates the TAM receptor and thus the test agent is an immunosuppressor.

In still another example, determining whether the test agent modulates TAM receptor activity includes determining TLR-induced NF-kB activation. In this example, a substantial increase in T LR-induced NF-kB activation in the presence of the test agent relative to the control level (such as an increase in TLR-induced NF-kB activation by at least about 20%, at least about 30%, at least about 40%, at least about 50%, at least about 60%, at least about 70%, at least about 80%, at least about 90%, at least about 100%, or at least about 200% as compared to such a control indicates that the test agent inhibits TAM receptor activity) indicates that the test agent inhibits TAM receptor activity. In contrast, a substantial decrease in TLR-induced NF-kB activation relative to control indicates that the test agent is a TAM receptor agonist and thus the test agent is an immunoenhancer. For example, a substantial decrease in TLR-induced NF-kB activation by at least about 10%, at least about 20%, at least about 30%, at least about 40%, at least about 50%, at least about 60%, at least about 70%, at least about 80%, or at least about 90% as compared to such a control indicates that the test agent activates the TAM receptor and thus the test agent is an immunosuppressor. Assays for measuring NF-kB activation are known in the art. For instance kits for measuring NF-κB activation are made by Panomics, Inc., Fremont, CA, and Tebu-Bio, Inc., Boechout, Belgium.

In yet still another example, determining whether the test agent inhibits TAM receptor activity includes determining a control level of SOCS1 and SOCS 3 expression before contacting the cell with the test agent, contacting the cell with the test agent, and determining whether contacting the cell with the test agent reduces SOCS1 and SOCS 3 expression as compared to the control level of SOCS 1 and SOCS 3 expression. In this example, a reduction in SOCS1 and SOCS 3 expression in the presence of the test agent relative to the control level (such as a decrease in SOCS1 and/or SOCS 3 expression by at least about 20%, at least about 30%, at least about 40%, at least about 50%, at least about 60%, at least about 70%, at least about 80%, or at least about 90% as compared to control indicates that the test agent inhibits TAM receptor activity) indicates that the test agent inhibits TAM receptor activity and that the test agent is an immunoenhancer.

In yet even still another example, determining whether the test agent activates the TAM receptor includes determining a control level of SOCS1 and SOCS 3 expression before contacting the cell with the test agent, contacting the cell with the test agent, and determining whether contacting the cell with the test agent increases SOCS1 and SOCS 3 expression as compared to the control level of SOCS1 and SOCS 3 expression. In this example, an increase in SOCS1 and SOCS 3 expression in the presence of the test agent relative to the control level (such as an increase in SOCS1 and/or SOCS 3 expression by at least about 20%, at least about 30%, at least about 40%, at least about 50%, at least about 60%, at least about 70%, at least about 80%, at least about 90%, at least about 100%, or at least about 200% as compared to control) indicates that the test agent is a TAM receptor agonist and thus is an immunosuppressor. Methods for measuring SOCS 1/3 expression levels are known. For instance using the Assays-on-Demand Gene Expression assay (Applied Biosystems, Foster City, CA), total RNA is prepared using Trizol reagent (Invitrogen) according to the manufacturer's protocol. cDNA synthesis is carried out using High-Capacity cDNA Archive kit (Applied Biosystems, Foster City, CA) to synthesize single-stranded cDNA according to the manufacturer's protocol. PCR is performed together with endogenous control such as eukaryotic 18S rRNA. Relative quantification of the expression of SOCS 1/3 is carried out by normalizing the target gene signals with the 18S endogenous control.

In another example, screening to identify immunomodulators includes determining whether a test agent alters binding of a TAM ligand to a TAM receptor. For example, the method can include contacting a cell expressing a TAM receptor with a test agent, and determining whether the test agent inhibits binding of a ligand (*e.g.,* GAS6 or Protein S) to a TAM receptor. For example, the ligand can be labeled (*e.g*., with a fluorophore or other detectable label) and incubated with cells expressing a TAM receptor. Bound ligand can be detected using routine method in the art, such as flow cytometry, spectroscopy, and fluorescence microscopy. This assay is cell-based in some examples, whereas in other examples it is cell-free (for examples, those manufactured by Biacore, Stockholm, Sweden). A substantial decrease in ligand binding relative to control indicates that the test agent is a TAM receptor inhibitor. A control can be a known value indicative of ligand binding in a sample, such as ligand binding to a cell not treated with a test agent. For example, a decrease in ligand binding by at least about 20%, at least about 30%, at least about 40%, at least about 50%, at least about 60%, at least about 70%, at least about 80%, or at least about 90% as compared to such a control indicates that the test agent is a TAM receptor inhibitor, and thus the test agent is an immunoenhancer. In contrast, a substantial increase in ligand binding relative to control indicates that the test agent is a TAM receptor agonist. For example, an increase in ligand binding to a TAM receptor by at least about 20%, at least about 30%, at least about 40%, at least about 50%, at least about 60%, at least about 70%, at least about 80%, at least about 90%, at least about 100%, or at least about 200% as compared to such a control indicates that the test agent is a TAM receptor agonist and thus is an immunosuppressor.

The following examples are provided to illustrate certain particular features and/or embodiments. These examples should not be construed to limit the disclosure to the particular features or embodiments described.

### EXAMPLES

### Example 1: Materials and Methods

This Example describes materials and methods that were used in performing Examples 2-9. Although particular methods are described, one of skill in the art will understand that other, similar methods also can be used.

### Mice

C57BL/6J mice were purchased from The Jackson laboratory, and *STAT1* knock-out mice and genetically matched controls were purchased from Taconic. The mutations in the *Tyro3^{-l-}, Axl*^{*-*/}*⁻ Mer*^{*-*/*-*} and *Ifnar1* mice have been described previously Lu et al., (1999) Nature 398, 723-728; Muller et al. (1994) Science 264, 1918-1921).

### Antibodies

Axl (M-20), IκBβ (C-20), TRAF6, TRAF3, IFNAR1, and IFNAR2 antibodies were obtained from Santa Cruz Biotechnology; phospho-STAT1 Tyr 701, -STAT2 Tyr690, STAT3 Tyr705, STAT1, STAT2, STAT3, IκBα, phospho-P38 Thr180/Tyr 182, β-actin, and ERK 1/2 antibodies were obtained from Cell Signaling Technology; phospho-ERK1/2 Thr183/Tyr185, β-tubulin and ubiquitin antibodies were purchased from Sigma; and p38α and IFNAR1 were obtained from R&D Systems. Rabbit anti-mouse Mer and anti-mouse Tyro 3 were generated essentially as described in Lai et al., (1994) Oncogene 9, 2567-2578 and Prasad et al., (2006) Mol Cell Neurosci 33, 96-108. Peroxidase-labeled secondary antibodies were purchased from Amersham and anti-rabbit Alexa Fluor 680 and anti-mouse IRDye 800 CW obtained from Molecular Probes.

### Splenic DC isolation and Bone Marrow (BM) cultures

Spleens were removed and incubated with collagenase D (1 mg/ml; Roche) for 20 minutes at 37°C. Splenocytes were collected by homogenization through a 100-µm tissue strainer. Cells were resuspended in a Tris-NH₄Cl buffer for 3 minutes to lyse red blood cells. Splenic DCs were isolated using magnetic beads coated with anti-mouse CD11c (Miltenyi Biotec).

BM cells were isolated from femurs and tibias and incubated at 2x10⁶ cells per ml in RPMI complete medium (10% fetal bovine serum, 2 mM L-glutamine, 100 U/ml of penicillin, 100 µg/ml of streptomycin and 50 nM β-mercaptoethanol) containing 100 ng/ml of Flt3L (Amgen) for 8-10 days. At day 8, approximately 90% of the cells were CD11c⁺, and approximately 60% of these CD11c⁺ cells displayed the phenotype of the splenic CD11b⁺ conventional DC subset, while approximately 20% displayed the phenotype of B220⁺ plasmacytoid DCs.

### DC activation assays

Splenic DC or BM-DCs (1x10⁶ cells/ml) were cultured overnight in serum-free medium (StemSpan, StemCell Technologies Inc.) in the presence of the indicated concentrations of LPS from *Salmonella minnesota* R595 Re (Alexis), CpG-ODN 1668 (TCCATGACGTTCCGATGCT; SEQ ID NO: 19; Integrated DNA Technologies) or Poly I:C (InvivoGen) alone or in the presence of murine recombinant Gas6 (R&D Systems) or murine recombinant Protein S (produced as described in Prasad et al., (2006) Mol Cell Neurosci 33:96-108). Cytokine production was quantified in cell-free supernatants by ELISA (ebioscience for IL-12, IL-6 and TNFα and PBL-biomedical for IFNα) or by a luciferase assay using a cell line containing an ISRE-responsive element luciferase reporter construct for Type I IFN (Jiang et al., (2005) Nat Immunol 6:565-570).

Splenic DCs (1x10⁶ cells/ml) or BM-DCs (5x10⁶ cells/ml) were cultured in serum-free medium and treated with the indicated concentrations of murine recombinant Gas6, murine recombinant Protein S, the TLR ligands indicated above, recombinant mouse IFNα (PBL Biomedical laboratories), recombinant mouse Axl-FC chimera (R&D), cyclohexamide (Sigma) or Actinomycin D (Sigma). Whole cell lysates and RNA extraction were performed as indicated below.

### Immunoblots and Immunoprecipitations

DCs were washed in PBS and harvested in NuPage LDS sample buffer (Invitrogen) with 100µM DTT. Whole cell lysates were resolved by electrophoresis, proteins were transferred to a PVDF membrane (Immobilon P, Millipore), blocked in 3%BSA/TBS-Tween-20 and probed with the indicated antibodies. For fluorescent western blotting with the Odyssey system, proteins were transferred to an Immobilon-FL PVDF membrane (Millipore) and incubated with two primary antibodies in 0.1% casein/PBS-Tween-20. Following incubation with fluorescence-labeled secondary antibodies, membranes were scanned with the Odyssey imaging system (LI-COR).

For ubiquitylation assays, 5 x10⁶ BM-DCs per condition were harvested in PBS containing 1% SDS and boiled for 5 minutes. PBS containing 1% Triton X-100, 1mM DTT, 1 mM Na₃VO₄, 5 mM NaF and protease inhibitors (Complete, Roche) was added and the mixture was sonicated. Lysates were precleared for 1 hour by incubation with Immobilized rProteinA (RepliGen) and incubated overnight with primary antibody at 4°C. Immobilized rProteinA was added for the last 2 hours. The immunoprecipitates were washed in PBS containing 0.2% NP-40 and 0.5 M LiCl and resuspended and boiled in NuPage LDS sample buffer (Invitrogen) with 100 µM DTT.

For co-immunoprecipitation assays, DCs were lysed in 10 mM Tris HCl, pH 7.5, 150 mM NaCl, 2 mM MgCl₂, 2 mM EGTA, 6 mM β-mercaptoethanol, 1% Triton X-100, and protease inhibitors (complete) and tyrosine phosphatase inhibitors from Sigma.

### RNA extraction, reverse transcription and real-time PCR analysis

After stimulation, cells were harvested, and total RNA was isolated using the RNeasy mini kit (Qiagen). Reverse transcription was performed according to manufacturer instructions with RT Superscript III (Invitrogen). PCR reactions were performed on an ABI Prism 7700 Sequence Detection System using SYBRGreen PCR master mix (Applied Biosystems). Each reaction was normalized against the expression of β-actin or GAPDH. Analyses of dissociation curves were performed with SDS software (Applied Biosystems) to ensure the absence of nonspecific amplification. Q-PCR primers used herein are listed below.

### Flow cytometric analysis

Cells were incubated for 15 minutes with a rat mAb to CD16/32 to block Fc receptors and then with primary antibodies for 20 minutes on ice. The following mAbs against murine molecules were used: allophycocyanin- and phycoerythrin-conjugated anti-CD11c, FITC-conjugated anti-MHC class I (H-2K^{b}) and anti-MHC class II (I-A^{b}), PerCP-Cy5.5-conjugated anti-CD3, anti-CD19 and anti-NK1.1 (all from BD Pharmingen). For Axl staining, cells were processed as described, fixed with 10% paraformaldehyde, permeabilized with Saponin (0.1%, Sigma), incubated with Axl (M-20) antibody for 30 minutes and then with anti-goat Cy5 antibody (Jackson ImmunoResearch). Fluorescent cells were acquired with a FacsSort flow cytometer (Becton Dickinson) and analyzed with FlowJo (Tree Star) software.

### Statistical analysis

Differences between the means of experimental groups were analyzed with a two-tailed Student's t-test. Differences with ap value of 0.05 or less were considered significant.

### Example 2: Dendritic cell hyper-activation and expansion in TAM triple knock-outs

This Example demonstrates dendritic cell hyper-activation and expansion in TAM triple knock-outs. The immune system phenotypes of TAM TKOs indicated that their autoimmune syndromes might be due to abnormalities in APC physiology (Lu & Lemke (2001) Science 293:306-311). Therefore, the status of the DC subset of professional APCs was assessed in TAM TKO spleens. The percentage of splenic CD11c+ cells was markedly elevated in TAM TKOs relative to wild-type (WT), as was the absolute number of CD11c+ cells (FIGS. 1A, 1B). Splenic DCs were also hyperactivated in the triple mutants. CD11c+ DCs in TAM TKOs expressed higher levels of MHC class I and class II (FIG. 1C), and BAFF/BlyS (FIG. ID), which is elevated in patients with autoimmune diseases such as SLE (Collins et al., (2006) Arthritis Res Ther 8:R6).

TAM TKO CD11c+ DCs express super-elevated levels of MHC class II and B7.2 in response to intraperitoneal injection of LPS (Lu & Lemke (2001) Science 293:306-311). These and other indices of immune activation, such as autoantibody titers and splenomegaly, show a gene dosage effect through the TAM mutant series: they are more severe in *Tyro 3-*/*-Axl-*/*-*, *Axl-*/*-Mer-*/*-*, and *Tyro 3-*/-*Mer-*/*-* double mutants than in any single mutant, and are most severe in TAM triple mutants (Lu & Lemke (2001) Science 293:306-311). In agreement with these earlier observations, splenic DCs isolated from WT, *Axl-*/*-, Mer-*/*-,* or *TAM* TKO mice show progressive hyper-responsiveness to TLR activation, exhibiting an additive gene dosage effect that is consistent with the co-expression of TAM receptors documented in many cell types (Lu et al., (1999) Nature 398:723-728; Prasad et al., (2006) Mol Cell Neurosci 33:96-108). In response to the activation of TLR9 with CpG, or TLR4 with LPS, *Axl*^{*-*/*-*}, *Mer*^{*-*/*-*}, and *TA*M TKO DCs produced elevated levels of IL-6 and TNF-α relative to WT DCs, with the levels produced by triple mutant DCs being highest (FIGS. IE, IF). Activation of TLR3 with poly I:C yielded comparable results. The increase in cytokine production in mutant DCs cannot be attributed to a mutation-induced shift in subpopulations of DCs, since the percentage of conventional and plasmacytoid CD11c+ cells was comparable between WT and mutant splenic DC cultures.

### Example 3: TAM receptor activation inhibits TLR-induced cytokine production in DCs

This Example demonstrates that TAM receptor agonists inhibit TLR-driven cytokine production in WT DCs. Given the results described in Example 2, it was determined whether treatment with TAM receptor agonists might inhibit TLR-driven cytokine production in WT DCs. Analyses were performed in both splenic CD11c+ DCs, and in DCs differentiated from mouse bone marrow (BM) in the presence of FLT-3 ligand (Gilliet et al., (2002) J Exp Med 195:953-958).

In both DC populations, the major TAM receptors expressed were Axl and Mer (FIG. 1G), with no detectable Tyro 3. The TAM receptors are activated by the binding of two closely-related, soluble ligands - Gas6 and Protein (ProS; Prasad et al., (2006) Mol Cell Neurosci 33: 96-108; Stitt et al., (1995) Cell 80: 661-670). Overnight (15 hours) co-incubation of recombinant Gas6 with CpG substantially inhibited TLR9-induced production of Type I IFNs, IL6, and TNFα in wild-type DCs (FIG. 1H). TAM inhibition was not limited to TLR9. TLR4 was activated by LPS and TLR3 by poly I:C, and a comparable Gas6-mediated inhibition of LPS-induced IL6 and TNFα production (FIG. 11), and of poly I:C-induced Type I IFN, was also observed (FIG. 1J). DC viability was unaffected by Gas6 treatment, and the inhibitory effect of Gas6 was seen in both BM-DCs and DCs acutely isolated from spleen. Finally, ProS, the second agonist for TAM receptors, was equally effective at inhibiting TLR-induced cytokine production in WT DCs (FIG. 8A). Thus, TAM activation broadly inhibits the production of cytokines induced by the ligation of TLR3, TLR4, and TLR9.

### Example 4: TAM-mediated inhibition acts on conserved components of TLR signaling pathways and requires new gene expression

This Example demonstrates that TAM-mediated inhibition acts on conserved components of TLR signaling pathways and requires new gene expression. TLR activation leads to the engagement of multiple downstream signaling cascades. ERK1/2 and p38 MAP kinase, for example, are well-characterized effectors that are activated (phosphorylated) by TLR ligation (Dong et al., (2002) Annu Rev Immunol 20: 55-72). Treatment of DCs with 1 µM CpG resulted in the phosphorylation of both p38 (FIG. 2A) and ERK1/2 (FIG. 2B) within 60 minutes, and these activating phosphorylations were completely blocked by 2-hour prior incubation with Gas6 (FIGS. 2A, 2B). TLR signaling also leads to the degradation of the NF-κB inhibitors IκBα/β, and the consequent activation of NF-κB-driven transcription (Hoffmann & Baltimore (2006) Immunol Rev 210: 171-186). Treatment of BM-DCs with CpG led to the degradation of both IκBα and IκBβ beginning at 60 minutes (FIG. 2C), and this degradation was again completely blocked by prior incubation with Gas6 (FIG. 2C). The other TAM ligand, ProS, displayed similar effects in inhibiting CpG-induced phosphorylation of p38 and ERK1/2 (FIG. 8B), and CpG-induced degradation οf IκBα and IκBβ (FIGS. 8C, 8D). Thus, TAM activation inhibits conserved components of TLR signaling pathways in DCs.

TAM inhibition of these pathways operates downstream of multiple TLRs. Activation of the MAP kinase and NF-κB pathways by TLR3, for example, was also inhibited by prior treatment with Gas6 (FIGS. 9A, 9B). Similarly, activation of p38 and ERK1/2, as a consequence of TLR4 ligation, was also inhibited by prior incubation with Gas6 (FIGS. 9C, 9D). The TLR9 and TLR3 pathways are classical examples of MyD88-dependent and MyD88-independent/TRIF-dependent pathways respectively, and both of these pathways are inhibited by TAM signaling.

TAM inhibition of TLR signaling is not an acute process, but rather can require new gene expression and protein synthesis. In the presence of either the protein synthesis inhibitor cycloheximide or the RNA synthesis inhibitor actinomycin D, both the kinetics and magnitude of CpG-induced degradation of IκBα and IκBβ were the same, irrespective of the presence or absence of Gas6 (FIGS. 9D, 9E). These observations indicate that activation of the TAM receptors results in the induction of one or more genes and proteins that negatively regulate TLR signaling.

### Example 5: TAM receptor activation induces SOCS1 and SOCS3 and inhibits ubiquitylation of the TLR receptor-proximal elements TRAF3/6

This Example demonstrates that TAM receptor activation induces SOCS 1 and SOCS3 and inhibits ubiquitylation of the TLR receptor-proximal elements TRAF3/6. Several TLR inhibitors have been described (Liew et al., (2005) Nat Rev Immunol 5:446-458). A candidate-based approach was adopted to test whether any of these negative regulators might be induced in response to TAM receptor activation. The suppressor of cytokine signaling (SOCS) proteins function broadly in immune cells as inhibitors of both TLR and cytokine receptor signaling (Frobose et al., (2006) Mol Endocrinol 20:1587-1596; Mansell et al., (2006) Nat Immunol 7:148-155; Wormald & Hilton (2007) Curr Opin Hematol 14:9-15; Yoshimura et al., (2005) Arthritis Res Ther 7:100-110), and are induced in DCs by the cytokine receptor activation that follows TLR ligation. Of particular interest is SOCS1, since SOCS1 knock-out mice that are reconstituted for SOCS 1 expression in lymphocytes display many of the same autoimmune phenotypes as the TAM TKOs (Hanada et al., (2003) Immunity 19:437-450). Both SOCS1 and SOCS3 were found to be induced in BM-DCs as a direct consequence of TAM receptor activation (FIG. 3A). SOCS3 mRNA expression increased by 30 minutes after the addition of Gas6, and peak expression (approximately 10-fold) occurred around 90 minutes. SOCS1 mRNA expression increased by approximately 10-fold at 120 minutes. mRNAs encoding six other negative regulators of TLR signaling also were examined - IRAK-M (Kobayashi et al., (2002) Cell 110:191-202), the inositol phosphatase SHIP (Sly et al., (2004) Immunity 21:227-239), ATF-3 (Gilchrist et al. (2006) Nature 441: 173-178), IRF-4 (Negishi et al. (2005) Proc Natl Acad Sci USA 102:15989-15994), Triad3A (Chuang & Ulevitch (2004) Nat Immunol 5:495-502), and Tollip (Zhang & and Ghosh (2002) J Biol Chem. 277:7059-7065). In contrast to SOCS1, none of these mRNAs displayed significant induction following TAM receptor activation (FIG. 3B). Thus, the SOCS1 and SOCS3 genes are specifically induced downstream of TAM signaling.

The TNF receptor associated factors (TRAFs) are major early signal transducers for both the TNF receptor and the IL-1R/TLR superfamilies, and two of these proteins, TRAF3 and TRAF6, are critical for TLR signaling; they activate the MAPK and NF-κB signaling pathways in DCs (Hacker et al., (2006) Nature 439:204-207). TRAF6 functions as an ubiquitin ligase and is itself activated by ubiquitylation (Deng et al., (2000) Cell 103:351-361), while TRAF3 shares extensive homology with TRAF6 and also contains a RING-finger domain. Polyubiquitylated forms of both TRAF6 and TRAF3 were readily detected in BM-DCs within 30 minutes after TLR 4 activation with LPS, and this polyubiquitylation is potently inhibited by 2-hour prior incubation with Gas6 (FIGS. 3C, 3D). Thus, TAM-mediated inhibition operates at a receptor proximal point in TLR cascades, eliciting a pleiotropic down-regulation of TLR-activated signaling.

### Example 6: The IFNAR/STAT1 signaling cassette is an essential effector of TAM-mediated inhibition

This Example demonstrates that STAT1 is an effector of TAM-mediated TLR inhibition. This example describes methods to demonstrate that STAT1 mediates the induction of SOCS1/3 and other TAM-dependent genes. Inactive STAT proteins are classically activated by tyrosine phosphorylation, frequently by the JAK family of tyrosine kinases, in response to interferons, interleukins, and a variety of other extracellular signals (Levy & Darnell (2002) Nat Rev Mol Cell Biol 3:651-662). This activation results in the translocation of phosphorylated STAT dimers from the cytoplasm to the nucleus, where they drive the expression of downstream genes. Phosphorylation of STAT1 at Y701 is required for STAT1 transcriptional activation (Shuai et al., (1993) Science 261:1744-1746).

Using an antibody specific for phospho-STAT1 (Y701), it was observed that STAT1 was activated in DCs following incubation with Gas6 (FIG. 4A). This activation was inhibited by treatment with soluble Axl-Fc, which competes with TAMs for Gas6 binding (FIG. 4B), and was not seen in TAM triple mutant DCs (FIG. 10A). These observations are consistent with a report that a constitutively active and oncogenic form of chicken Mer, designated v-Eyk, stimulates constitutive tyrosine phosphorylation of STAT1, and that this stimulation requires Eyk kinase activity (Zong et al., (1996) Embo J 154515-4525). Two other members of the STAT family, STAT2 and 3, also were assayed for activation in response to the addition of Gas6 to DC cultures. Using Western blotting with specific phospho-STAT2 and 3 antibodies, TAM-mediated activation of these STATs was not detected (FIG. 10B).

In order to assess if STAT1 is required for TAM inhibition of TLR signaling, the ability of Gas6 to up-regulate SOCS1 and SOCS3 expression was assayed in BM-DCs prepared from STAT1 knock-outs. Gas6-mediated induction of SOCS1 mRNA, which is typically approximately 10-fold after 120 minutes in WT DCs, was completely abolished in these *STAT1*^{*-*/*-*} DCs (FIG. 4C). Gas6 induction of SOCS3 mRNA, while not eliminated entirely, was nonetheless substantially reduced (FIG. 4D). Basal levels of protein and mRNA for Axl, a TAM component in DCs, were comparable between WT and *STATI*^{*-*/*-*} DCs (see FIGS. 5C, 5D below). The ability of Gas6 to inhibit the expression of a panel of cytokines in response to TLR activation also was assayed in the presence or absence of endogenous STAT1. However, most of these cytokines, including IFN-α and IL-12, are themselves almost entirely dependent upon STAT1 for their TLR-induced activation, since they require a STAT1-dependent, cytokine-receptor-mediated amplification step (Gautier et al., (2005) J Exp Med 201:1435-1446; Honda et al., (2006) Immunity 25:349-360). Thus, secretion of IFNα and IL12 was not detected in *STAT1*^{*-*/*-*} DCs in response to LPS and CpG application. However, both LPS- and CpG-triggered expression of IL-6, albeit reduced, were still readily detected in STAT1-deficient DCs (FIGS. 4E, 4F).

In contrast to wild-type DCs, the expression of IL6 triggered by LPS (FIG. 4E) or CpG (FIG. 4F) was not inhibited by Gas6 in *STAT1*^{*-*/*-*} DCs. Indeed, cytokine production was often slightly elevated by Gas6 treatment in *STAT1*^{*-*/*-*} DCs (FIGS. 4E, 4F). Together, these results demonstrate that STAT1 is essential for both TAM-mediated induction of SOCS genes and inhibition of TLR-driven cytokine production in DCs. Not unexpectedly, STAT1-dependent SOCS expression is also critical for the inhibition of receptor-proximal steps of TLR cascades. TAM inhibition of LPS-induced TRAF6 ubiquitylation and LPS-induced IκBα degradation were both lost in BM-DCs prepared from *STAT1*^{*-*/*-*} mice (FIGS. 10C, 10D).

As noted above, STAT proteins, including STAT1, are typically associated with DC signal transduction cascades downstream of cytokine receptors, most notably the type I IFN receptor. It was determined, therefore, whether the STAT1-dependent, TAM-mediated inhibition of inflammation documented above might reflect a broader requirement for signaling by the type I IFN receptor (IFNAR). BM-DCs were prepared from *Ifnar* knock-outs, which are mutant for the R1 subunit of the α/β receptor and lack all type I signaling, and these mutant cells were assayed for STAT1 tyrosine phosphorylation in response to Gas6 application. In contrast to wild-type BM-DCs, *Ifnar*^{*-*/*-*} cells were incapable of activating STAT1 upon TAM receptor activation (FIG. 4G). [This effect was not due to a lack of TAM receptor expression in the IFNAR-deficient DCs, as Axl and Mer were still detected in the mutant cells; and also was not due to any Gas6 induction of type IFN production in wild-type cells, as this was not observed.] In keeping with the loss of Gas6-induced STAT1 phosphorylation, Gas6 was unable to up-regulate SOCS1 expression (FIG. 4H), and unable to inhibit LPS-induced production of IL-6, in *Ifnar*^{-/-} DCs (FIG. 4I). Consistent with these effects, the R1 chain of the type I IFN receptor and Axl physically associate in BM-DCs, in a TAM-ligand-dependent fashion, and can be reciprocally co-immunoprecipitated (FIG. 4J). These results indicate that TAM inhibition of inflammation in DCs is dependent on the presence of the type I IFN receptor. Thus, TAM receptors employ the IFNAR/STAT1 signaling cassette, which is initially used to trigger inflammation, to subsequently inhibit inflammation.

### Example 7: Regulation of TAM receptor signaling is itself a STAT1-dependent feature of the innate immune response

This Example demonstrates that regulation of TAM receptor signaling is itself a STAT1-dependent feature of the innate immune response. The above results indicate that TAM receptor signaling broadly inhibits inflammatory responses driven by TLR and cytokine receptor activation. This indicates that the TAM system is not fully engaged at the beginning of the innate immune response and, further, that some feature of the system, for instance, TAM receptor or ligand expression, is up-regulated subsequent to TLR and cytokine receptor activation.

Therefore, the expression of Axl, Mer, Gas6, and ProS mRNA and protein was assayed in BM-DCs, which were incubated for varying times with LPS, CpG, or poly I:C to activate their respective TLRs. Axl mRNA and protein levels were markedly elevated upon treatment with multiple TLR agonists (FIGS. 5A, 5B). In contrast, no significant change was detected in Mer, Gas6, or ProS mRNA expression in BM-DCs subsequent to TLR activation. [Although the addition of exogenous TAM ligands consistently stimulates TAM signaling in our DC cultures, both endogenous Gas6 and ProS mRNA were detected in cultures, indicating that in BM-DCs, as in many other cell types, TAM signaling is at least partially autocrine/paracrine (Lu et al., (1999) Nature 398:723-728; Lu & Lemke (2001) Science 293:306-311; Prasad et al., (2006) Mol Cell Neurosci 33:96-108).]

This finding of TLR-driven Axl up-regulation is of interest, given the demonstration that type I IFNs, which are themselves induced downstream of TLR activation and which can in certain settings also be immunosuppressive, also markedly up-regulate Axl expression in macrophages (Sharif et al., (2006) J Exp Med 203:1891-1901). IFN induction of Axl is required for IFN suppression of TLR- or IgG immune-complex-driven TNFα production, since no suppression is observed in Axl^{-/-} macrophages (Sharif et al., (2006) J Exp Med 203:1891-1901). In light of these findings and the results presented in FIGS. 5A and 5B, it was determined whether Axl up-regulation in response to either TLR ligation or type I IFN treatment is itself dependent on the IFNAR/STAT1 signaling cassette. This is indeed the case, for both forms of DC stimulation. Splenic DCs were isolated from either wild-type, *STAT1*^{-/-}*,* or *Ifnar*^{*-*/*-*} mice, the cells were treated for varying periods of time with either poly I:C or IFNα, and then were assayed for Axl expression by Western blot. Both poly I:C and IFNα induced substantial up-regulation of Axl in WT DCs (FIGS. 5C-5E). In marked contrast, Axl induction following treatment with either poly I:C or IFNα was lost in *STAT1*^{*-*/*-*} DCs (FIGS. 5C, 5D). At the same time, poly I:C induction of Axl was also lost in *Ifnar*^{-/-} DCs (FIG. 5E).

Together, these results indicate that the Axl up-regulation detected in response to TLR ligation is due to the induction of type I IFNs following TLR activation, and that it is these cytokines that then induce Axl. Thus, proinflammatory activation of the innate immune response results in the downstream activation of anti-inflammatory TAM signaling.

### Example 8: TLR, IFNAR/STAT1, and TAM signaling are integrated into a cyclic innate immune response

This Example demonstrates that TLR, IFNAR/STAT1, and TAM signaling are integrated into a cyclic innate immune response. Given both the results described above and the observation that TAM activation directly induces SOCS genes (FIG. 3A), it was determined whether the well-described induction of these genes in response to type I IFNs and other cytokines (Wormald & Hilton (2007) Curr Opin Hematol 14:9-15; Yoshimura et al., (2005) Arthritis Res Ther 7:100-110) depend on TAM signaling.

The ability of IFNα to elevate expression of SOCS1 mRNA was assayed in WT or *TA*M TKO splenic DCs. Although 300 U/ml IFNα stimulated robust induction of SOCS1 mRNA after 2 hours and sustained upregulation up to 6 hours in WT DCs, SOCS1 induction was significantly blunted in *TA*M TKO cells (FIG. 6A). This effect was already evident at 2 hours after the addition of IFNα, but was even more pronounced at later time points, consistent with IFNα upregulation of Axl in wild-type DCs (FIG. 5D; Sharif et al., (2006) J Exp Med 203:1891-1901). Integrated over the 6-hour time course (FIG. 6A), IFN-treated *TA*M TKO DCs expressed only ∼24% of the SOCS1 mRNA expressed by IFN-treated WT DCs. mRNA levels for two positive effectors of IFN signaling, IRF-7 and IFI-204, also were assayed in the same RNA samples. In contrast to mRNA for the SOCS 1 inhibitor, the IFNα-induced mRNA levels for these stimulators of IFN signaling were indistinguishable between WT and *TA*M TKO DCs (FIGS. 6B, 6C).

These observations led to an examination of the potential interaction of IFN receptor and TAM receptor signaling in more detail. Consistent with the above results and in contrast to the SOCS inhibitors, the IRF-7 and IFI-204 positive effectors were not induced in BM-DCs by direct TAM activation (addition of Gas6 alone; FIG. 6D). More tellingly, addition of Gas6 and IFNα in combination resulted in higher induced levels of SOCS1 mRNA than those seen with either ligand alone (FIG. 6D, left bars), whereas the expression of both IRF-7 and IFI-204 mRNA was markedly lower in BM-DCs treated with Gas6 and IFNα in combination than in cells treated with IFNα alone (FIG. 6D middle and right bars). Thus, TAMs and IFN receptors interact positively to induce the expression of cytokine signaling inhibitors, but interact negatively to inhibit the expression of cytokine signaling stimulators.

Taken together, the results of FIGS. 4, 5, and 6 indicate that TAM signaling is induced in a sequential, IFNAR/STAT1-dependent fashion subsequent to first TLR and then cytokine receptor activation. This induced TAM signaling, rather than cytokine signaling, accounts for most of the SOCS protein elevation that is seen as a consequence of cytokine receptor activation. This SOCS elevation, which appears to be a major component of TAM-mediated inhibition, depends on the ability of the TAM receptors to hijack the pro-inflammatory IFNAR/STAT1 signaling cassette to inhibit inflammation. These results indicate that the TAM pathway serves as the terminal component of a tripartite inflammatory cycle, composed of initial inflammation, subsequent cytokine amplification, and then restorative, TAM-mediated inhibition (FIG. 7).

### Example 9: Summary

This Example summarizes the results described in Examples 2-8, above.

The results detailed above demonstrate that TAM activation in DCs inhibits the secretion of a panoply of TLR- and cytokine-receptor-driven cytokines. Thus, both TAM engagement and action are integrated as components of a cycle of inflammation that is initiated by TLR ligation (FIG. 7B). The output of the first stage of this cycle is an initial burst of cytokines. This burst is then amplified via a feed-forward loop through cytokine receptors, a process that is almost entirely dependent on STAT proteins, notably STAT1 (Gautier et al., (2005) JExp Med 201, 1435-1446; Honda et al., (2006) Immunity 25, 349-360). In addition to elevated cytokine levels, the data indicate that a key output from this second stage of inflammation is the induction of Axl (FIG. 5D; Sharif et al., (2006) J Exp Med 203: 1891-1901). The data further indicate that Axl induction by both IFNAR/STAT1 signaling (FIGS. 5D, 5E), and TLR ligation (FIG. 5C), occurs largely through TLR activation of this feed-forward cytokine pathway. The final stage of the proposed inflammatory cycle involves the engagement of TAM signaling, the transcription of SOCS genes, and the pleiotropic inhibition of both cytokine receptor and TLR signaling pathways. In concert with TAM activation, this final inhibitory phase again employs the IFNAR/STAT1 signaling cassette (FIGS. 4C-4J, 10C, 10D). As discussed below, the data, and their synthesis in the cycle of FIG. 7, account for several previously described but incompletely understood response phenomena in DCs, and also have implications for the understanding and treatment of human immune system disorders.

### Control points for TAM inhibition

TAM inhibition of TLR signaling is seen in multiple read-outs, and acts at receptor-proximal points in TLR signaling cascades. TLR3-, TLR4-, and TLR9-induced activation of MAP kinases and NF-κB are all markedly reduced upon TAM engagement (FIGS. 2, 8, 9), but so is the TLR-induced ubiquitylation and activation of TRAF6 (FIG. 3C, FIG. 7A). TRAF3 is also required for a response to TLR3 and TLR4 activation (Hacker et al., (2006) Nature 439: 204-207), and since TRAF3 shares extensive homology with TRAF6 and contains a ring-finger domain, TRAF3 ubiquitylation was assayed following LPS treatment. TRAF3 is also poly-ubiquitylated upon TLR4 activation, this had not been demonstrated prior to this disclosure, and that this ubiquitylation is also inhibited by TAM activation (FIG. 3D). TRAF6 and TRAF3 function at the convergence of signaling pathways that are immediately downstream of multiple TLRs (Beutler et al., (2006) Annu Rev Immunol 24: 353-389; Saha & Cheng (2006) Cell Cycle 5: 804-807).

At the same time, TAM activation leads to the IFNAR/STAT1-dependent appearance of SOCS1 and SOCS3 (FIGS. 4C, 4D, 4H). SOCS1 has recently been demonstrated to promote the degradation of the TLR4 adaptor protein MAL (Mansell et al., (2006) Nat Immunol 7, 148-155), and SOCS3 over-expression has also been reported to inhibit TRAF6 ubiquitylation (Frobose et al., (2006) Mol Endocrinol 20: 1587-1596). Thus SOCS1 and SOCS3 are bona fide negative regulators of TLR signaling.

TAM-dependent inhibition is also exerted at the second stage of inflammation. This later inhibitory activity reflects the well-described role that SOCS1 and SOCS3 play in the inhibition of the JAK-STAT pathway and the attenuation of cytokine receptor signaling (red inhibition in FIG. 7; Wormald & Hilton (2007) Curr Opin Hematol 14: 9-15; Yoshimura et al., (2005) Arthritis Res Ther 7: 100-110). SOCS activity at this point is also cytokine-receptor-proximal. Thus, TAM-mediated, IFNAR/STAT1-dependent upregulation of SOCS1 and SOCS3 allows for the inhibition of both TLR-proximal signaling events (TRAF3/6 ubiquitylation, MAP kinase, and NF-κB signaling), and the feed-forward amplification of cytokine production through the cytokine receptor/JAK-STAT cascade. This dual activity leads to efficient pleoitropic inhibition of both first and second stage inflammation pathways.

### STAT1 redux

It was known that STAT1 plays a role as a transducer of multiple cytokine receptors, such as the interferon (α/β/γ) and interleukin receptors, in DCs and other APCs (Levy & Darnell (2002) Nat Rev Mol Cell Biol 3,: 651-662). Following cytokine receptor and associated JAK kinase activation, STAT1 becomes phosphorylated and translocates to the nucleus, where it drives the transcription of multiple pro-inflammatory target genes. Among the most prominent of these targets during the second stage of the inflammation cycle are cytokine genes themselves (Gautier et al., (2005) J Exp Med 201: 1435-1446; Honda et al., (2006) Immunity 25: 349-360). The data disclosed herein demonstrate that an additional target of IFNAR/STAT1 signaling at this time is the Axl gene (FIGS. 5C-E). At the same time that the IFNα signaling cascade is using STAT1 to amplify the cytokine burst produced at the end of the TLR-driven first stage of inflammation, this cascade is also using the same transcription factor to elevate Axl levels. That is, the seeds of the pathway that will eventually inhibit cytokine production are being sown at the same time that cytokine levels are being amplified, and STAT1 is used for both of these events.

However, this is not the end for STAT1 in inflammation. Both the data disclosed herein (FIGS. 4A, 4B) and the earlier work of Zong et al. ((1996) Embo J 15: 4515-4525) demonstrate that STAT1 is also specifically activated (tyrosine phosphorylated) as a consequence of TAM receptor activation. More significantly, it is demonstrated herein that STAT1, in conjunction with the type I IFN receptor, is required for the TAM-mediated induction of SOCS1 and SOCS3 (FIGS. 4C, 4D, 4H). The loss of Gas6-stimulated SOCS1 induction in *STAT1*^{*-*/*-*} DCs documented herein cannot be explained by lower basal levels of Axl in the mutant cells, since these levels were in fact comparable between *STAT1*^{*-*/*-*} and wild-type cells (FIGS. 5C, 5D). Thus, STAT1 activation is involved in TAM-mediated up-regulation of SOCS1 and SOCS3.

Perhaps more remarkable still is the observation that TAM-mediated up-regulation of SOCS1 appears to account for the majority of the up-regulation of this protein previously documented in response to type I IFN (Wormald & Hilton (2007) Curr Opin Hematol 14: 9-15). These earlier observations, which have been attributed to a direct, negative feedback loop in which IFN (and other cytokine) receptors are inhibited by the SOCS proteins that are induced by the STAT1 that these receptors activate, appear instead to reflect a significant indirect effect of IFNα: this cytokine induces Axl, and it is TAM receptors that then induce SOCS. The TAMs use the IFN receptor-STAT1 signaling cassette to do this, and thus hijack a pro-inflammatory pathway to inhibit inflammation. The data disclosed herein (FIG. 4J) indicate that this is achieved by binding of the TAMs to the R1 subunit of the IFN receptor, which associates with the R2 subunit, whose cytoplasmic domain binds STAT1. Taken together, these results are consistent with a TAM-mediated subversion of the STAT1 transcription factor - from immune activator that drives inflammation to immune suppressor that drives expression of the SOCS inhibitors.

What is different about the STAT1 that is activated downstream of TAM receptor/cytokine receptor engagement versus that activated solely by cytokine receptors? Without being bound by theory, it is proposed that alternative post-translational modifications of STAT1, or alternative recruitment/sequestering of cofactors/repressors such as the Twist proteins (Sharif et al., (2006) J Exp Med 203: 1891-1901) account for this 'blue STAT/red STAT' dichotomy and the differential activation of STAT1 target genes following the activation of cytokine receptors (for instance, IRF-7) versus TAM receptors (for instance, SOCS1). In this context, it is important to note that the data disclosed herein indicate that the TAM system and type I IFN receptors clearly interact, both physiologically (FIGS. 6A-6D), and physically (FIG. 4J). Similarly, an equivalent physiological and physical interaction between Axl and another cytokine receptor - the IL-15 receptor- has been documented (Caraux et al., (2006) Nat Immunol 7: 747-754; Budagian et al., (2005) EMBO J 24: 4260-4270). Without being bound by theory, activation of first a cytokine receptor alone, and then subsequently a TAM receptor/cytokine receptor complex, could have different consequences for both STAT1 modification and the induction of STAT1 transcription factor partners.

### Physiological implications of the inflammation cycle

As outlined above, the activation of TAM signaling effectively inhibits the innate immune response. This in turn indicates that in the absence of continued TLR activation and subsequent cytokine signaling, components of the TAM pathway, such as Axl, which are up-regulated in their expression or activity during the inflammation cycle, must turn over with a half-life that allows responding cells to return to baseline (gray cell in FIG. 7B). This turnover should in fact be integral to the cycle, since Axl up-regulation is itself dependent on cytokine production and IFNAR/STAT1 signaling (Sharif et al., (2006) J Exp Med 203: 1891-1901; FIGS. 5C-5E), which are inhibited by TAM signaling. De-activation of TAM signaling is required if a dendritic cell is to be fully responsive to any subsequent encounter with a new pathogen. At the same time, the cyclic nature of the inflammation response described herein means that if this encounter occurs before the TAM system has had time to wind down, then the TLR response to the new pathogen should be blunted. As such, the cycle provides an explanation for endotoxin tolerance and immunosuppression, a phenomenon in which hypo-responsiveness to TLR engagement is induced by prior TLR activation (Broad et al., (2006) Curr Med Chem 13: 2487-2502). In addition, the data disclosed herein also account for the more recently described phenomenon of cross-tolerance, in which exposure to one TLR ligand, for instance, LPS for TLR4, suppresses the subsequent response to an unrelated ligand, for instance, CpG for TLR9 (Dalpke et al., (2005) Immunology 116: 203-212). This is because the activation of any TLR should lead to the up-regulation of TAM signaling (FIGS. 7A, 7B), which in turn will inhibit many or all TLRs.

Removal of the TAM pathway from the inflammation cycle is predicted to lead to a sustained hyper-response to TLR activation, and this is what was observed in the TAM receptor knock-outs. The source of activating ligands for the TAM receptors during the inflammation cycle *in vivo* is of interest; both Gas6 and ProS are detectable in the DC cultures, and so, without being bound by theory, it is possible that at least a fraction of TAM signaling in these cells is autocrine/paracrine. At the same time, TAM ligands could be delivered to DCs by T cells, and in particular by suppressor or regulatory T cells [T(regs)]. DC/T cell interactions are reciprocal, and the activation status of DCs is subject to modulation by T(regs) (Bluestone & Tang (2005) Curr Opin Immunol 17: 638-642). These suppressor T cells play pivotal roles in the maintenance of peripheral T cell tolerance, and can exert immunosuppressive activity against DCs (Bluestone & Tang (2005) Curr Opin Immunol 17: 638-642). Primary T cells express ProS, and their secretion of this TAM ligand is induced by IL-4 (Smiley et al., (1997) Proc Natl Acad Sci USA 94: 11484-11489). At the same time, T(regs) express IL-4 receptors, and respond to IL-4 *in vitro* with increased immunosuppressive activity (Maerten et al., (2005) J Autoimmun 25: 112-120).

### TAM signaling in human biology and disease

In mice, reduced TAM receptor levels lead to autoimmune disease (Lu & Lemke (2001) Science 293: 306-311). Autoimmunity results from both sustained APC hyperactivation in the absence of TAM signaling (Lu & Lemke (2001) Science 293: 306-311; also described herein), and also from the delayed phagocytic clearance of apoptotic cells, a process in which TAM signaling also plays an important role (Scott et al. (2001) Nature 411: 207-211; Lemke & Lu (2003) Curr Opin Immunol 15: 31-36). [The signal transduction events associated with TAM activation by apoptotic cells (Sen et al. (2007) Blood 109: 653-660) are tied to those documented herein.] With regard to autoimmunity, low circulating levels of free Protein S in patients with SLE have been reported (Brouwer et al., (2004) Blood 104: 143-148; Meesters et al., (2007) Blood Coagul Fibrinolysis 18: 21-28; Song et al., (2000) Arthritis Rheum 43: 557-560). Since ProS is a TAM ligand, without being bound by theory, low ProS levels should result in reduced TAM signaling and consequently, unrestrained immune activation. In this regard, it is of interest that SLE patients are prone to thrombotic strokes (Ruiz-Irastorza et al., (2001) Lancet 357: 1027-1032), and that, in addition to its role as a TAM ligand, ProS is also a blood anti-coagulant.

Elevated TAM signaling also can lead to disease. For example, hyperactive TAM signaling may play a role in acute sepsis. Circulating Gas6 levels are consistently elevated in severe sepsis patients, relative both to patients with organ failure not related to infection and to healthy subjects matched for age and gender (Borgel et al., (2006) Crit Care Med 34: 219-222). Gas6 elevation is correlated with a patient's Organ Dysfunction and Infection (ODIN) scores and with the occurrence of septic shock. Patients who succumb to sepsis are subject to the immunosuppression phenomenon discussed above, which compromises their ability to eradicate their primary infection and also predisposes them to nosocomial infections. Previous exposure to the LPS of Gram-negative bacteria, for example, leads to a decreased capacity to respond to subsequent bacterial challenges, and the innate immune systems of immunotolerant patients eventually fail in the face of infection (Cook et al., (2004) Nat Immunol 5: 975-979). Immune tolerance and collapse are well-described phenomena in sepsis, but are incompletely understood. It is interesting to note that high levels of a TAM ligand that inhibits the innate immune response; for instance, Gas6, would facilitate just such a collapse.

Another setting in which hyperactive TAM can play a role is cancer. TAM receptors and ligands have been shown to be over-expressed in many malignancies (Green et al., (2006) Br J Cancer 94 1446-1451; Shieh et al., (2005) Neoplasia 7: 1058-1064; Sun et al., (2003) Ann Oncol 14: 898-906). Without being bound by theory, elevated TAM signaling could lead to tumor progression due to the intrinsic oncogenic potential of this receptor protein tyrosine kinase family (Lai et al., (1994) Oncogene 9: 2567-2578; Zong et al. (1996) Embo J 15: 4515-4525), but the results described herein indicate that elevated TAM signaling might also result in the induction of tumor tolerance via the inhibition of tumor-associated DCs.

Finally, one immediate therapeutic application of the results described herein is in the area of improved adjuvants for immunization. The efficacy of many recombinant-antigen- and synthetic-peptide-based vaccines is compromised both by limited immunogenicity and the requirement for repeated immunization. Although several approaches based on positive stimulation of the immune response have been attempted (Pulendran & Ahmed, (2006) Cell 124: 849-863), it is likely that stimulation of activation pathways will in general be compensated by obligatory negative feedback mechanisms such as described herein. Small molecule inhibitors that target the TAM pathway, inhibitors of the inhibitors, can get around these compensatory pathways, and are now useful as improved adjuvants.

### Conclusions

The TAM signaling network represents a previously unknown, yet powerful and broadly-acting pathway for the inhibition of inflammation in DCs. The sequential induction of this pathway by, and its integration with, upstream TLR and cytokine signaling networks delimits a cycle of inflammation that governs innate immune system homeostasis. This cycle and its regulatory mechanics have important uses for therapeutic intervention in human immune system disorders.

### Example 10: Use of a TAM receptor inhibitor as an adjuvant with a BioThrax vaccine

This Example demonstrates the use of a TAM receptor inhibitor as an adjuvant with a BioThrax vaccine. One skilled in the art will appreciate that similar methods can be used with other vaccines.

Wild-type and TAM mutant mice are immunized with BioThrax. Groups of 10 mice, both males and females at 6-8 weeks of age, across the TAM mutant series generated and maintained at the Salk Institute, are immunized via subcutaneous injection with undiluted (0.5 ml) or 1/10 dilution of BioThrax vaccine. The mice are on a mixed C57/Bl6x129sv background.

BioThrax vaccine (Anthrax Vaccine Adsorbed; United States Pharmacopeia. 27 ed. Rockville, MD, USP, 2004:p. 3042-4) is prepared as a sterile culture filtrate from the avirulent *Bacillus anthracis* V770-NP1-R strain. The vaccine is adjuvanted with Alhydrogel®, and contains approximately 600 µg aluminum per 0.5 mL dose. Dilutions of BioThrax are prepared using Dulbecco's phosphate buffered saline, without calcium or magnesium, as the diluent.

Sera are collected after immunization, and IgG concentrations of anti-PA are assayed by ELISA. Retro-orbital test bleeds are performed on mice at 2, 4, 6, and 8 weeks following immunization, and anti-PA (protective antigen) IgG concentrations are assessed by enzyme-linked immunosorbent assay (ELISA). Working standards for the assay are as described by Gu et al. (2007) (Vaccine. 25(3):526-34). In the ELISA, 96-well Maxisorp™ (Nunc, Rochester, NY) plates are coated with 100 µl of rPA at 1 µg/ml in PBS at 4 °C overnight. The plates are washed with PBS containing 0.05% Tween-20 (PBST) and mouse serum samples serially diluted in triplicate in PBST containing 5% instant non-fat dry milk (Giant Food) are added. Standards in the assay range from 16 ng/ml to 0.25 ng/mL of specific rPA antibody (IgG), prepared as described by Gu et al. (2007) (Vaccine. 25(3):526-34). After incubation at 4 °C overnight, the plates are washed with PBST and a 1:5000 dilution of HRP-labeled goat anti-mouse IgG (H + L) (KPL, Gaithersburg, MD) added, and the plates then incubated for 1 hour at 37 °C. Following a wash with PBST, 2,2'-azino-di(3-ethylbenzthiazoline-6-sulfonate) (ABTS) substrate (KPL) is added. Absorbance values are read at 405 nm. Anti-PA IgG concentrations are interpolated from a standard curve fit with a 4-parameter curve using SoftMax Pro software (Molecular Devices, Sunnyvale, CA). The limit of detection in this mouse anti-PA IgG ELISA is ∼2 ng/ml. The concentrations of serum anti-PA IgG between the different TAM mutant genotypes are compared.

Next, the neutralizing capacity of the anti-PA humoral response is assayed *in vitro.* The neutralizing capacity of the anti-PA humoral immune response throughout the TAM mutant series for anthrax lethal toxin is examined *in vitro* in a macrophage-based anthrax toxin neutralization assay (TNA). Mouse anti-PA standards used in the TNA are the same as those used in ELISAs outlined above. Cells from the J774.1 macrophage cell line (ATCC, Manassas, VA) are seeded at 5 × 10⁴ cells/well in 96-well plates in DMEM (BioWhittaker, Walkersville, MD) containing 10% FBS, 1 mM sodium pyruvate, 2 mM l-glutamine and penicillin/streptomycin. On a separate plate, mouse sera are serially diluted in medium and incubated in triplicate with 150 ng/mL of rPA and 250 ng/mL of recombinant Lethal Factor (rLF) for 1 hour at 37 °C. The mixture is then added to cells for 3.5 hours at 37 °C. Twenty-five microliters of 5 mg/mL MTT (3-(4,5-dimethyl-2-thiazolyl)-2,5-diphenyl-2H tetrazolium bromide; Sigma, St. Louis, MO) is then added for 2 hours to monitor cell death, after which time lysis buffer (20% SDS in 50% N,N-dimethylformamide (DMF), pH 4.7; Sigma) is added. Plates are read at 570 nm and neutralization activity of serum samples expressed as the NF50 (neutralization factor 50%), calculated as the effective dilution of the test serum resulting in 50% cell protection (ED50) divided by the ED50 of the reference standard. Serum samples that result in values that are below the limit of quantitation in the assay are arbitrarily assigned a value of one half the lowest possible value obtainable. For the mouse TNA, the limit of detection reported by Gu et al. (2007) (Vaccine. 25(3):526-34) was 0.06 µg/mL anti-PA IgG or 0.3 ED50 units.

The following protocols validate the efficacy of small molecule, kinase-domain-based inhibitors of TAM receptor signaling as potent immunoadjuvants for an improved anthrax vaccination regimen. By inhibiting TAM-mediated inhibition of the inflammatory response to vaccination, these small molecules substantially boost the humoral immune response to BioThrax.

Groups of 10 wild-type mice are immunized with either BioThrax alone (1:1 and 1:10), or supplemented with increasing doses of TAM small molecule inhibitors (for example, from about 1µg/kg to about 50 mg/kg). The DBA strain of mice is used for these wild-type studies, since there is an extensive prior literature of response profiles to immunization with both BioThrax and recombinant PA in this mouse strain. The TAM inhibitors are related in their mode of action to orally active drugs used in cancer therapy, and are water soluble. They can therefore be added directly to the BioThrax vaccine. Groups of 10 wild-type mice are immunized with BioThrax vaccine alone or supplemented with increasing doses of Axl (TAM) inhibitor.

As noted above, the currently available inhibitors from SuperGen and Rigel, such as AXL-9 and SGL-AXL-277, operate with IC50s in the low (1-5) µM range. Newer-generation inhibitors that inhibit TAM receptor autophosphorylation will be isolated, however, that have lower IC50 values. Thus, in one example, the TAM inhibitor is one that exhibits specificity for the TAM receptors relative to other tyrosine kinases, but which at the same time does not distinguish between Axl, Tyro 3, and Mer. (Different subsets of APCs express varying combinations of the receptors, but have always been observed to express more than one.) However, even an inhibitor that is exquisitely specific to a single TAM receptor (for instance, Axl) is useful as an adjuvant. One series of immunizations is performed at its IC50 (for instance, measured for inhibition of Axl autophosphorylation in response to 10 nM recombinant Gas6 in Axl-expressing MEFs in culture), and a second and third series at 10-fold lower and 10-fold higher concentrations than this IC50.

After immunization, sera are collected and assayed for IgG concentrations of anti-PA by ELISA. Retro-orbital test bleeds are performed on mice at 2, 4, 6, and 8 weeks following immunization with BioThrax in the presence or absence of varying concentration of TAM inhibitor, and anti-PA (protective antigen) IgG concentrations are assessed by ELISA, as described above. Once an optimal inhibitor dose for the potentiation of humoral antibody response to PA is determined, a dilution analysis is performed that is similar to that reported by Gu and colleagues (2007) Vaccine. 25(3):526-34). That is, adjuvant efficacy is assessed in vaccination through dilution of the vaccine in the presence and absence of fixed optimal concentrations of the anti-TAM-based adjuvant. Sets of 10 mice are immunized subcutaneously with either undiluted BioThrax, or with vaccine diluted 1:5, 1:10, or 1:30 in the presence or absence of the TAM inhibitor. Anti-PA IgG assays are performed on sera collected at 6 weeks (day 42).

Next, the neutralizing capacity of the anti-PA humoral response is assayed *in vitro.* The anthrax lethal toxin neutralizing capacity of the anti-PA humoral immune response in mice vaccinated with BioThrax, with and without varying concentrations of TAM inhibitor (for example, from about 1µg/kg to about 50 mg/kg) is assessed *in vitro* in a macrophage-based anthrax toxin neutralization assay (TNA) as described above.

These results can be confirmed in guinea pigs. An immunogenicity study performed in guinea pigs is analogous to those performed with DBA mice described above. Groups of 10 animals are immunized s.c. with BioThrax (1:1 and 1:10) or BioThrax + three varying concentrations of TAM inhibitor (IC50, 0.1-fold IC50, and 10-fold IC50). The guinea pig anti-PA IgG ELISA protocol does not differ significantly from the mouse ELISA, with the exception that a guinea pig anti-rPA IgG standard is used, and an HRP-labeled goat anti-guinea pig IgG secondary antibody is used for detection. The limit of detection in the mouse and guinea pig anti-PA IgG ELISAs has been reported to be 2 ng/mL and 30 ng/mL, respectively.

### Example 11: Use of a TAM Receptor Inhibitor as an Adjuvant with a DC-based Vaccine

This Example describes methods of using TAM receptor inhibitors as adjuvants with DC-based vaccines, for instance with tumor vaccines. Briefly, in a dendritic cell-based vaccine protocol, dendritic cells are isolated from a subject with a tumor, and the cells are contacted with a tumor-associated antigen or transfected with a vector *ex vivo.* The cells are then reintroduced into the subject's body, where they initiate an immune response against the tumor.

Dendritic cells (or cells from which DC can be derived, for instance, monocytes or CD34+ bone marrow progenitor cells) are obtained from a donor or from the subject (for instance, a cancer patient or a person at risk for cancer). Briefly, blood is obtained from a human subject, and the peripheral blood mononuclear cells are isolated and cultured in medium containing granulocyte-macrophage colony-stimulating factor and interleukin 4 (IL-4), or a combination of TNF, IL-1β, IL-6, and/or PGE2, also known as monocyte-conditioned media mimic, or a cytokine cocktail such as monophosphoryl lipid A plus IFNgamma, for approximately 4-14 days as mature DCs. The mature DCs are then induced to mature after further incubation in the presence of lipopolysaccharide and/or tumor necrosis factor-a for about 24-48 hours.

The DCs are loaded with tumor-associated antigens by incubation with tumor cell lysates according to the method of Nestle et al. ((1998) Nat. Med. 4:328). Briefly, day 6 BMDCs are plated in a 24-well plate containing 200 µl of medium supplemented with mGM-CSF/mIL-4 and polybrene (8 µg/ml). Concentrated tumor lysate is then added to the cells and the cells are incubated at 37 °C for 3 hours with gentle shaking every 30 minutes. The cells are then washed three times with PBS in order to collect the DCs. Antigen-exposed DCs are collected 2 days after exposure to antigen and are injected immediately into the subject as described below.

In some examples, a TAM receptor inhibitor (for instance, about 1µM to about ImM) is added to the DC culture medium contemporaneously with exposure of the DC to antigen. Approximately 5 x 10⁵ to 5 x 10¹⁰ cells are then reintroduced into the subject in conjunction with an effective amount of a TAM receptor inhibitor (for instance, about 1 µM to about 1 mM). The TAM receptor inhibitor is administered subcutaneously or by intravenous infusion in a single dose substantially contemporaneously with reintroduction of the DC into the subject, which are reintroduced by intravenous infusion. Optionally, following administration of the vaccine, periodic titers are obtained as described in Example 1 in order to assess the efficacy of the vaccine. Additionally, other measures of efficacy generally are used, such as monitoring the tumor size for growth or regression.

### Example 12: Use of a TAM receptor inhibitors as an adjuvant during vaccination -

This Example demonstrates the use of a TAM receptor inhibitor as an adjuvant during vaccination.

Normal (WT) mice as well as mice lacking Tyro3, or Axl, or Mer, or Axl and Mer, or Tyro3, Axl, and Mer, were immunized with recombinant protective antigen (rPA) from anthrax as a test immunogen. Serum antibody titers to rPA were analyzed by ELISA at two weeks after immunization. Briefly, prior to immunization, blood samples were collected from all mice by retro-orbital bleeding by use of heparinized capilars. Collected blood was dispensed into the desired labeled tube and allowed to coagulate at room temperature. Samples were then subjected to centrifugation (10 mins at 1500 rpm at room temperature). The upper phase (serum) of the sample was transferred to a new tube and store at -80C. On day 1, mice were immunized with 20 ug Anthrax Protective antigen adsorved in alum (1:1). Blood samples were collected every 7 days (day 14, 21, 28 and 35 post immunization) by retroorbital bleeding. Serum was prepared and stored as done for preimmune serum. On day 42, serum was collected by retroorbital bleeding and mice were given a second dose of 20ug of Anthrax Protective antigen (alone, no alum). Following the second boost, blood samples were collected again every 7 days (day 49, 56, 63 and 70 post immunization) by retroorbital bleeding..

As illustrated in FIGS. 11 and 12, higher antibody titers were detected in the receptor single mutants, and highest of all in mice that lack all three TAM receptors. These results demonstrate that inhibition of TAM signaling a method for the enhancement of antibody responses to immunization.

The application discloses, inter alia, the following

### SUMMARY PARAGRAPHS

1. A method of enhancing an immune response in a subject, comprising:
   administering to a subject in need of immunoenhancement a therapeutically effective amount of a TAM receptor inhibitor, thereby enhancing the immune response in the subject.
2. A method of suppressing an immune response in a subject, comprising:
   administering to a subject in need of immunosuppression a therapeutically effective amount of a TAM receptor agonist, thereby suppressing the immune response in the subject.
3. The method as in paragraphs 1 or 2, wherein the TAM receptor is Tyro3, Axl, or Mer.
4. The method of any of paragraphs 1-3, wherein the TAM receptor inhibitor has an IC₅₀ of less than about 50 µM or the TAM agonist has an EC₅₀ of less than about 50 µM.
5. The method as in paragraph 4, wherein the TAM receptor inhibitor has an IC₅₀ of from about 1 pM to about 5 µM or the TAM agonist has an EC₅₀ of from about 1 pM to about 5 µM.
6. The method of any of paragraphs 1 or 3 to 5, wherein the TAM receptor inhibitor binds to an intracellular ATP binding site of Tyro3, Axl, or Mer.
7. The method of any of paragraphs 1 or 3-6, wherein the TAM receptor inhibitor is MP470, SGI-AXL-277, AXL-1, AXL-2, AXL-3, AXL-4, AXL-5, AXL-6, AXL-7, AXL-8, AXL-9, or derivatives thereof.
8. The method of any of paragraphs 1-4, wherein the TAM receptor inhibitor has a chemical structure of: 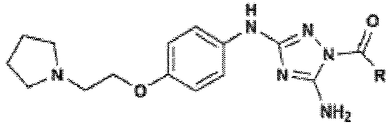 or 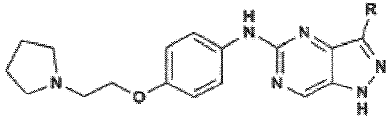 or 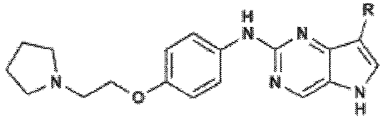 wherein R is a hydrogen or methyl group.
9. The method of any of paragraphs 1 or 3-5, wherein the TAM receptor inhibitor binds to an extracellular domain of the TAM receptor or a TAM receptor ligand.
10. The method of any of paragraphs 1, 3-6, or 8, wherein the TAM receptor inhibitor is a small molecule inhibitor, a monoclonal antibody, or an siRNA.
11. The method of any of paragraphs 1, 3-5, or 9-10, wherein the TAM receptor inhibitor binds to Gas6 or Protein S and interferes with the binding of Gas6 or Protein S to the extracellular domain of the TAM receptor and/or activation of the TAM receptor.
12. The method of any of paragraphs 1 or 3-11, wherein the method further comprises:
   administering to the subject a therapeutically effective amount of a vaccine, thereby enhancing the immune response to the vaccine.
13. The method as in paragraph 12, wherein the TAM receptor inhibitor is administered to the subject substantially concurrently with the vaccine.
14. The method as in paragraph 13, wherein the vaccine comprises dendritic cells (DCs) activated against a tumor in the subject.
15. The method of any of paragraphs 1 or 3-14, wherein the method is a method of treating a tumor in the subject, a method of treating a disease of immunosuppression, or a method of treating sepsis.
16. The method as in paragraph 15, wherein the subject has severe sepsis, septic shock, SIRS, or severe SIRS.
17. The method of any of paragraphs 1, 3-13 or 15-16, wherein the method is a method of treating an infection in a subject in need thereof.
18. The method of any of paragraphs 1-17, wherein the subject is immunocompromised or immunosuppressed.
19. The method of any of paragraphs 2-5, wherein the TAM receptor agonist is GAS6, Protein S, an amino-terminally truncated GAS6, an amino-terminally-truncated Protein S, an antibody that binds to an extracellular domain of the TAM receptor and can cross-link and activate the TAM receptor in a TAM receptor dimer, or a small molecule that binds to an extracellular domain of the TAM receptor and can activate the TAM receptor.
20. The method as in paragraphs 2-5, wherein the TAM receptor agonist is a GAS6 or Protein S protein that does not have one or more Gla or EGF domains and can activate the TAM receptor.
21. The method of any of paragraphs 2-5 or 19-20, wherein the method further comprises:
   administering to the subject a therapeutically effective amount of an immunosuppressive agent, thereby suppressing the immune response in the subject.
22. The method as in paragraph 21, wherein the TAM receptor agonist is administered to the subject substantially concurrently with the immunosuppressive agent.
23. The method of any of paragraphs 2-5 or 19-22, wherein the method is a method of treating or preventing an autoimmune disease, an allergy, graft-versus-host (GVH) disease, or transplant rejection.
24. The method as in paragraph 23, wherein the autoimmune disease is rheumatoid arthritis, Hashimoto's thyroiditis, pernicious anemia, Crohn's disease, ulcerative colitis, psoriasis, renal fibrosis, pulmonary fibrosis, hepatic fibrosis, Addison's disease, type I diabetes, systemic lupus erythematosus, dermatomyositis, Sjogren's syndrome, multiple sclerosis, myasthenia gravis, Reiter's syndrome, or Grave's disease.
25. A method of screening for an immunomodulator agent, comprising:
   contacting a cell expressing a TAM receptor with a test agent; and
   determining if
      (a) the test agent alters TAM receptor activity; or
      (b) the test agent inhibits binding of a ligand to the TAM receptor, wherein test agents that increase TAM receptor activity or enhance binding of a ligand to the TAM receptor are identified as immunosuppressive agents and wherein test agents that inhibit TAM receptor activity or reduce or inhibit binding of a ligand to the TAM receptor are identified as immunoenhancing agents.
26. The method as in paragraph 25, wherein the TAM receptor is Tyro3, Axl, or Mer.
27. The method of any of paragraphs 25-26, further comprising:
   selecting a test agent indicated to be an immunoenhancing agent or an immunosuppressive agent for further analysis.
28. The method of any of paragraphs 25-27, wherein the cell is in a laboratory mammal, and contacting the cell with the test agent comprises administering the test agent to the mammal.
29. The method of any of paragraphs 25-28, wherein the method is a method of screening for an immunoenhancing agent and determining whether the test agent inhibits TAM receptor activity comprises:
   contacting the cell with the test agent; and
   determining whether contacting the cell with the test agent:
      i. alters TAM autophosphorylation as compared to a control; wherein a reduction in TAM autophosphorylation in the presence of the test agent relative to the control indicates that the test agent inhibits TAM receptor activity;
      ii. alters TLR-induced cytokine production as compared to a control; wherein an increase in TLR-induced cytokine production in the presence of the test agent relative to the control indicates that the test agent inhibits TAM receptor activity;
      iii. alters TLR-induced stimulation of MAP kinase activation as compared to a control; wherein an increase in TLR-induced stimulation of MAP kinase activation in the presence of the test agent relative to the control indicates that the test agent inhibits TAM receptor activity; and/or
      iv. alters TLR-induced NF-kB activation as compared to a control;
   wherein an increase in TLR-induced NF-kB activation in the presence of the test agent relative to the control indicates that the test agent inhibits TAM receptor activity.
30. The method of any of paragraphs 25-28, wherein the method is a method of screening for an immunosuppressive agent and determining whether the test agent increases TAM receptor activity comprises:
   contacting the cell with the test agent; and
   determining whether contacting the cell with the test agent
      i. alters TAM autophosphorylation as compared to the control level of TAM autophosphorylation; wherein an increase in TAM autophosphorylation in the presence of the test agent relative to a control indicates that the test agent increases TAM receptor activity;
      ii. alters TLR-induced cytokine production as compared to a control; wherein a decrease in TLR-induced cytokine production in the presence of the test agent relative to a control indicates that the test agent increases TAM receptor activity;
      iii. alters TLR-induced stimulation of MAP kinase activation as compared to a control; wherein a decrease in TLR-induced stimulation of MAP kinase activation in the presence of the test agent relative to a control indicates that the test agent increases TAM receptor activity; and/or
      iv. alters TLR-induced NF-kB activation as compared to a control; wherein a decrease in TLR-induced NF-kB activation in the presence of the test agent relative to the control indicates that the test agent increases TAM receptor activity.
31. The method of any one of paragraphs 29-30, wherein the control is a value obtained for a cell not contacted with the test agent or is a reference value.
32. The method of any of paragraphs 25-28, wherein determining whether the test agent alters TAM receptor activity comprises:
   (a) determining a control level of SOCS1 and SOCS 3 expression before contacting the cell with the test agent;
   (b) contacting the cell with the test agent; and
   (c) determining whether contacting the cell with the test agent alters SOCS1 and SOCS 3 expression as compared to the control level of SOCS1 and SOCS 3 expression; wherein a reduction in SOCS1 and SOCS 3 expression in the presence of the test agent relative to the control level indicates that the test agent inhibits TAM receptor activity and such test agents are identified as immunoenhancing agents, and wherein an increase in SOCS1 and SOCS 3 expression in the presence of the test agent relative to the control level indicates that the test agent increases TAM receptor activity and such test agents are identified as immunosuppressive agents.
33. The method of any of paragraphs 25-28, or 30 or -32, wherein the method is a method of screening for an immunosuppressive agent and wherein contacting the cell expressing the TAM receptor with the test agent comprises contacting the cell with the test agent in the presence of a TAM receptor agonist.
34. The method as in paragraph 33, wherein the TAM receptor agonist is Gas6, an amino-terminally-truncated Gas6, Protein S, or an amino-terminally-truncated Protein S.
35. The method as in paragraph 34, wherein the TAM receptor agonist is an antibody that binds to the extracellular domain of the TAM receptor and crosslinks and activates the TAM receptor in a TAM receptor dimer.

## Claims

1. A TAM receptor agonist for use in a method of treating or preventing an autoimmune disease, an allergy, a graft-versus-host disease, or a transplant rejection by suppressing the immune response in a subject, wherein the TAM receptor agonist is an antibody that binds to an extracellular domain of the TAM receptor, and wherein the TAM receptor is Axl.

2. The TAM receptor agonist for use of claim 1, wherein the TAM receptor agonist can cross-link and activate the TAM receptor in a TAM receptor dimer.

3. The TAM receptor agonist for use of claim 1 or 2, wherein the method further comprises administering to the subject an immunosuppressive agent, thereby suppressing the immune response in the subject.

4. The TAM receptor agonist for use of claim 3, wherein the TAM receptor agonist is administered to the subject substantially concurrently with the immunosuppressive agent.

5. The TAM receptor agonist for use of any of claims 1-4, wherein the autoimmune disease is rheumatoid arthritis, Hashimoto's thyroiditis, pernicious anemia, Crohn's disease, ulcerative colitis, psoriasis, renal fibrosis, pulmonary fibrosis, hepatic fibrosis, Addison's disease, type I diabetes, systemic lupus erythematosus, dermatomyositis, Sjogren's syndrome, multiple sclerosis, myasthenia gravis, Reiter's syndrome, or Grave's disease.

## Patentansprüche

1. TAM-Rezeptor-Agonist zur Anwendung in einem Verfahren zur Behandlung oder Prävention einer Autoimmunerkrankung, einer Allergie, einer Graft-versus-Host-Krankheit oder einer Transplantatabstoßung durch Unterdrücken der Immunantwort in einem Subjekt, wobei der TAM-Rezeptor-Agonist ein Antikörper ist, der an eine extrazelluläre Domäne des TAM-Rezeptors bindet und wobei der TAM-Rezeptor Axl ist.

2. TAM-Rezeptor-Agonist zur Anwendung nach Anspruch 1, wobei der TAM-Rezeptor-Agonist den TAM-Rezeptor zu einem TAM-Rezeptor-Dimer vernetzen und aktivieren kann.

3. TAM-Rezeptor-Agonist zur Anwendung nach Anspruch 1 oder 2, wobei das Verfahren ferner das Verabreichen eines Immunsuppressivums an das Subjekt umfasst, wodurch die Immunantwort in dem Subjekt unterdrückt wird.

4. TAM-Rezeptor-Agonist zur Anwendung nach Anspruch 3, wobei der TAM-Rezeptor-Agonist dem Subjekt im Wesentlichen gleichzeitig mit dem Immunsuppressivum verabreicht wird.

5. TAM-Rezeptor-Agonist zur Anwendung nach einem der Ansprüche 1 bis 4, wobei die Autoimmunerkrankung rheumatoide Arthritis, Hashimoto-Thyreoiditis, perniziöse Anämie, Morbus Crohn, Colitis ulcerosa, Psoriasis, Nierenfibrose, Lungenfibrose, Leberfibrose, Morbus Addison, Diabetes Typ I, systemischer Lupus erythematodes, Dermatomyositis, Sjögren-Syndrom, Multiple Sklerose, Myasthenia gravis, Reiter-Syndrom oder Morbus Grave ist.

## Revendications

1. Agoniste de récepteur TAM pour utilisation dans un procédé de traitement ou de prévention d'une maladie auto-immune, d'une réaction allergique, d'une maladie du greffon contre l'hôte, ou d'un rejet de greffe en supprimant la réponse immunitaire chez un sujet, dans lequel l'agoniste de récepteur TAM est un anticorps qui se lie à un domaine extracellulaire du récepteur TAM, et dans lequel le récepteur TAM est Axl.

2. Agoniste de récepteur TAM pour utilisation selon la revendication 1, dans lequel l'agoniste de récepteur TAM peut réticuler et activer le récepteur TAM dans un dimère de récepteur TAM.

3. Agoniste de récepteur TAM pour utilisation selon la revendication 1 ou 2, dans lequel le procédé comprend en outre l'administration au sujet d'un agent immunosuppresseur, supprimant ainsi la réponse immunitaire chez le sujet.

4. Agoniste de récepteur TAM pour utilisation selon la revendication 3, dans lequel l'agoniste de récepteur TAM est administré au sujet de manière substantiellement simultanée avec l'agent immunosuppresseur.

5. Agoniste de récepteur TAM pour utilisation selon l'une quelconque des revendications 1 à 4, dans lequel la maladie auto-immune est la polyarthrite rhumatoïde, la thyroïdite de Hashimoto, l'anémie pernicieuse, la maladie de Crohn, la colite ulcéreuse, le psoriasis, la fibrose rénale, la fibrose pulmonaire, la fibrose hépatique, la maladie d'Addison, le diabète de type I, la maladie systémique lupus érythémateux, la dermatomyosite, le Syndrome de Sjögren, la sclérose en plaques, la myasthénie grave, le Syndrome de Reiter, ou la maladie de Basedow.
